(19) **Europäisches Patentamt / European Patent Office / Office européen des brevets**

(11) **EP 1 779 727 A1**

(12) # EUROPEAN PATENT APPLICATION
published in accordance with Art. 158(3) EPC

(43) Date of publication:
**02.05.2007 Bulletin 2007/18**

(21) Application number: **05765698.5**

(22) Date of filing: **12.07.2005**

(51) Int Cl.:
*A01N 59/16* (2006.01)    *A01N 43/40* (2006.01)
*A01N 43/78* (2006.01)    *A01N 47/18* (2006.01)
*A01N 59/20* (2006.01)    *A01N 43/52* (2006.01)
*C08K 3/00* (2006.01)    *C08K 5/00* (2006.01)
*C08L 101/00* (2006.01)    *C11D 3/48* (2006.01)
*B32B 27/18* (2006.01)    *E04F 15/02* (2006.01)

(86) International application number:
**PCT/JP2005/012834**

(87) International publication number:
**WO 2006/006594 (19.01.2006 Gazette 2006/03)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.07.2004 JP 2004206538**
**09.05.2005 JP 2005136366**
**09.05.2005 JP 2005136708**

(71) Applicant: **Idemitsu Technofine Co. Ltd**
**Sumida-ku,**
**Tokyo 130-0015 (JP)**

(72) Inventors:
• **FUKATSU, Fumioki**
**2990205 (JP)**

• **KUSAMOTO, Nobuo**
**1300015 (JP)**
• **AMANO, Kazutoshi**
**1300015 (JP)**
• **UBARA, Atsuhiko**
**2990205 (JP)**
• **MACHIDA, Yoshinori**
**2990205 (JP)**

(74) Representative: **Hucker, Charlotte Jane**
**Gill Jennings & Every LLP**
**Broadgate House**
**7 Eldon Street**
**GB-London EC2M 7LH (GB)**

(54) **ANTIBACTERIAL COMPOSITION, ANTIBACTERIAL MOLDING, SOLUTION CONTAINING ANTIBACTERIAL COMPOSITION, DETERGENT, SURFACE OF TATAMI MAT AND TATAMI MAT**

(57)    An antibacterial composition containing an organic antibacterial agent and an inorganic antibacterial agent is provided. Zirconium phosphate having supported thereon silver or copper or a salt thereof may be used as the inorganic antibacterial agent, and 2-mercaptopyridine-N-oxide sodium, carbendazim (methyl 1H-2-benzimidazole carbamate), or thiabendazole (2-(4-thiazolyl)-1H-benzimidazole) may be used as the organic antibacterial agent. Those components contain no halogen, so the antibacterial composition can be made halogen-free. The antibacterial composition may be applied to form an antibacterial molding, for example, by molding it together with a resin material or applying it together with a coating agent on a resin molding.

**EP 1 779 727 A1**

## Description

TECHNICAL FIELD

The present invention relates to an antibacterial composition containing an organic antibacterial agent and an inorganic antibacterial agent, an antibacterial molding containing the antibacterial composition, and a solution, a detergent, a tatami facing mat, and a tatami mat each containing the antibacterial composition.

BACKGROUND ART

Many microorganisms exist in the life environment of humans. Particularly, Japan where it is hot and humid provides a favorable environment that allows a wide variety of prokaryotic organisms such as bacteria, eukaryotic organisms such as fungi and yeasts, and molds and algae to propagate. Also, recent changes in life environment, such as increase of more closed rooms due to popularization of aluminum sashes and the like and maintenance of indoor temperature and indoor humidity due to popularization of air-conditioners result in providing an environment that is suitable for the propagation of microorganisms. Further, in places where there is much water, such as a bathroom and a kitchen, resins and organic substances that cover the surface of resins may often become a seedbed for fungi, and therefore various countermeasures against microorganisms have been taken in such an environment. As a typical measure, various antibacterial agents are added to the resins that are used in the bathroom.

Here, examples of known organic antibacterial agents to be added to resins include diiodomethyl-p-trisulfone, 2,4,5,6-tetrachloroisophthalonitrile, 2,3,5,6-tetrachloro-4-methylsulfonylpyridine, 2-methyl-4-isothiazolin-3-one, 2-n-octyl-4-isothiazolin-3-one, and 2-(4-thiazolyl)- benzimidazole. On the other hand, examples of inorganic antibacterial agents include inorganic compounds such as cuprous oxide, a copper component, zinc sulfate, and copper-nickel alloy, those containing metals supported on an inorganic substance such as calcium phosphate or zeolite, and those having a photocatalytic function such as titanium oxide.

However, many of the conventional antibacterial agents including the organic antibacterial agents exhibit an antibacterial effect slowly and can have antibacterial activities to only limited microorganisms. Further, some of the components of the conventional antibacterial agents are water-soluble, and in this case, sustention of the effect has become problematic.

On the other hand, to solve such problems of the organic antibacterial agents, composite type organic antibacterial compositions that contain a plurality of organic antibacterial agents as components have been studied. For example, antibacterial compositions that contain a nitrile antibacterial agent, a pyridine-based antibacterial agent, a haloalkylthio-based antibacterial agent, an organoiodo-based antibacterial agent, a thiazole-based antibacterial agent, or a benzimidazole-based antibacterial agent as active ingredients have been proposed (see, for example, Patent Document 1).

As mentioned above, it has been known that a synergistic effect is obtained by applying, to each microorganism, a combination of two or more chemicals as an antibacterial composition that removes or repels microorganisms including, for example, prokaryotic organisms such as bacteria, eukaryotic organisms such as fungi and yeasts, and algae.

That is, using two or more kinds of chemicals will provide a synergistic effect such as a broadening of antibacterial spectrum or a decrease of MIC (Minimum Inhibitory Concentration) value (ppm) as compared with using a chemical alone. As a method of using chemicals of different kinds in combination, there is known a constitution using an organic antibacterial agent and an inorganic antibacterial agent (see, for example, Patent Document 1).

The composition disclosed in Patent Document 2 contains: inorganic oxide fine particles that are composed of a metal component such as silver, copper, or zinc and an inorganic oxide other than the metal component and have antibacterial/fungi-preventing/algae-preventing effects; and an organic antibacterial/mold-preventing/algae-preventing agent of at least one of a thiazole-based compound and an imidazole-based compound. The inorganic oxide fine particles are adjusted to have an average particle size of 500 nm or less in view of their influence on dispersibility and surface color of the article to be treated. Further, the content of the inorganic oxide fine particles is set to 0.001 wt% or more for the effect of combined use.

[Patent Document 1]: JP 8-92012 A (Claim 2, [0030])
[Patent Document 2]: JP 2004-339102 A (p.4-10)

DISCLOSURE OF THE INVENTION

PROBLEMS TO BE SOLVED BY THE INVENTION

However, although the composite type organic antibacterial composition disclosed in Patent Document 1 as mentioned above can cope with a wider variety of microorganisms than the conventional antibacterial agents can, they are still insufficient, and slow exhibition of antibacterial performance which is a problem specific to organic antibacterial agents still remains to be solved. In addition, organic antibacterial agents that contain a halogen component such as chlorine or fluorine in their components have been widely used. However, antibacterial agents containing a halogen component will generate dioxin when they are burned, causing a problem in view of safety, and also raise a problem that when they are kneaded in resins to form moldings, they will corrode a metallic part such as a metallic mold. Further, many of antibacterial agents containing a halogen component cause skin irritation.

On the other hand, the inorganic antibacterial agents include those that are imparted with antibacterial effects by supporting a metal such as silver or copper while suppressing its elution. These have no problem from the viewpoint of safety. However, for exhibiting antibacterial effects, the inorganic antibacterial agents need direct contact with the microorganism since metal atoms are supported thereon. Further, although some metals exhibit their antibacterial activity by generation of active oxygen, their antibacterial effect is insufficient since generation of active oxygen needs optical energy and the generated active oxygen is readily eliminated with organic substances other than the microorganisms.

Meanwhile, since the antibacterial composition is used in the life environment, it must contain a chemical that gives no adverse influence such as irritation to the human body even when it is attached to the skin while it is applied to an article to be treated or when a user contacts a molding that is coated with or contains the antibacterial composition. Also, it is necessary to use a chemical that generates no toxic substance such as dioxin when a molding that is coated with or contains the antibacterial composition is subjected to incineration disposal.

Those chemicals desirably cause no corrosion to manufacturing appliances such as vessels used for mixing and metallic molds used for molding in a manufacturing process, for example, when preparing an antibacterial composition or when forming a molding that contains an antibacterial composition. That is, it is desirable to use those chemicals that cause no inconveniences such as a decrease in constructivity of manufacturing appliance or an increase in cost because of needs for a special apparatus in the manufacturing appliance, such as using anticorrosive materials for the manufacturing appliance.

However, the above-mentioned antibacterial compositions that are intended to exhibit effects by combination of the conventional organic and inorganic antibacterial agents as disclosed in Patent Document 1 can not exhibit satisfactory synergistic effects and can obtain synergistic effects only on limited microorganisms. That is, they can not largely broaden their antibacterial spectrum. Further, to exhibit antibacterial activity with a broadened antibacterial spectrum, the MIC value is to be increased, that is, the amount of the antibacterial agent to be added is to be increased, thus failing to providing an efficient antibacterial effect, and also causing an inconvenience that the moldability of a molding is decreased due to an increase in the amount of the antibacterial agent to be added. Also, an allergenic substance such as 2-(n-octyl)-4-isothiazol-3-one (abbreviation: OIT) is used.

Therefore, it is an object of the present invention to provide an antibacterial composition having excellent initial antibacterial performance and excellent sustention of antibacterial performance, being capable of coping with many kinds of microorganisms, and causing no problem in safety, an antibacterial molding provided with the antibacterial composition, and a solution, a detergent, a tatami facing mat, and a tatami mat each containing the antibacterial composition.

It is another object of the present invention to provide an antibacterial composition and an antibacterial molding that can exhibit an antibacterial effect on many kinds of microorganisms, giving an efficient antibacterial effect and having no adverse influence on the human body and environment, a solution, a detergent, a tatami facing mat, and a tatami mat each containing the antibacterial composition.

MEANS FOR SOLVING THE PROBLEMS

In order to achieve the above-mentioned objects, the antibacterial composition of the present invention is characterized by including an inorganic antibacterial agent and an inorganic antibacterial agent.

The antibacterial composition of the present invention adopts a constitution that contains an organic antibacterial agent and an inorganic antibacterial agent. Therefore, the antibacterial composition has a broad antibacterial spectrum, can cope with significantly increased kinds of microorganisms, and has excellent antibacterial effects. Further, blending the inorganic antibacterial agent results in an increase in initial antibacterial performance and sustention of the antibacterial effects as well as a decrease in eluates, so environmental pollution can be advantageously suppressed and the antibacterial composition also has excellent safety. Since the antibacterial composition of the present invention is suited to be blended in resins, good resin moldability can be obtained.

Note that the term "antibacterial property (antibacterial effect)" as used herein refers to an antibacterial effect itself that prevents growth and propagation of microorganisms such as fungi and bacteria and, in addition thereto, to a fungi-preventing effect, an antifungal effect, and an algae-preventing effect.

The antibacterial composition of the present invention is preferably one in which the inorganic microbial agent is zirconium or salts thereof or zeolite having supported thereon a metal, and particularly preferably one in which the antibacterial agent is zirconium phosphate or salts thereof having supported thereon silver or copper.

According to the present invention as mentioned above, adoption of zirconium or salts thereof or zeolite having supported thereon a metal, in particular, zirconium phosphate or salts thereof or zeolite having supported thereon silver or copper results in an antibacterial composition that has an excellent safety to the human body, exhibits antibacterial effect quickly, and has excellent sustention of antibacterial performance.

Further, in the present invention, the above-mentioned inorganic antibacterial agent is preferably at least one of the silver-based antibacterial agent and zinc oxide.

In the present invention, at least one of the silver-based antibacterial agent and zinc oxide that can provide a synergistic effect with an imidazole-based organic antibacterial agent is used as the inorganic antibacterial agent, so a significant antibacterial property can be obtained. Then, by using a silver-based antibacterial agent and zinc oxide in combination, antibacterial effects by the silver-based antibacterial agent and the zinc oxide by themselves can be obtained. In addition, use of different species of inorganic antibacterial agents of the same group in combination can also provide a synergistic effect in antibacterial actions, so a significant antibacterial property can be readily obtained.

Further, in the present invention, the silver-based antibacterial agent is preferably zirconium or salts thereof or zeolite having supported thereon silver.

In the present invention, since zirconium or salts thereof or zeolite having supported thereon silver is used as the silver-based antibacterial agent, silver which is a precious metal is used in a minimum amount that can exhibit an antibacterial action, so an antibacterial action can be obtained efficiently by the inorganic antibacterial agent and also a synergistic effect of antibacterial action with the organic antibacterial agent can be obtained, thus making it possible to easily reduce cost.

Also, in the present invention, it is preferable that the inorganic antibacterial agent contain the zirconium or salts thereof or zeolite having supported thereon silver and the zinc oxide in a blend ratio of 1:1 to 1:10 by mass.

In the present invention, since the zirconium or salts thereof or zeolite having supported thereon silver and the zinc oxide are used in combination, use of different species of the inorganic antibacterial agents of the same group in combination can also provide a synergistic effect in antibacterial actions, so a significant antibacterial property can be readily obtained. Further, the antibacterial action by an inorganic antibacterial agent by itself, the synergistic effect in the antibacterial action by use of inorganic microbial agents in combination, and the synergistic effect in the antibacterial action by use of an inorganic antibacterial agent with an organic antibacterial agent are not deteriorated, and the amount of silver, a precious metal, to be used is reduced, so a reduction of cost can be readily attained. Further, the blend ratio of the zirconium or salts thereof or zeolite having supported thereon silver to the zinc oxide being 1:1 to 1:10 by mass leads to a proper decrease in the amount of silver to be used without deteriorating the antibacterial property of the antibacterial composition.

Here, it is to be noted that if the blend ratio of the zirconium or salts thereof or zeolite having supported thereon silver to the zinc oxide is 1 to less than 1, i.e., zinc oxide is in a smaller amount than 1:1 by mass, then a sufficient cost reduction by a decrease in the amount of silver to be used will be difficult to obtain. Also, there is the possibility that discoloration due to oxidation of silver may arise. On the other hand, if zinc oxide is in a ratio more than 1:10 by mass, there is the possibility that a sufficient antibacterial action by silver will be difficult to obtain. Therefore, it is preferable that the blend ratio of the zirconium or salts thereof or zeolite having supported thereon silver to the zinc oxide be set to 1:1 to 1:10 by mass.

In the antibacterial composition of the present invention, it is preferable that the organic antibacterial agent be a pyridine-based antibacterial agent or a benzimidazole-based antibacterial agent, and it is particularly preferable that the pyridine-based antibacterial agent be 2-mercaptopyridine-N-oxide sodium and the benzimidazole-based antibacterial agent be at least one of carbendazim (methyl 1H-2-benzimidazole carbamate) and thiabendazole (2-(4-thiazolyl)-1H-benzimidazole).

According to the present invention as mentioned above, use of a pyridine-based antibacterial agent and a benzimidazole-based antibacterial agent in combination as the organic antibacterial agent results in that an antibacterial property can be exhibited to microorganisms on which individual components of the microbial composition have no effect. The antibacterial property will be more advantageously exhibited when 2-mercaptopyridine-N-oxide sodium is adopted as the pyridine-based antibacterial agent and at least one of carbendazim (methyl 1H-2-benzimidazole carbamate) and thiabendazole (2-(4-thiazolyl)-1H-benzimidazole) is adopted as the benzimidazole-based antibacterial agent.

The antibacterial composition of the present invention preferably is one in which the organic antibacterial agent includes two species selected from the benzimidazole-based antibacterial agents. More preferably, the benzimidazole-based antibacterial agents are one that has a thiazolyl group on a benzimidazole ring and one that has a carbamate group on the benzimidazole ring. Particularly preferably, the one that has a thiazolyl group is 2-(4-thiazolyl)-1H-benzimidazole and the one that has a carbamate group on the benzimidazole ring is methyl 2-benzimidazole carbamate.

According to the present invention as mentioned above, an antibacterial property can be exhibited by the synergistic effect to microorganisms on which individual components of the microbial composition have no effect. The antibacterial property will be more advantageously exhibited when 2-(4-thiazolyl)-1H-benzimidazole and methyl 2-benzimidazole carbamate are adopted as the benzimidazole-based antibacterial agent.

It is preferable that the antibacterial composition of the present invention include at least two species selected from the imidazole-based organic antibacterial agents and the inorganic antibacterial agent.

In the present invention, since at least two imidazole-based organic antibacterial agents and an inorganic antibacterial agent are used in combination, in particular, at least two imidazole-based organic antibacterial agents alone (two species from the same group) and an inorganic antibacterial agent are used in combination, no skin irritation occurs and in addition, a significantly broad antibacterial spectrum can be obtained even at a low minimum inhibitory concentration (MIC value) due to a synergistic effect, so a high antibacterial action can be obtained readily and efficiently.

Conventionally, to broaden the antibacterial spectrum, it has been necessary to use chemically different antibacterial agents. However, in the present invention, a significantly broad antibacterial spectrum can be attained by a combination of imidazole-based antibacterial agents alone. This effect is quite unexpectable from the known knowledge.

Note that in the present invention, the term "antibacterial property (antibacterial effect)" as used herein refers to an antibacterial effect itself that prevents growth and propagation of microorganisms such as fungi and bacteria and, in addition thereto, to a fungi-preventing effect, an antifungal effect, and an algae-preventing effect.

Further, in the present invention, it is preferable that the blend ratio of the imidazole-based organic antibacterial agent to the inorganic antibacterial agent be 1:1 to 5:1 by mass.

In the present invention, by setting the blend ratio of the imidazole-based organic antibacterial agent to the inorganic antibacterial agent at 1:1 to 5:1 1 by mass, a significant synergistic effect in an antibacterial action can be properly obtained by use of the organic antibacterial agent and the inorganic antibacterial agent in combination as well as the antibacterial actions by the organic antibacterial agents and the inorganic antibacterial agent, respectively.

Here, it is to be noted that if the blend ratio of the imidazole-based organic antibacterial agent to the inorganic antibacterial agent is less than 1 to 1, i.e., the organic antibacterial agent is in a smaller amount than 1:1 by mass, then there is the possibility that no broadening of the antibacterial spectrum at a low MIC value will be obtained. On the other hand, when the organic antibacterial agent is more than 5:1 by mass, the ratio of the organic antibacterial agent that has a slow initial antibacterial performance and sustention of antibacterial performance of which tends to be decreased as compared with the inorganic antibacterial agent is greater, so there is the possibility that a significant antibacterial property that is stable from the beginning of use for a long period of time will not be obtained. Therefore, it is preferable that the blend ratio of the benzimidazole-based organic antibacterial agent to the inorganic antibacterial agent is set at 1:1 to 5:1 by mass.

It is preferable that the antibacterial composition of the present invention contain substantially no halogen in the organic antibacterial agent and the inorganic antibacterial agent.

According to the present invention as mentioned above, since the components, i.e., the organic antibacterial agent and the inorganic antibacterial agent contain substantially no halogen, the antibacterial composition can be made halogen-less (non-halogen), so that even when the antibacterial composition is subjected to incineration disposal, no dioxin is generated, or when a molding is formed from a resin that contains the antibacterial composition, the metallic mold for molding can be prevented from corrosion.

Here, the term "substantially" refers to an idea that also includes the case where an extremely small amount of a halogen component (halogen atom) is intentionally allowed to be present in the constitution of the antibacterial composition as far as the effect of the invention is not adversely influenced.

Further, in the present invention, it is preferable that the antibacterial compositions contain no halogen compound and be substantially insoluble in water.

In the present invention, since the above-mentioned antibacterial composition of the present invention is made to contain no halogen compound and be substantially insoluble in water, if the antibacterial composition of the present invention or a molding or a solution that contains the antibacterial composition is heated for incineration disposal, it causes no inconvenience such as generation of a toxic substance such as dioxin. Further, since the antibacterial composition is insoluble to water, so it is free of the inconvenience that the antibacterial agent is flown away under use conditions such as being exposed to rains and dews, thus failing to stably provide antibacterial property for a long period of time, and it becomes easier to mix the antibacterial composition with a resin material well to provide a molding having an antibacterial property, and general versatility can also be increased with ease.

It is preferable that the antibacterial composition of the present invention contain the inorganic antibacterial agent in a rate of content of 0.1 mass% or more and 70 mass% or less with respect to the total composition.

According to the present invention, since the rate of content of the inorganic antibacterial agent to the total antibacterial composition is set in a specified range, the effect by inclusion of the inorganic antibacterial agent, such as an increase in initial antibacterial property and sustention of antibacterial property can be exhibited at most, so the above-mentioned effects of the present invention can be more advantageously exhibited.

The antibacterial molding of the present invention is characterized by including the antibacterial composition of the present invention as mentioned above.

The antibacterial molding of the present invention is characterized by containing the antibacterial composition of the present invention.

In the present invention, since the molding of the present invention contains the above-mentioned antibacterial composition, there can be provided a molding that exhibits the effect of having no adverse influence on the human body and environment and providing a significantly broad antibacterial spectrum due to the synergistic effect even at low MIC values and efficiently giving a high antibacterial action. Since the molding itself has a significant antibacterial property, it can be utilized with ease.

In addition, in the present invention, it is preferable that the antibacterial molding of the present invention contain the antibacterial composition in an amount of 0.01 mass% or more and 10.0 mass% or less.

In the present invention, by adjusting the content of the antibacterial composition to 0.01 mass% or more and 10.0 mass% or less, a molding that exhibits a significant antibacterial property without deteriorating characteristics such as, for example, strength and appearance can be provided.

Here, it is to be noted that if the content of the antibacterial composition is less than 0.01 mass%, there is the possibility that broadening of antibacterial spectrum at low MIC values will be difficult to obtain and a sufficient antibacterial property will be difficult to obtain. On the other hand, if the content of the antibacterial composition is more than 10.0 mass%, there is the possibility that inconveniences may occur that the characteristics of the molding is deteriorated or the workability upon molding is decreased. Therefore, it is preferable that the content of the antibacterial composition be set to 0.01 mass% or more and 10.0 mass% or less.

Further, by preparing the antibacterial molding of the present invention in the form of a film or a sheet or a laminate of these, it can be used in various applications and is convenient.

Further, it is preferable that the antibacterial molding of the present invention includes the antibacterial composition such that the inorganic antibacterial agent is contained in a ratio of less than 0.5 mass% with respect to the total mass of the molding and the sterilization activity (for general applications) stipulated by Japan Textile Evaluation Technology Council, corporate juridical person is defined by the following conditions.

$$\log (A/C) \geq 0;$$

A: Number of microorganism on a standard cloth immediately after inoculation;
C: Number of viable microorganism on a processed cloth after incubation for 18 hours;
Kind of microorganism: *Staphylococcus aureus* and *Klebsiella pneumoniae.*

In the present invention, when the antibacterial composition of the present invention is blended in the molding, the sterilization activity stipulated by Japan Textile Evaluation Technology Council, corporate juridical person satisfies log (A/C)≥0 even when the inorganic antibacterial agent contained in the molding is less than 0.5 mass% and exhibits a broad antibacterial spectrum, thus exhibiting an antibacterial effect at low MIC values.

In particular, when the inorganic antibacterial agent is in an amount of 0.05 mass% or more, preferably 0.1 mass% or more and 0.4 mass% or less, this antibacterial effect is exhibited well. Even when such an antibacterial composition is in a low concentration, the antibacterial composition of the present invention exhibits a broad antibacterial spectrum that can not be attained by the conventional antibacterial compositions and exhibits an excellent antibacterial effect at low MIC values.

The antibacterial molding of the present invention is a multilayer sheet, which may be formed such that the layer that contains the antibacterial composition is not placed outside.

The antibacterial composition of the present invention has an effect of repelling microorganisms so that it can exhibit an antibacterial effect without a direct contact with the microorganisms. Accordingly, when the molding is prepared in the form of a multilayer sheet, the sheet can be advantageously imparted with the effect exhibited by the antibacterial composition even when the layer that contains the antibacterial composition is not placed outside, for example, as an intermediate layer.

The solution containing the antibacterial composition of the present invention is characterized by having dispersed therein the antibacterial composition as mentioned above.

In the present invention, since the antibacterial composition is dispersed in the solution uniformly, an antibacterial solution can be provided that exhibits the effect of increasing contact with the microorganisms in the solution to exhibit a sufficient antibacterial effect even when the antibacterial composition is in low concentrations, giving no adverse influence on the human body and environment, and giving a significantly broad antibacterial spectrum due to a synergistic effect even at low MIC values, thus providing a high antibacterial action readily and efficiently. Further, since the solution itself exhibits a significant antibacterial property, it can be utilized with ease and general versatility can be increased with ease. Note that the solution in which the antibacterial composition of the present invention is to be blended may be any of liquid organic substances such as water, organic solvents, oils, and paints and also combinations of these. In particular, when solutions are used in products that humans may contact, such as cleaners and waxes that are applied on the floor or walls, it is preferable that the solution be aqueous or contain mainly water in view of safety and decreasing the environmental load.

In the antibacterial composition-containing solution of the present invention, the antibacterial composition is dispersed in a concentration of preferably 10 ppm or more and 1,000 ppm or less upon use.

In the present invention, since the concentration of the antibacterial composition is set to 10 ppm or more and 1,000 ppm or less, a good antibacterial action showing a broad antibacterial spectrum can be efficiently exhibited even at low MIC values. That is, the antibacterial composition of the present invention can exhibit a sufficient antibacterial effect even in a low concentration of 10 ppm or more and 1,000 ppm or less.

Here, it is to be noted that if the concentration of the antibacterial composition is lower than 10 ppm, there is the possibility that the broadening of antibacterial spectrum at low MIC values will be difficult to obtain and a sufficient antibacterial property will be difficult to exhibit. On the other hand, the concentration of the antibacterial composition higher than 1,000 ppm is not desirable since the increase in the antibacterial effect does not exceed the increase in cost due to increase in the bending amount of the antibacterial composition, thus decreasing the economical effect. In addition, there is the possibility that uniform dispersion will be difficult to attain. Therefore, the concentration of the antibacterial composition is set to 10 ppm or more and 1,000 ppm or less.

Further, it is preferable that the antibacterial composition-containing solution of the present invention be produced, transported, and stored as a solution in which the antibacterial composition of the present invention is in a concentration of 0.1 mass% or more and 50 mass% or less from the viewpoint of economy and reducing labor.

The solution having this concentration is usually used as a so-called master batch that is diluted to the above-mentioned concentrations before it is used.

Here, it is to be noted that at a concentration of less than 0.1 mass%, the effect of being as a master batch is not exhibited so much while at a concentration of more than 50 mass%, the antibacterial composition will be difficult to uniformly disperse in the solution. Therefore, the concentration of the antibacterial composition is set to 0.1 mass% or more and 50 mass% or less.

The detergent of the present invention is characterized by containing the antibacterial composition-containing solution of the present invention.

Since the detergent of the present invention contains the above-mentioned antibacterial composition-containing solution, a detergent that exhibits the effect of providing a significantly broad antibacterial spectrum even at low MIC values can be provided. Here, the detergent is not particularly limited to those that are designed for washing in the main and also includes waxes such as floor waxes. Further, the detergent of the present invention can provide the above-mentioned antibacterial effect at the time of cleaning or coating, and can prevent emergence of microorganisms after the cleaning, so its usability is improved. Therefore, it can be used as a cleaner or wax that is mainly applied to floor surfaces or as a coating agent having the functions of both of the cleaner and wax.

Note that the solvent in which the antibacterial composition is blended may be any of liquid organic substances such as water, organic solvents, oils, and paints and also combinations of these. In particular, when the solution is used in products that humans may contact, such as cleaners and waxes that are applied on the floor or walls, it is preferable that the solution be aqueous or contains mainly water in view of safety and decreasing the environmental load.

The tatami facing mat of the present invention is characterized by being formed from a film that contains the antibacterial composition of the present invention.

Since the tatami facing mat of the present invention is formed by the film that contains the above-mentioned antibacterial composition, a tatami facing mat that exhibits the effect of having no adverse influence on the human body and environment and providing a significantly broad antibacterial spectrum due to a synergistic effect even at low MIC values and efficiently giving a high antibacterial property with ease. Further, the present invention provides good antibacterial effect also in a tatami facing mat that has protrusions and depressions where microorganisms tend to emerge and that is directly contacted by the human body.

The tatami mat of the present invention is characterized by including a film that contains the antibacterial composition of the present invention.

Since the tatami mat of the present invention includes the antibacterial composition-containing film, a tatami mat that exhibits the effect of having no adverse influence on the human body and environment and providing a significantly broad antibacterial spectrum due to a synergistic effect even at low MIC values and efficiently giving a high antibacterial property with ease. Further, emergence of microorganisms can be well prevented even at portions that are not visible in usual conditions, such as backside of the tatami facing mat.

BRIEF DESCRIPTION OF DRAWING

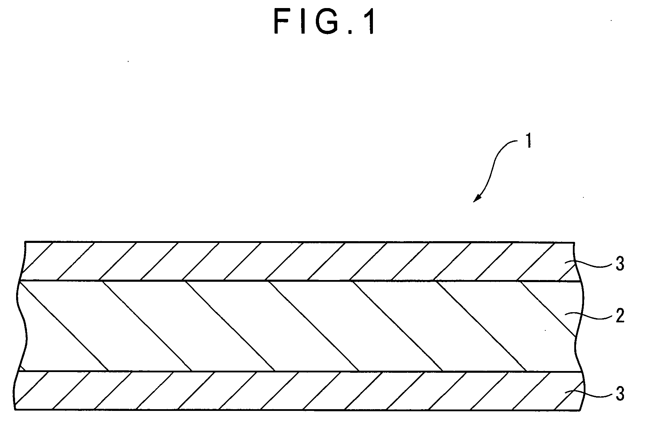

Fig. 1 is a cross-sectional view showing a multilayer sheet having three layers of one mode of the antibacterial molding according to the present invention.

EXPLANATION OF CODES

1    Multilayer sheet
2    Intermediate layer
3    Outer layer

BEST MODE FOR CARRYING OUT THE INVENTION

(First Embodiment)

The antibacterial composition of the present invention employs an organic antibacterial component and an inorganic antibacterial agent in combination, and contains the combination therein.

(Components of antibacterial agent)

Here, as the organic antibacterial agent that constitutes the antibacterial composition, it is preferable to use a pyridine-based antibacterial agent and a benzimidazole-based antibacterial agent, and it is particularly preferable to use both of them in combination. Use of the pyridine-based antibacterial agent and the benzimidazole-based antibacterial agent in combination is preferable since an antibacterial property can be exhibited by a synergistic effect on microorganisms on which individual components are not effective.

It is preferable to use pyridine derivatives as the pyridine-based antibacterial agent and examples thereof include 2-chloro-6-trichloromethylpyridine, 2-chloro-4-trichloromethyl-6-methoxypyridine, 2-chloro-4-trichloromethyl-6-(2-furylmethoxy)pyridine, di(4-chlorophenyl)pyridylmethanol, 2,3,5,6-tetrachloro-4-methylsulfonylpyridine, 2-chloro-6-trichloromethylpyridine, and sulfonylhalopyridine compounds such as 2,3,5,6-tetrachloro-4-methylsulfonylpyridine and 2,3,5-trichloro-4-(n-propylsulfonyl)pyridine. Also, 2-mercaptopyridine-N-oxide sodium or the like can be used. Among those, it is preferable to use pyridine derivatives that contain no halogen atom, and it is particularly preferable to use 2-mercaptopyridine-N-oxide sodium. If the pyridine derivative contains a halogen atom, dioxin will be generated when the antibacterial composition is subjected to incineration disposal, or, when a molding is formed from a resin that contains the antibacterial composition, the metallic mold may be corroded. Therefore, it is preferable to use pyridine derivatives that contain substantially no halogen atom.

Examples of the benzimidazole-based antibacterial agent include benzimidazole carbamate compounds, sulfur atom-containing benzimidazole compounds, and cyclic benzimidazole compound derivatives. Also, carbendazim (methyl 1H-2-benzimidazole carbamate) and thiabendazole (2-(4-thiazolyl)-1H-benzimidazole) may be used. In the present invention, it is preferable to use carbendazim (methyl 1H-2-benzimidazole carbamate) and thiabendazole (2-(4-thiazolyl)-1H-benzimidazole). The benzimidazole-based antibacterial agent does not contain halogen, so when they are subjected to incineration disposal, no dioxin is generated. Further, when a molding is formed form a resin that contains any one of those antibacterial compositions, a metallic component such as a metallic mold is not corroded.

Examples of the benzimidazole carbamate compound include methyl 1H-2-benzimidazole carbamate, methyl 1-butylcarbamoyl-2-benzimidazole carbamate, methyl 6-benzoyl-l H-2-benzimidazole carbamate, and methyl 6-(2-thiophenecarbonyl)- H-2-benzimidazole carbamate.

Further, examples of the sulfur atom-containing benzimidazole compound include 1H-2-thiocyanomethylthiobenzimidazole and 1-dimethylaminosulfonyl-2-cyano-4-bromo-6-trifluoromethylbenzimidazole.

Examples of the cyclic of benzimidazole compound derivatives include 2-(4-thiazolyl)-1 H-benzimidazole, 2-(2-chlorophenyl)-1 H-benzimidazole, 2-(1-(3,5-dimethylpyrazolyl))-1H-benzimidazole, and 2-(2-furyl)-1H-benzimidazole.

The antibacterial composition of the present invention is used as a combination of the organic antibacterial agent and the inorganic antibacterial agent. With the organic antibacterial agent is used alone, it may take a long time for the antibacterial property to be exhibited. However, by adding the inorganic antibacterial agent to the antibacterial composition, it can advantageously cope with cases where antibacterial effects must be exhibited in a short time as in textile applications, for example. That is, use of an organic antibacterial agent and an inorganic antibacterial agent in combination increases initial antibacterial performance and efficiently sustain the antibacterial performance of the antibacterial composition.

Examples of the inorganic antibacterial agent that can be used include inorganic metal compounds such as cuprous oxide, copper powder, copper thiocyanate, copper carbonate, copper chloride, copper sulfate, zinc oxide, zinc sulfate, nickel sulfate, and a copper-nickel alloy, as well as zirconium phosphate and zirconium phosphate having supported thereon a metal. In particular, it is preferable to use zirconium phosphate having supported thereon a metal such as silver or copper or zeolite. The zirconium phosphate having supported thereon a metal such as silver or copper and zeolite are preferable since they have an excellent safety to the human body, a high antibacterial rate and an excellent antibacterial performance.

Note that the organic antibacterial agent and the inorganic antibacterial agent mentioned above are all known compounds, so they can be obtained conveniently by conventional methods. Further, many of them are commercially available and such commercially available products may also be used.

Further, the antibacterial composition of the present invention may contain besides the above-mentioned organic antibacterial agent and the inorganic antibacterial agent that are essential components, conventional components (optional components) that are used in antibacterial agents as far as the effects of the present invention are prevented.

(Blend ratio of antibacterial agents)

The rates of content of the organic antibacterial agent and the inorganic microbial agent to the antibacterial composition are set such that the content of the inorganic microbial agent is preferably 0.1 to 70 mass% and particularly preferably 0.4 to 60 mass% with respect to the total antibacterial composition. If the rate of content of the inorganic antibacterial agent is less than 0.1 mass% with respect to the total antibacterial composition, the effect due to an inclusion of the inorganic antibacterial agent, such as an increase in initial antibacterial performance, can not be exhibited in some cases. On the other hand, if the rate of content of the inorganic antibacterial agent is more than 70 mass%, it is sometimes the case that overall antibacterial performance is decreased.

Further, it is preferable that the antibacterial composition of the present invention contain substantially no halogen in the organic antibacterial agent and the inorganic antibacterial agent. When the components, i.e., the organic antibacterial agent and the inorganic antibacterial agent contain substantially no halogen, the antibacterial composition itself can also be made halogen-less (non-halogen). Therefore, even when the antibacterial composition is subjected to incineration disposal, no dioxin that is a toxic substance is generated, or, when a molding is formed from a resin that contains the antibacterial composition, the metallic mold, the metal component, and the like can be advantageously prevented from corrosion.

Further, it is preferable that the antibacterial composition of the present invention contain substantially no halogen in the organic antibacterial agent a nd the inorganic antibacterial agent. When the components, i.e., the organic antibacterial agent and the inorganic antibacterial agent contain substantially no halogen, the antibacterial composition itself can also be made halogen-less (non -halogen). Therefore, even when the antibacterial composition is subjected to incineration disposal, no dioxin that is a toxic substance is generated, or, when a molding is formed from a resin that contains the antibacterial composition, the metallic mold, the metal component, and the like can be advantageously prevented from corrosion.

The antibacterial composition of the present invention can be conveniently prepared by mixing the organic antibacterial agent and the inorganic antibacterial agent by a conventional method. Further, the form of the obtained antibacterial composition is not particularly limited and the antibacterial composition can be applied in various forms such as water-like, powder-like and solvent-like forms.

Since the antibacterial composition of the present invention contains the organic antibacterial agent and the inorganic antibacterial agent in combination, the antibacterial composition has a broad antibacterial spectrum and can cope with an overwhelmingly increased number of kinds of microorganisms, thus exhibiting excellent antibacterial effects. Further, blending an inorganic antibacterial agent results in an increase in initial antibacterial performance and improvement in sustention of antibacterial effect as well as a decrease in an eluate, so environmental pollution can be advantageously prevented and also excellent safety can be obtained. Further, the antibacterial composition of the present invention is suitable for being blended in resins, so it has good resin moldability.

As mentioned above, the antibacterial composition of the present invention has a broad antibacterial spectrum and can exhibit excellent antibacterial effects such that it can cope with a large number of kinds of microorganisms. The kinds of the microorganisms (fungi, bacteria, algae and the like) on which the antibacterial composition of the present invention can exhibit antibacterial effect include, for example, those microorganisms shown in Tables 2 to 7 below (209 kinds of fungi, 148 kinds of bacteria, and 27 kinds of algae).

Further, the antibacterial composition of the present invention can exhibit an antibacterial property on those microorganisms, whose propagation can not be prevented by an individual organic antibacterial agent or an individual inorganic antibacterial agent, by a synergistic effect obtained by using both the antibacterial agents, or on those microorganisms (including algae) on which the individual components are not effective. Similarly, use of a pyridine-based antibacterial agent and a benzimidazole-based antibacterial agent in combination results in that the antibacterial composition of the present invention can exhibit antibacterial property by a synergistic effect on those microorganisms (including algae) on which the individual components are not effective. In particular, adoption of 2-mercaptopyridine-N-oxide sodium as the pyridine-based antibacterial agent and of at least one of carbendazim (methyl 1H-2-benzimidazolecarbamate) and thiabendazole (2-(4-thiazolyl)-1H-benzimidazole) as the benzimidazole-based antibacterial agent results in efficient exhibition of the antibacterial property.

Then, the antibacterial composition of the present invention can be made halogen-less (non-halogen) by using an organic antibacterial agent and an inorganic antibacterial agent that contain substantially no halogen, so even when the antibacterial composition is subjected to incineration disposal, no dioxin is generated, or, when a molding is formed from a resin that contains the antibacterial composition, the metallic mold can be prevented from corrosion.

Note that antibacterial composition of the present invention also has an effect of repelling microorganisms, so that it can exhibit an antibacterial effect without direct contact with the microorganisms or the like.

(Target articles)

The method of applying the antibacterial composition of the present invention is not particularly limited. For example, it may be applied by any method. For example, the antibacterial composition may be blended in a paint to form a coating material, the antibacterial composition may be blended in a resin material, which may then be molded, or the antibacterial composition may be applied together with a coating material such as a paint on a molding that is formed in advance to have the antibacterial composition provided with a resin molding to obtain an antibacterial molding. The resin molding or coating material imparted with such an antibacterial property can be widely applied to parts used in an environment in which microorganisms can easily propagate. Specific examples of the parts include: resin parts such as parts of an air-conditioner and car air-conditioner (preferably drain portions or the like where water tends to accumulate); inner resin portions of a washing machine, a refrigerator, a dish dryer, or the like; home appliances such as a toilet seat, a water purifier, and a cased toilet brush; textile products (apron, cloth piece, hospital service uniform, furniture cloth, curtain, and the like); water-related utensils such as a chopping board and a water-cut bag; chemical products such as adhesives and wood preservatives; building cleaners; paints for interiors and exteriors and wood surface treating agents; coating agents such as gel coating agents; interior materials for vehicles; carpets; joint sealers; sealing materials; algae-preventing agents for cooling towers; polyurethane sponges for use in baths and kitchens (for example, bath mats and washing sponges); chemical tatami mats and a tatami facing mat; waxes; and cleaners.

As mentioned above, the antibacterial composition of the present invention can be blended in resin materials, which are then molded, or applied on a molding that is formed in advance together with a coating material such as a paint to form an antibacterial molding that is composed of a resin molding provided therewith, thus providing an antibacterial molding that can advantageously exhibit the above-mentioned effect.

Here, resin materials that can constitute the antibacterial molding are not particularly limited and resin materials such as polyethylene-based resins, polypropylene-based resins, polyurethane-based resins, polycarbonate-based resins, polystyrene-based resins, polyester-based resins, acrylic resins, and polyvinyl chloride-based resins may be used singly or two or more of them may be used in combination. Also, the resin materials may be added to fiber reinforced plastics (FRP).

An antibacterial molding can be obtained, for example, by blending the antibacterial composition in the above-mentioned resin material, mixing them, integrating them by kneading or the like, and molding the resultant into a predetermined form by a known molding process such as an injection molding process, an extrusion molding process, a blow molding process, or an inflation molding process.

Here, the rate of content of the antibacterial composition to the antibacterial molding is preferably 0.01 to 10.0 mass%, and particularly preferably 0.05 to 2.0 mass% with respect to the molding. If the rate of content of the antibacterial composition to the antibacterial molding is less than 0.01 mass%, there is sometimes the case where the effect of the composition can not be imparted to the molding. On the other hand, if the rate of content of the antibacterial composition to the molding is more than 10.0 mass%, substantially no change in antibacterial property will be obtained any longer by adding more of the antibacterial composition. Further, the moldability of a molding may be influenced in some cases.

Further, the components that constitute the antibacterial composition are generally expensive, so a problem arises from the viewpoint of cost.

Note that the term "rate of content of an antibacterial composition" as used herein refers to a rate of content thereof in a layer (layers) in which the antibacterial composition is present when an antibacterial molding is a laminate in which the antibacterial composition is present in a part of the layers.

Further, in the case where the antibacterial molding is made in the form of a sheet, the antibacterial composition of the present invention is made to be contained in a single-layer sheet to impart the effect to be exhibited by the antibacterial composition to the sheet. Further, in the present invention, the sheet may be a multilayer and the antibacterial composition may be contained by a layer that appears on the outer surface (outer layer). However, the effect of the antibacterial composition can be advantageously imparted to the surface of the sheet even by arranging the layer containing the antibacterial composition such that the layer does not appear as an outer layer.

Further, it is preferable that the antibacterial composition of the present invention use a resin material that has a relatively low crystallinity. That is, the antibacterial composition that is present in the resin material can more easily exhibit antibacterial action with the resin material having a low crystallinity.

Fig. 1 shows a cross-section of a three-layer multilayer sheet 1 according to one embodiment of the antibacterial molding of the present invention. Of course, when the antibacterial composition is added to outer layers 3 of the three-layer multilayer sheet 1, the propagation of microorganisms on the outer layers 3 can be prevented. However, making the best of the repelling effect of the antibacterial composition of the present invention to the microorganisms, an intermediate layer 2 instead of the outer layers 3 may be made to contain the antibacterial composition to prevent the propagation of the microorganisms in the outer layer 3.

Note that when the intermediate layer 2 is made to contain the antibacterial composition as mentioned above, the thickness of the outer layer 3 may be 1 mm or less, preferably 300 μm or less.

Similarly, when the antibacterial molding of the present invention is in the form of a laminate structure, making a portion that corresponds to an outer layer of the molding to contain the antibacterial composition enables prevention of the growth of the microorganisms in the outer layer, and making a portion other than the outer layer (intermediate layer) of the molding to contain the antibacterial composition also enables prevention of the growth of the microorganisms in the outer layer.

Further, the antibacterial molding may be formed such that a coating film that contains the antibacterial composition on a surface of the molding made of the above-mentioned resin material is formed. As the coating film-forming resin, various known materials such as solvent-based, water-based, or UV-curing type materials, for example, urethane-based resins, acrylic resins, polyester-based resins, and vinyl-based resins may be used. Further, to coat a molding with these materials that contain the antibacterial composition, various coating methods such as spray coating, knife coating, gravure coating, flow coating, die coating, and comma coating and various printing methods such as screen printing, pad printing, and offset printing may be selected appropriately depending on the kind of the material to be used and the purpose.

Further, the antibacterial composition of the present invention may be made to be contained in aqueous resin solutions or emulsions of polyurethane-based resins, unsaturated polyester-based resins, acrylic resins, vinyl-based resins, and the like, and the resultant preparation may be used as coating materials such as a coating agent.

Note that the above-mentioned embodiment is one of embodiments of the present invention. It is needless to say that the present invention is not limited to the embodiment and variations and improvements may be embraced by the present invention as far as objects and effects of the present invention are attained. Specific structures and shapes in practicing the present invention may be replaced without problems by other structures and shapes as far as the objects and effects of the present invention are attained.

For example, in Fig. 1 as mentioned above, a three-layer multilayer sheet 1 is exemplified as an example of the antibacterial molding. However, the multilayer sheet is not limited to a three-layer one but may be a multilayer sheet of two layers, four-layers, or more. Even when the antibacterial molding is prepared as a sheet as mentioned above, it does not have to be made into a multilayer sheet but it may be made into a single-layer sheet without problems.

Although use of a pyridine-based antibacterial agent and a benzimidazole-based antibacterial agent in combination as the organic antibacterial agent is exemplified above, only the benzimidazole-based antibacterial agent may be used. Specifically, it is more preferable to use in combination two kinds or more of the benzimidazole agents, in particular, those having a thiazolyl group on the benzimidazole ring, for example, 2-(4-thiazolyl)-1H-benzimidazole and those having a carbamate group on the benzimidazole ring, for example, methyl 2-benzimidazolecarbamate. Only with the benzimidazole-based antibacterial agents, use of one having a thiazolyl group on the benzimidazole ring and one having a carbamate group on the benzimidazole ring in combination can exhibit an antibacterial property by a synergistic effect on those microorganisms (including algae) on which individual components alone have no or a low effect. In particular, use of two kinds, i.e., 2-(4-thiazolyl)-1H-benzimidazole and methyl 2-benzimidazolecarbamate results in more efficient exhibition of the antibacterial property.

Note that the benzimidazole-based antibacterial agent may be any other benzimidazole-based antibacterial agents, for example, methyl methyl-2-benzoimidazolecarbamate and methyl ethyl-2-benzimidazolecarbamate.

As for a method of applying the antibacterial composition, various forms such as powder and solutions may be used for various applications as mentioned above. For example, the antibacterial composition can be utilized as an additive to detergents such as detergents for clothes and an additive for dishes, a spray agent for clothes and furniture, an additive to lubricants for a cutting machine such as a lathe, floor waxes and cleaners as detergents, and the like.

In addition, the specific structure and shapes in practicing the present invention may be replaced by other structures and the like as far as the objects of the present invention are achieved.

(Second Embodiment)

Hereinafter, an embodiment relating to an antibacterial resin sheet, which is an antibacterial molding containing the antibacterial composition of the present invention, is explained.

The present embodiment uses at least two kinds of imidazole-based organic antibacterial agents (two kinds of the same group) alone, or an inorganic antibacterial agent in combination.

Note that a sheet-molded constitution is explained as an antibacterial molding of the present embodiment. However, the antibacterial molding is not limited to a sheet-molded one and it may be constructed in various forms and further it is not limited to a single-layer sheet but may be formed into a sheet shape of a multilayer structure. On the other hand, the antibacterial molding is not limited to a resin molding but may be applied as an inorganic molding or the like such as, for example, concrete.

(Constitution of antibacterial resin sheet)

Antibacterial resin sheets are not particularly limited in their application and can be used for various applications directly or by being bonded, stuck, or sandwiched on surfaces of portions to which they are to be attached, for example, parts or sites used in environments where microorganisms (fungi, bacteria, algae, etc.) easily propagate, specifically, wallpaper, synthetic leather, a backside of a tatami mat opposite to a tatami facing mat, and the like.

The antibacterial resin sheet is obtained, for example, by molding a resin material into a sheet by using a known molding process such as an injection molding process, an extrusion molding process, a blow molding process, or an inflation molding process.

The antibacterial resin sheet is obtained, for example, by appropriately blending and mixing the antibacterial composition of the present invention with a resin material, making the mixture substantially homogeneous by, for example, kneading to integrate the antibacterial composition with the resin, and molding the obtained resin into a form of a sheet by using a known molding process as mentioned above. It is preferable that the antibacterial resin sheet contain the antibacterial composition specifically described hereinafter in an amount of 0.01 mass% or more and 10.0 mass% or less and particularly preferably 0.05 mass% or more and 2.0 mass% or less.

Here, if the content of the antibacterial composition is less than 0.01 mass%, there is the possibility that a sufficient antibacterial property can not be exhibited. On the other hand, if the content of the antibacterial composition is more than 10.0 mass%, substantially no change in the antibacterial property is obtained. On the other hand, there is the possibility that characteristics will be influenced, for example, a decrease in the strength of the antibacterial resin sheet and deterioration of appearance such as surface smoothness, or that an inconvenience such as a decrease in workability and moldability upon molding will occur. Accordingly, to allow a sufficient antibacterial property to exhibit with a minimum content of the antibacterial composition while preventing cost from increasing due to an increase in the content of the antibacterial composition, it is preferable that the content of the antibacterial composition be 0.01 mass% or more and 10.0 mass% or less.

The resin material used for a layer to be provided on a surface side of the antibacterial resin sheet of a multilayer structure is not particularly limited and may include polyethylene-based resins, polypropylene-based resins, polyurethane-based resins, polycarbonate-based resins, polystyrene-based resins, polyester-based resins such as polyethylene terephthalate, nylon-based (polyamide-based) resins, acrylic resins, polyvinyl chloride-based resins, acrylonitrile-butadiene-styrene (ABS) resins, and the like, which may be used singly or two or more of which may be used in combination.

Note that it is preferable that those resin materials that have relatively low crystallinities are used in the case of crystalline resins. That is, the antibacterial composition that is present in the resin material can more easily exhibit antibacterial actions with the resin material having a low crystallinity.

(Antibacterial composition)

The antibacterial composition contained in the antibacterial resin sheet includes two kinds selected from imidazole-based organic antibacterial agents alone and an inorganic antibacterial agent.

The antibacterial composition exhibits antibacterial effects even at low MIC values to microorganisms (fungi, bacteria, algae, and the like) that are microorganisms shown in Tables 25 to 30 described hereinbelow, thus showing a significantly broad antibacterial spectrum.

That is, when MIC values are set to severe levels, e.g., 50 ppm or less, the antibacterial spectrum covers 214 kinds of fungi, 131 kinds of bacteria, and 27 kinds of algae (already confirmed at present time).

Examples of the imidazole-based organic antibacterial agent include benzimidazole carbamate compounds, sulfur atom-containing benzimidazole compounds, and benzimidazole cyclic compound derivatives.

Examples of the benzimidazole carbamate compound include methyl 1H-2-benzimidazole carbamate, methyl I-butylcarbamoyl-2-benzimidazole carbamate, methyl 6-benzoyl-1H-2-benzimidazole carbamate, and methyl 6-(2-thiophenecarbonyl)-1 H-2-benzimidazole carbamate.

Examples of the sulfur atom-containing benzimidazole compound include 1H-2-thiocyanomethylthiobenzimidazole and 1-dimethylaminosulfonyl-2-cyano-4-bromo-6-trifluoromethylbenzimidazole.

Examples of the benzimidazole cyclic compound derivatives of include 2-(4-thiazolyl)-1 H-benzimidazole, 2-(2-chlorophenyl)-1 H-benzimidazole, 2-(1-(3,5-dimethylpyrazolyl))-1H-benzimidazole, and 2-(2-furyl)-1 H-benzimidazole.

The imidazole-based organic antibacterial agent uses at least two kinds selected from imidazole-based organic antibacterial agents alone in combination. Even in the case of using the antibacterial agents belong to the same group, use of two different kinds of antibacterial agents can give rise to a synergistic effect in the antibacterial effect on microorganisms. In particular, it is preferable to use one having a thiazolyl group on the benzimidazole ring and one having a carbamate group on the benzimidazole ring since a significant synergistic effect can be obtained.

Examples of the thiazolyl group include 2-thiazolyl, 4-thiazolyl, and 5-thiazolyl. In addition, examples of the carbamate group include carbamate groups in which a hydrocarbon group therein is preferably an alkyl group such as a methyl group, an ethyl group, an n-2propyl group, or an iso-propyl group, and particularly preferably a methyl group or an ethyl group.

A specific example of the compound having a thiazolyl group includes 2-(4-thiazolyl)-1H-benzimidazole (Thiabendazole (TBZ)). In addition, specific examples of the compound having a carbamate group include methyl-2-benzimidazole methyl carbamate (Carbendazim (BCM)) and methyl ethyl-2-benzimidazole carbamate. It is particularly preferable that 2-(4-thiazolyl)-1H-benzimidazole and 2-benzimidazole methyl carbamate be used, because they have a relatively high heat stability, can easily be used especially as a resin molding, has already been used as a fungi-preventing agent (food additive) for grapefruit, orange, banana, or the like, and was found to be a material which provides a relatively few influence for a human body.

The imidazole-based organic antibacterial agent is preferable since it contains no halogen, so that it generates no toxic substance such as dioxin and thus gives no adverse influence on environment even when the antibacterial composition or the antibacterial resin sheet as the antibacterial molding is subjected to incineration disposal. Further, the imidazole-based organic antibacterial agent is preferable since it causes no inconvenience such as corrosion of metallic parts in a production line, such as metallic molds when an antibacterial resin sheet is molded from a resin material containing the antibacterial composition, so the production appliance requires no apparatus that are made of a special material. This readily leads to simplification of production appliance, an increase in productivity, a decrease in cost for the apparatus, and the like.

Further, the imidazole-based organic antibacterial agent is substantially insoluble in water, so it is free of the inconvenience that the antibacterial agent is flown away under use conditions such as being exposed to rains and dews, thus failing to stably provide antibacterial property for a long period of time. Further, it becomes easier to mix the imidazole-based organic antibacterial agent with the resin material well to provide a molding having an antibacterial property, and general versatility can also be increased with ease.

On the other hand, examples of the inorganic antibacterial agent that can be used include inorganic metal compounds such as cuprous oxide, copper powder, copper thiocyanate, copper carbonate, copper chloride, copper sulfate, zinc oxide, zinc sulfate, nickel sulfate, and copper-nickel alloys, and zirconium phosphate, zeolite having supported thereon a metal, or a salt thereof such as zirconium phosphate. In particular, zirconium phosphate having supported thereon silver or copper as the metal is preferable and more preferably zirconium phosphate having supported thereon silver which is a silver-based antibacterial agent having a high antibacterial property is used. Note that the silver-based antibacterial agent is not limited to a supported form but elemental metal silver may also be used.

Zirconium phosphate or zeolite having supported thereon a metal such as silver or copper is preferable since it has excellent safety to the human body, a high antibacterial rate, and excellent antibacterial performance and also it provides a reduction in cost by supporting silver, which is a precious metal, on zirconium phosphate or zeolite.

In particular, when silver-supporting zirconium phosphate or zeolite is used, it is more preferable to use zinc oxide in combination. Use of the silver-supporting zirconium phosphate or zeolite and zinc oxide in combination is preferable since antibacterial effects by the silver-supporting zirconium phosphate by itself and of zinc oxide by itself can be obtained and, simultaneously, inorganic antibacterial agents of the same inorganic group can provide a synergistic effect when used in combination, so a more significant antibacterial property can be obtained. Further, use of the silver-supporting zirconium phosphate or zeolite is preferable since its combined use with zinc oxide can decrease the content of the silver-supporting zirconium phosphate or zeolite, so that a decrease in cost due to a decreased usage of silver, which is a precious metal, can be readily obtained. Further, discoloration due to oxidation of silver can be prevented.

It is preferable that the blend ratio of the imidazole-based organic antibacterial agent to the inorganic antibacterial agent in the antibacterial composition is 1:1 to 5:1, in particular, 2:1 by mass.

Here, it is to be noted that if the blend ratio of the organic antibacterial agent to the inorganic antibacterial agent is less than 1 to 1, i.e., the organic antibacterial agent is in a smaller amount than 1:1 by mass, then there is the possibility that no broadening of the antibacterial spectrum at a low MIC value will be obtained. On the other hand, when the organic antibacterial agent is more than 5:1 by mass, the ratio of the organic antibacterial agent that has slow initial antibacterial performance and sustention of antibacterial performance of which tends to be decreased as compared with the inorganic antibacterial agent is greater, so there is the possibility that a significant antibacterial property that is stable from the beginning of use for a long period of time will not be obtained. Therefore, it is preferable that the blend ratio of the benzimidazole-based organic antibacterial agent to the inorganic antibacterial agent be set at 1:1 to 5:1 1 by mass to allow a significant a synergistic effect in an antibacterial action by use of an organic antibacterial agent and an inorganic antibacterial agent in combination as well as the antibacterial actions of the organic antibacterial agent by itself and the inorganic antibacterial agent by itself to be properly exhibited.

Further, in a case of using 2-(4-thiazolyl)-1H-benzimidazole and methyl 2-benzimidazole carbamate in combination as an imidazole-based organic antibacterial agent, the blend ratio thereof is preferably set to 1:1 to 5:1 by mass. Here, when the blend ratio of 2-(4-thiazolyl)-1H-benzimidazole to methyl 2-benzimidazole carbamate is less than 1:1 by mass or more than 5:1 by mass, the number of antibacterial spectrum capable of indicating an antibacterial action with a low MIC value may reduce, accordingly, additive amounts of the antibacterial composition may increase. For this reason, the blend ratio of 2-(4-thiazolyl)-1H-benzimidazole to methyl 2-benzimidazole carbamate is preferably set to 1: 1 to 5:1 by mass.

Further, when the silver-supporting zirconium phosphate or zeolite and zinc oxide are used in combination as the inorganic antibacterial agent, the blend ratio of the silver-supporting zirconium phosphate to zinc oxide is set at preferably 1:1 to 1:10, more preferably about 1:2.

Here, it is to be noted that if the blend ratio of the silver-supporting zirconium phosphate or zeolite to the zinc oxide is 1 to less than 1, i.e., zinc oxide is in a smaller amount than 1:1 by mass, then a sufficient cost reduction by a decrease in the amount of silver to be used will be difficult to obtain. Also, there is the possibility that discoloration due to oxidation of silver may arise. On the other hand, when zinc oxide is in a ratio of more than 1:10 by mass, there is the possibility that a sufficient antibacterial action by silver will be difficult to obtain, so addition amount of the antibacterial composition will be increased. From this, it is preferable that the blend ratio of the silver-supporting zirconium or zeolite to the zinc oxide is set to 1:1 to 1:10 by mass to properly exhibit a significant synergistic effect in an antibacterial action by the combined use.

(Action and effect of second embodiment)

According to the antibacterial composition of the present invention as mentioned above, which uses at least two kinds of imidazole-based organic antibacterial agents each containing no halogen and thus causing no skin irritation and an inorganic antibacterial agent in combination, in addition to the synergistic effect due to the combined use of the organic antibacterial agent and the inorganic antibacterial agent, a synergistic effect by use of two kinds of the organic antibacterial agents of the same imidazole group, in particular, by use of the two kinds only in combination can be obtained. Therefore, even when the antibacterial composition is attached to the skin, or when the user or manufacturer contacts a molding that contains the antibacterial composition, the antibacterial composition gives no adverse influence such as irritation to the human body. Also, the antibacterial composition generates no toxic substance such as dioxin at the time of incineration disposal, so that environmental pollution can be well prevented and an antibacterial action having no adverse influence to the human body and environment and having excellent safety can be provided. Further, in addition to the antibacterial actions by the two kinds of the imidazole-based organic antibacterial agent by themselves and by the inorganic antibacterial agent by itself, an antibacterial property as a result of the synergistic effect by combined use of an organic antibacterial agent and an inorganic antibacterial agent can be obtained on those microorganisms whose propagation can not be prevented by use of individual antibacterial agents, a significantly broad antibacterial spectrum even at low MIC values can be obtained, and high antibacterial actions can be readily and efficiently obtained.

Since the antibacterial composition of the present invention uses as the imidazole-based organic antibacterial agent two kinds, i.e., one having a thiazolyl group on the benzimidazole ring and one having a carbamate group on the benzimidazole ring in combination, antibacterial effects having no adverse influence on the human body and environment and giving a significant broad antibacterial spectrum even at low MIC values can be readily obtained from antibacterial agents of the same benzimidazole group. In particular, use of these in combination results in a significant antibacterial property.

In particular, since two kinds, i.e., one having a thiazolyl group on the benzimidazole ring, 2-(4-thiazolyl)-1H-benzimidazole and another having a carbamate group on the benzimidazole ring, methyl 2-benzimidazole carbamate are used in combination, a significant antibacterial property can be exhibited by a synergistic effect by the combined use. Further, 2-(4-thiazolyl)-1H-benzimidazole and methyl 2-benzimidazole carbamate are produced relatively easily and easily available, and are materials that have already been utilized and confirmed for their safety, so these can be readily utilized to increase general versatility.

Further, according to the antibacterial composition of the present invention, a significant antibacterial property can be readily obtained since at least one of the silver-supporting zirconium phosphate and zinc oxide that can provide a synergistic effect with the imidazole-based organic antibacterial agent is used as the inorganic antibacterial agent. In particular, use of the silver-supporting zirconium phosphate and zinc oxide in combination can provide antibacterial actions by the silver-supporting zirconium phosphate by itself and of the zinc oxide by itself and, in addition, a synergistic effect in an antibacterial action by these inorganic antibacterial agents of the same group, thus exhibiting a more significant antibacterial property. Further, use of the silver-supporting zirconium phosphate and zinc oxide in combination can decrease usage of silver, which is a precious metal, without deteriorating its antibacterial property, so that cost can be decreased more easily.

Further, as a form of using silver showing a high antibacterial property, a form is used in which silver is supported on zirconium phosphate. As a result, the antibacterial action of silver, which is a precious metal, can be exhibited with a minimum amount of silver, so the synergistic effect between the antibacterial action by the inorganic antibacterial agent and the antibacterial action by the organic antibacterial agent can be efficiently exhibited to more readily decrease cost.

(Variation of second embodiment)

Note that the above-mentioned embodiment is one of embodiments of the present invention. It is needless to say that the present invention is not limited to the embodiment and variations and improvements may be embraced by the present invention as far as objects and effects of the present invention are attained. Specific structures and shapes in practicing the present invention may be replaced without problems by other structures and shapes as far as the objects and effects of the present invention are attained.

That is, the constitution in which the antibacterial composition of the present invention is made to be contained in a molding of an antibacterial resin sheet has exemplified above. However, as mentioned above, the molding is not limited to one in the form of a sheet but may be various moldings such as a film, a fiber, an injection molding, and a blow molding and can be used for a chemical tatami mat, which is a tatami mat, wallpaper, synthetic leather, flooring material, and the like.

Note that, for example, as a tatami facing mat, there can be exemplified one that is obtained by molding a polyolefin resin having dispersed therein the above-mentioned antibacterial composition into a film by inflation molding, twisting the film like a twist of paper to prepare fibers, and interweaving the fibers into a tatami facing mat.

Further, when the antibacterial composition of the present invention is used after dispersing it in a solution substantially homogeneously, the efficiency of its contact with bacteria, fungi, algae, and the like in the solution is increased, so even solutions at a particularly low concentration, for example, solutions having a concentration of the antibacterial composition upon use of 10 ppm or more and 1,000 ppm or less can be used in practice without problems. That is, the solutions can give sufficient antibacterial effects and have excellent economical efficiency and safety.

Then, applications in which the antibacterial composition is incorporated in solutions include, for example, cooling water for a cooling tower, detergents for washing clothes and the like, or an additive to lubricants for a cutting machine such as a lathe for exhibiting antibacterial effects. Thus, the antibacterial composition of the present invention can be applied to various sites for controlling microorganisms.

Further, the antibacterial composition of the present invention can be formed into any forms including not only a sheet but also a molding for resin parts, resin fibers, and woven fabric or nonwoven fabric of the resin fibers.

Further, the antibacterial composition of the present invention can be applied not only to the resin member but also to concrete products produced by adding the antibacterial composition to freshly-mixed concrete, and to plywood laminates prepared by mixing the antibacterial composition with wood chip or the like and an adhesive and molding the resultant into a plate.

Further, the antibacterial composition can be widely applied, and specific applications thereof include: air trunks or drain portions of air-conditioners and car air-conditioners; home appliances such as a washing machine, a refrigerator, a dish dryer, a toilet seat, a water purifier, and a cased toilet brush; textile products (apron, cloth piece, hospital service uniform, furniture cloth, curtain, and the like); water-related utensils such as a chopping board, a water-cut bag, a bath mat, and a bath tub, and water-related sites such as kitchen or bath; building cleaners; paints for interiors and exteriors; interior materials for vehicles; carpets; portions of cooling water path of a cooling tower and irrigation channels; flowerbeds and vases, and the like.

Further, the antibacterial composition has been explained above as having a constitution of including two kinds of imidazole-based organic antibacterial agents alone and an inorganic antibacterial agent. However, it is needless to say that a constitution that contains unavoidably included substances is also embraced by the present invention. The present invention also embraces those constitutions having added there to additive members that function independently of respective components of the antibacterial composition without interfering the functions thereof, such as: synthetic resins that serve as base materials for the molding, solvents; magnetic powder that are utilized as a magnet; glass fibers or resin fibers for increasing the strength of the molding, such as fiber reinforced plastic (FRP); and pigments such as inks.

Note that in the case where a coating film as the antibacterial molding containing the antibacterial composition is formed by coating or dispersing, various known materials such as solvent-based, water-based, or UV-curing type materials, for example, urethane-based resins, acrylic resins, polyester-based resins, and vinyl-based resins may be used. Further, to coat a molding with these materials that contain the antibacterial composition, various coating methods such as spray coating, knife coating, gravure coating, flow coating, die coating, and comma coating and various printing methods such as screen printing, pad printing, and offset printing may be selected appropriately depending on the kind of the material to be used and the purpose.

Further, the antibacterial composition of the present invention may be made to be contained in aqueous resin solutions or emulsions of polyurethane-based resins, unsaturated polyester-based resins, acrylic resins, vinyl-based resins, and the like, and the resultant preparation may be used as coating materials such as a dipping processing agent, a fiber exhaustion processing agent, and coating agent.

Further, the imidazole-based organic antibacterial agent is not limited to 2-(4-thiazolyl)-1H-benzimidazole and methyl 2-benzimidazole carbamate, and constitutions in which various benzimidazole compositions as mentioned above are combined may be applied.

Further, respective blend ratios may be set appropriately corresponding to portions to which the antibacterial agent is to be applied or applications.

Besides, the specific structure and shapes in practicing the present invention may be replaced by other structures and the like as far as the objects of the present invention are achieved.

(Examples)

Hereinafter, the present invention is explained in more detail by way of examples, comparisons, and the like. However, the present invention should not be construed as being limited to the examples and the like.

(Examples 1 and 2 and Comparison 1) Based on the above-mentioned first embodiment, respective components of the formulation described in Table 1 were mixed to prepare antibacterial compositions of Examples 1 and 2 and Comparison 1.

Note that the constitution of Comparison 1 was such that no inorganic antibacterial agent was blended in the antibacterial composition of Example 1 and respective components were mixed in equal amounts (1/3), respectively (in Table l, rate of content was described as 33.3 mass% for descriptive purposes).

(Formulation of antibacterial composition)

[Table 1]

| | | | Example 1 | Example 2 | Comparison 1 |
|---|---|---|---|---|---|
| | | | | | (Unit: mass%) |
| Pyridine-based antibacterial agent | 2-mercaptopyridine-N-oxide sodium | | 15 | 13 | 33.3 |
| | 2,3,5,6-tetrachloro-4-methylsulfonylpyridine | | - | 13 | - |
| Benzimidazole-based antibacterial agent | Carbendazim (methyl 1 H-2-benzimidazole carbamate) | | 15 | 13 | 33.3 |
| | Thiabendazole (2-(4-thiazolyl)-1H-benzimidazole) | | 15 | 13 | 33.3 |
| Inorganic antibacterial agent | Silver-supporting zeolite | | 55 | 48 | - |

(Test Example 1)

Antibacterial performance test of antibacterial composition:
The antibacterial composition obtained in Example 1 was measured for minimum inhibitory concentration (MIC value) (ppm) and antibacterial performance thereof was evaluated.

(Test method)

(i) The antibacterial composition was diluted into predetermined concentrations (1,000 ppm, 100 ppm, 50 ppm, and the like) with dimethyl sulfoxide to prepare antibacterial agent suspensions.

(ii) In a 9-cm Petri dish, 9 ml of an agar medium sterilized in an autoclave at 121°C for 20 minutes was cast and 1 ml of the antibacterial suspension prepared in the section (i) above was added thereto and agitated. Then, the Petri dish was left to stand at room temperature to solidify the agar medium.

(iii) On the other hand, a test strain was separately diluted to $1 \times 10^6$ CFU/ml, and the resultant test strain dilution and 5 ml of a sterilized 0.9% agar medium which had been incubated at 40°C were mixed to prepare a test strain-containing agar solution.

(iv) The test strain-containing agar solution prepared in the section (iii) was overlaid on the agar medium in the section (ii) above and solidified. In an incubator, fungi were cultivated at 27°C for 72 hours and bacteria were cultivated at 30°C for 24 hours, and then their growth was confirmed. Among the media in which the test microorganism did not grow, the one having the lowest concentration of the antibacterial composition was defined as a medium containing the antibacterial composition at minimum inhibitory concentration (MIC value: ppm). Tables 2 to 7 show the results.

(Results: Fungi (1))

[Table 2]

| 類 | No. | 和名 | Latin name | MIC (単位 ppm) |
|---|---|---|---|---|
| 真菌類 | 1 | アルタルナリア アルタナータ | Alternaria alternata | 1 |
| | 2 | アルタルナリア テヌイス | Alternaria tenuis | 1 |
| | 3 | アルタルナリア ピシ | Alternaria pisi | 6 |
| | 4 | アルタルナリア カンジダス | Alternaria candidus | 2 |
| | 5 | アルタルナリア ブラシコラ | Alternaria brassicicola | 4 |
| | 6 | アスペルギルス アワモリ | Aspergillus awamori | 6 |
| | 7 | アスペルギルス ニガー | Aspergillus niger | 6 |
| | 8 | アスペルギルス オリザエ | Aspergillus oryzae | 6 |
| | 9 | アスペルギルス フラバス | Aspergillus flavus | 3 |
| | 10 | アスペルギルス バーシカラ | Aspergillus versicolor | 10 |
| | 11 | アスペルギルス フミガータス | Aspergillus fumigatus | 3 |
| | 12 | アスペルギルス ニデュランス | Aspergillus nidulans | 3 |
| | 13 | アスペルギルス グラーカス | Aspergillus glaucus | 3 |
| | 14 | アスペルギルス テレーウス | Aspergillus terreus | 8 |
| | 15 | アスペルギルス ホエニカス | Aspergillus phoenicus | 8 |
| | 16 | アスペルギルス タマリー | Aspergillus tamari | 3 |
| | 17 | アスペルギルス ウェンティ | Aspergillus wentii | 3 |
| | 18 | アスペルギルス レストリクタス | Aspergillus restrictus | 8 |
| | 19 | アスペルギルス オクラセウス | Aspergillus ochraceus | 8 |
| | 20 | アスペルギルス クラバタス | Aspergillus clavatus | 8 |
| | 21 | アスペルギルス アスタス | Aspergillus ustus | 1 |
| | 22 | アスペルギルス カンジダス | Aspergillus candidus | 1 |
| | 23 | アスペルギルス パラシティカス | Aspergillus parasiticus | 1 |
| | 24 | アスペルギルス ルーチェンシス | Aspergillus luchensis | 5 |
| | 25 | アブシディア コリムビフェラ | Absidia corymbifera | 1 |
| | 26 | アブシディア グラウカ | Absidia glauca | 5 |
| | 27 | オーレオバシディウム プルランス | Aureobasidium pullulans | 2 |
| | 28 | アスコスフェラ アピス | Ascosphaera apis | 10 |
| | 29 | アファノミセス コクリオデス | Aphanomyces cochlioides | 1 |
| | 30 | アファノミセス ラファニー | Aphanomyces raphani | 1 |
| | 31 | ボトリティス サイネラ | Botrytis cinera | 1 |
| | 32 | ビソクラミス ニベア | Byssochlamys nivea | 10 |
| | 33 | カンジダ アルビカンス | Candide albicans | 3 |
| | 34 | セレスポーラ ベティコラ | Ceraspora beticola | 1 |
| | 35 | セレスポーラ ムサオ | Cerespora musao | 1 |
| | 36 | クラビセプス パーピュレー | Claviceps purpurea | 3 |
| | 37 | コレトトリカム トリフォリー | Colletotrichum trifolii | 3 |
| | 38 | セラトシスティス コラ | Ceratocystis cora | 1 |
| | 39 | セラトシスティス アルミ | Ceratocystis ulmi | 1 |
| | 40 | ケタミウム クリバセウム | Chaetomium clivaceum | 1 |
| | 41 | ケタミウム グラボサム | Chaetomium globosum | 3 |
| | 42 | クラドスポリウム クラドスポリオイデス | Cladosporium cladosporioides | 6 |
| | 43 | クラドスポリウム サフェロスペルマム | Cladosporium sphaerospermum | 3 |
| | 44 | クラドスポリウム ハーバラム | Cladosporium herbarum | 3 |
| | 45 | クラドスポリウム レジナエ | Cladosporium resinae | 6 |
| | 46 | クラドスポリウム カルパフィーラム | Cladosporium carpophilum | 6 |
| | 47 | クルバラリア ゼニキュラータ | Curvularia geniculata | 6 |
| | 48 | クリソスポリウム サーモフィラム | Chrysosporium thermophilum | 4 |
| | 49 | シプトコッカス ネオフォーマン | Chyptococcus neoformans | 10 |
| | 50 | カンジダ ギリモンディ | Candida guilliermondii | 1 |
| | 51 | カンジダ リポリチカ | Candida lipolytica | 1 |
| | 52 | カンジダ ペリキュロー ザ | Candida pelliculose | 1 |
| | 53 | カンジダ トロピカ | Candida tropicalis | 1 |
| | 54 | カンジダ グラブラータ | Candida glabrata | 1 |
| | 55 | カンジダ アキュータス | Candida acutus | 10 |
| | 56 | カンジダ アティリス | Candida utilis | 10 |
| | 57 | コルチシウム ロルフシー | Corticium rolfsii | 1 |
| | 58 | コレトトリカム ホモイデス | Colletotrichum phomoides | 1 |
| | 59 | コレトトリカム フラガリエ | Colletotrichum fragariae | 1 |
| | 60 | コレトトリカム アトラメンタリウム | Colletotrichum arramentarium | 1 |
| | 61 | コレトトリカム リンデマチチャナム | Colletotrichum lindemuthianum | 6 |
| | 62 | クロストリジウム アセトブチリカム | Clostridium acetobutylicum | 8 |
| | 63 | クロストリジウム スポロゼネス | Clostridium sporogenes | 1 |
| | 64 | クルバラリア ルナータ | Curvularia lunata | 10 |
| | 65 | クリソスポリウム ケラチノフィラム | Chrysosporium keratinophilum | 4 |
| | 66 | ダクチリウム デルドロイデス | Dactylium derdroides | 1 |
| | 67 | ディプロディア ナタレンシス | Diplodia natalensis | 1 |
| | 68 | ドレッシェラ オストラリエンシス | Drechslera australiensis | 3 |
| | 69 | ユーロチウム タネフィラム | Eurotium tonophilum | 1 |
| | 70 | ユーロチウム レペンス | Eurotium repens | 2 |
| | 71 | ユーロチウム ルブラム | Eurotium rubrum | 2 |
| | 72 | ユーロチウム シバリエリ | Eurotium chevalieri | 1 |
| | 73 | ユーロチウム アムステロダミ | Eurotium amstelodami | 2 |
| | 74 | エピコッカム パープラセン | Epicoccum purpurascens | 2 |
| | 75 | エメリセラ ニデュランス | Emericella nidulans | 3 |
| | 76 | エキソフィアラ ジーンセルメイ | Exophiale jeanselmei | 3 |
| | 77 | フザリウム セミテクタム | Fusarium semitectum | 1 |
| | 78 | フザリウム オキシスポラム | Fusarium oxysporum | 1 |
| | 79 | フザリウム ボセウム | Fusarium voseum | 10 |
| | 80 | フザリウム モニリフォルメ | Fusarium moniliforme | 1 |

(Results: Fungi (2))

[Table 3]

EP 1 779 727 A1

(単位 ppm)　　　　　　　　　　　　　　　　　　　　　　　　　　　　　　　　(単位 ppm)

| No. | | | MIC | No. | | | MIC |
|---|---|---|---|---|---|---|---|
| 81 | フザリウム ソラニ | Fusarium solani | 8 | 121 | ペニシリウム パルプロゼラム | Penicillium purpurogenum | 1 |
| 82 | フザリウム ロゼアム | Fusarium roseum | 1 | 122 | ペニシリウム グローカム | Penicillium glaucum | 1 |
| 83 | フザリウム ニバレ | Fusarium nivale | 1 | 123 | ペニシリウム ロッケファラティ | Penicillium roquerforiti | 3 |
| 84 | フザリウム アベナシューム | Fusarium avenaceum | 1 | 124 | ペニシリウム ルテアム | Penicillium luteum | 3 |
| 85 | フザリウム アカミノタン | Fusarium acuminatum | 1 | 125 | ペニシリウム イクパンザム | Penicillium expansum | 3 |
| 86 | フザリウム プラリフェラタム | Fusarium proliferatum | 1 | 126 | ペニシリウム ビスカリム | Penicillium piscarium | 3 |
| 87 | フザリウム グラミネアラム | Fusarium graminearum | 1 | 127 | ペニシリウム ルグローザム | Penicillium rugulosum | 3 |
| 88 | フィマトリカム オムニバラン | Fhymatotricum omnivorum | 4 | 128 | ペニシリウム シクロピアム | Penicillium cyclopium | 3 |
| 89 | ゲオトリカム カンディダム | Geotricham candidum | 3 | 129 | ペニシリウム クリソゲナム | Penicillium chrysosgenum | 3 |
| 90 | ゲオトリカム ラクタス | Geotricham lactus | 6 | 130 | ペニシリウム シトレオビリデ | Penicillium citreo—viride | 10 |
| 91 | エルミンソスポリウム スピーシーズ | Helminthosporium sp. | 6 | 131 | ペニシリウム ナタタム | Penicillium notatum | 3 |
| 92 | エルミンソスポリウム グラミム | Helminthosporium gramineum | 20 | 132 | ペニシリウム ルブラム | Penicillium rubrum | 3 |
| 93 | オモデラドラム ペドロシ | Hormoderdrum pedrosoi | 3 | 133 | ペニシリウム オクザリカム | Penicillium oxalicum | 8 |
| 94 | レンズィティス トラバ | Lenzites trabea | 8 | 134 | ペニシリウム ファニキュロザム | Penicillium funiculosum | 10 |
| 95 | レンティナス レピデンス | Lentinus lepideus | 8 | 135 | ペニシリウム ディジタータム | Penicillium digitatum | 10 |
| 96 | メデュレラ マイセトミィ | Medurella mycetomii | 4 | 136 | ペニシリウム イスランディカム | Penicillium islandicum | 20 |
| 97 | ミクロスポラム カニス | Microsporum canis | 3 | 137 | ペニシリウム ニグリカンス | Penicillium nigricans | 3 |
| 98 | ミクロスポラム ジプセーム | Microsporum gypseum | 1 | 138 | ペニシリウム リラシナム | Penicillium lilacinum | 20 |
| 99 | ミクロスポラム オドウニ | Microsporum audouini | 10 | 139 | ペニシリウム スピヌロザム | Penicillium spinulosum | 3 |
| 100 | ムコール ラセマウセス | Mucor racemosus | 8 | 140 | ペニシリウム ステッキイ | Penicillium steckii | 3 |
| 101 | ムコール ムセド | Mucor mucedo | 4 | 141 | ペスタロティア アダスタ | Pestalotia adusta | 20 |
| 102 | ムコール パシラス | Mucor pusillus | 4 | 142 | ペスタロティア ネグレクタ | Pestalotia neglecta | 10 |
| 103 | ムコール スピネッセンス | Mucor spinescens | 1 | 143 | ホモプシス シトリ | Phomopsis citri | 3 |
| 104 | ムコール ルキシー | Mucor rouxii | 2 | 144 | ホモプシス フクシー | Phomopsis fukushii | 1 |
| 105 | ミロテシウム バルカリア | Myrothecium verrucaria | 4 | 145 | ホモプシス ベーサン | Phomopsis vexan | 1 |
| 106 | モナスカス ルバー | Monascus ruber | 6 | 146 | フォーマ シトリカルパ | Phoma citricarpa | 3 |
| 107 | モニリア カンディダ | Monilia candida | 1 | 147 | フォーマ テレスチアス | Phoma terrestius | 3 |
| 108 | モニリア フルクティガナ | Monilia fructigena | 10 | 148 | フォーマ グロミタラ | Phoma glomerata | 3 |
| 109 | モニリア ニグラ | Monilia nigral | 1 | 149 | フォーマ ピグメンティバロ | Phoma pigmentivara | 3 |
| 110 | モニリア ラクサ | Monilia laxa | 1 | 150 | ピッチア ミムブラナファシエンス | Pichia membranaefaciens | 20 |
| 111 | メノニエラ エキニータ | Menoniella echinita | 6 | 151 | ペプトコッカス スピーシーズ | Peptococcus sp. | 10 |
| 112 | ニューロスポラ クラッサ | Neurospora crassa | 10 | 152 | プロテウス ミラビリス | Proteus mirabilis | 1 |
| 113 | ニューロスポラ シトフィラ | Neurospora sitophila | 3 | 153 | ファシディフィクナス ファンフランセ | Phacidipycnus funfuracea | 6 |
| 114 | ニグロスポラ オリザエ | Nigrospora oryzae | 1 | 154 | ピシウム ドバリアナム | Pythium debaryanum | 1 |
| 115 | ニグロスポラ スフェリカ | Nigrospora sphaerica | 6 | 155 | ピシウム イレギュラレ | Pythium debaryanum | 1 |
| 116 | オキューレマニアム チャルティカラ | Ocuremonium charticola | 20 | 156 | ピシウム アファニデルマタム | Pythium aphanidermatam | 1 |
| 117 | プルラリア プルランス | Pullularia pullulans | 1 | 157 | フィトフィトラ メガスペルマ | Phytophthora megasperma | 1 |
| 118 | ペニシリウム フリークェンタス | Penicillium frequentance | 1 | 158 | フィトフィトラ ニコチアネ | Phytophthora nicotianae | 1 |
| 119 | ペニシリウム シトリナム | Penicillium citrinum | 3 | 159 | フィトフィトラ インフェタンス | Phytophthora infestans | 1 |
| 120 | ペニシリウム バリアブル | Penicillium variabile | 1 | 160 | フィトフィトラ カプシン | Phytophthora capsici | 1 |

真
菌
類

(Results: Fungi (3))

EP 1 779 727 A1

[Table 4]

(単位 ppm)

| | No. | | | MIC |
|---|---|---|---|---|
| 真菌類 | 161 | プラスモディオフォラ ブラシカエ | Plasmodiophora brassicae | 1 |
| | 162 | ピレノケーター リカパーバシ | Pyrenochaeta lycopersici | 1 |
| | 163 | ロドトルラ ミヌタ | Rhodotorula minuta | 8 |
| | 164 | ロドトルラ ムシラジナーサ | Rhodotorula muchilaginosa | 8 |
| | 165 | ロドトルラ テキセンシス | Rhodotorula texensis | 8 |
| | 166 | ロドトルラ グルチニス | Rhodotorula glutinis | 8 |
| | 167 | ロドトルラ ガリニス | Rhodotorula gulinis | 20 |
| | 168 | ロドトルラ ラクトーサ | Rhodotorula lactosa | 20 |
| | 169 | リゾプス ニグリカンス | Rhizopus nigricans | 3 |
| | 170 | リゾプス オリザエ | Rhizopus oryzae | 3 |
| | 171 | リゾプス ストロニファー | Rhizopus storonifer | 3 |
| | 172 | リゾプス デレマ | Rhizopus delemar | 8 |
| | 173 | リゾプス アラニ | Rhizopus solani | 3 |
| | 174 | リゾプス ジャバニカス | Rhizopus javanicus | 8 |
| | 175 | スポロトリカム シェンキー | Sporotrichum shenki | 10 |
| | 176 | スティコッカス バシラビス | Stichococcus bacillavis | 10 |
| | 177 | スクレロチニア フルクティカラ | Sclerotinia fructincola | 10 |
| | 178 | サッカロマイカダス パスタリアナス | Saccharomycodes pasteurianus | 3 |
| | 179 | スタキボトリス スピーシーズ | Stachybotrys sp. | 3 |
| | 180 | スパイカリア ボラシー | Spicaria violacea | 3 |
| | 181 | スコレコバシディウム カンストリクタム | Scolecobasidium constrictum | 3 |
| | 182 | セドスポリウム アピオスペルマ | Scedosporium apiospermum | 10 |
| | 183 | シンセファラストラム ラセモサム | Syncephalastrum racemosum | 3 |
| | 184 | スタキボトリス チャルトラム | Stachybotrys chartrum | 3 |
| | 185 | スポロスリックス シェンキー | Sporothrix schenckii | 1 |
| | 186 | スクレロチウム セピボラム | Sclerotium cepivorum | 1 |
| | 187 | スファエロセカ フムリ | Sphaerotheca humuli | 1 |
| | 188 | スクレロチニア スクレロチオラム | Sclerotinia sclerotiorum | 1 |
| | 189 | スコプラリオプシス ブレビカウリス | Scopulariopsis brevicaulis | 10 |
| | 190 | トリコヒィートン ミンタグルフィテス | Trichophyton mentagrophytes | 3 |
| | 191 | トリコヒィートン ジプセーム | Trichophyton gypseum | 10 |
| | 192 | トリコヒィートン ルブラム | Trichophyton rubrum | 1 |
| | 193 | トリコテシウム ロゼアム | Trichothecium roseum | 3 |
| | 194 | トリコフィートン アジェロイ | Trichophyton ajelloi | 1 |
| | 195 | トリコデルマ ビリデ | Trichoderma viride | 6 |
| | 196 | トリコデルマ コニンギー | Trichoderma koningii | 3 |
| | 197 | トリコデルマ T-1 | Trichoderma T-1 | 1 |
| | 198 | トリコデルマ ハルジアナム | Trichoderma harzianum | 6 |
| | 199 | トリコデルマ リグノラム | Trichoderma lignorum | 1 |
| | 200 | タラロプス カンジダ | Torulopsis candida | 6 |

(単位 ppm)

| No. | | | MIC |
|---|---|---|---|
| 201 | トリコスポルラム ククネウム | Trichosporum cutaneum | 1 |
| 202 | ウロクラディウム アトラム | Ulocladium atrum | 1 |
| 203 | ウスティラーゴ ゼア | Ustilago zeae | 10 |
| 204 | ヴェンティシリウム アルボアトラム | Verticillium albo-atrum | 10 |
| 205 | バーティシリウム ダーリエ | Verticillium dahliae | 1 |
| 206 | ワレミア セビ | Wallemia sebi | 1 |
| 207 | オオツヅラタケ | Tyromyces palustris | 1 |
| 208 | カワラタケ | Coriolus versicolor | 1 |
| 209 | ナミダタケ | Serpula lacrymans | 1 |

22

(Results: Bacteria (1))

[Table 5]

(単位 ppm)　　　　　　　　　　　　　　　　　　　　　　　　　(単位 ppm)

| No. | | | MIC | No. | | | MIC |
|---|---|---|---|---|---|---|---|
| 1 | アルカリゲネス フェカリス | Alcaligenes faccalis | 1 | 41 | フソバクテリウム ヌクレアタム | Fusobacterium nucleatum | 1 |
| 2 | アルカリゲネス ビスコラクティス | Alcaligenes viscolactis | 1 | 42 | フラボバクテリウム アミノゲン | Flavobacterium aminogenes | 10 |
| 3 | アスコフィーダ ピシ | Ascophyta pisi | 10 | 43 | フラボバクテリウム メニンガセプティカム | Flavobacterium meningosepticum | 1 |
| 4 | オートトロピック バクテリア | Autotrophic bacteria | 20 | 44 | グラコノバクター サバキダン | Gluconobacter suboxydans | 10 |
| 5 | アスタ エロウス | Aster yellows | 1 | 45 | ハンセヌラ アノマーラ | Hansenula anomala | 10 |
| 6 | アキネトバクター カリカセティカス | Acinetobacter calcoaceticus | 4 | 46 | クレブシエラ オキシカ | Klebsiella oxytoca | 10 |
| 7 | アカクロモバクター ガルダタス | Achromobacter gulvatus | 1 | 47 | クレブシエラ ニューモニエ | Klebsiella pneumoniae | 3 |
| 8 | アエロバクター アエロゼネス | Aerobacter aerogenes | 1 | 48 | ラクトバシルス アシドフィラス | Lactbacillus acidophilus | 8 |
| 9 | アエロバクター クローカエ | Aerobacter cloacae | 1 | 49 | ラクトバシルス プランタラム | Lactbacillus plantarum | 10 |
| 10 | ブラストマイセス イタリーカム | Blastomyces italicum | 1 | 50 | ラクトバシルス ブルガリクス | Lactbacillus bulgericus | 1 |
| 11 | バチルス セレウス | Bacillus cereus | 1 | 51 | リステリア モノシトゲネス | Listeria monoxytogenes | 10 |
| 12 | バチルス ミカイデス | Bacillus mycoides | 1 | 52 | レジオネラ ニューモフィラ | Legionella pneumophila | 1 |
| 13 | バチルス ズブチルス | Bacillus subtilis | 10 | 53 | レプトスピラ インテロガン | Leptospira interrogans | 10 |
| 14 | バチルス メガテリウム | Bacillus megaterium | 10 | 54 | レピタ クリストタ | Lepiota cristata | 1 |
| 15 | バチジス アンスラシス | Bacillus anthracis | 10 | 55 | レピタ カスタニー | Lepiota castanae | 1 |
| 16 | バチルス パンクタタン | Bacillus punctatum | 10 | 56 | ミクロコッカス グラタミカス | Micrococcus glatamicus | 15 |
| 17 | バクテリウム バルガ | Bacterium vulgare | 1 | 57 | ミクロコッカス カゾリティカス | Micrococcus caseolyticus | 80 |
| 18 | バクテリウム ロシアナム | Bacterium pyocyaneum | 1 | 58 | ミクロコッカス フォーゼン | Micrococcus pyogenes | 80 |
| 19 | ブラストマイセス ダマティアス | Blastomyces desmatidis | 1 | 59 | ミクロコッカス カンジダス | Micrococcus candidus | 80 |
| 20 | バクテロイド フラギリス | Bacteroid fragilis | 3 | 60 | ミクロコッカス アルバス | Micrococcus albus | 10 |
| 21 | カンピロバクター フィタス | Campylobacter fetus | 3 | 61 | ミクロコッカス アクイリス | Micrococcus aquilis | 10 |
| 22 | カンピロバクター ジュジュニ／コリ | Campylobacter jejuni／coli | 10 | 62 | ミクロコッカス コングロメラテス | Micrococcus conglomerates | 10 |
| 23 | クロストリジウム パーフリゲンス | Clostridium perfringens | 3 | 63 | ミクロコッカス バリアンス | Micrococcus varians | 10 |
| 24 | クロストリジウム ディフィシル | Clostridium difficile | 3 | 64 | ミクロバクテリウム ツベルクランス | Microbacterium tuberculosis | 15 |
| 25 | クロストリジウム ボトリジウム | Clostridium botulinum | 3 | 65 | パエシロミセス リカシナス | Paecilomyces lilacinus | 10 |
| 26 | カルティシアム ファシフォルメ | Corticium luciforme | 3 | 66 | パエシロミセス バリオティ | Paecilomyces variotti | 2 |
| 27 | クロエケラ アピキュレータ | Cloechera apiculata | 10 | 67 | パディオコッカス ソアエ | Pediococcus soyae | 10 |
| 28 | セルロモナス イウジス | Cellulomonas iugis | 1 | 68 | パディオコッカス アキライデティ | Pediococcus acidilactici | 10 |
| 29 | ダクティリウム デンドロイダス | Dactylium dendroides | 3 | 69 | シュードモナス エレギノーザ | Pseudomonas aeruginosa | 25 |
| 30 | ダイプロティア バイティコラ | Diplodia viticol | 3 | 70 | シュードモナス フルレセウス | Pseudomonas fluresceus | 3 |
| 31 | デバリアマイセス ハンシニー | Debaryamyces hansenii | 15 | 71 | シュードモナス シリンガ | Pseudomonas syringae | 3 |
| 32 | デザルホビブリオ デザルホリカンス | Desulfovibrio desulfuricans | 1 | 72 | シュードモナス ソラナセラム | Pseudomonas solanacearum | 3 |
| 33 | エンダチア パラシティカ | Endothia parasitica | 1 | 73 | ファフィア ロドシーマ | Phaffia rhodozyma | 10 |
| 34 | エスケリチア コリ | Escherichia coli | 15 | 74 | ピッチア アノモラ | Pichia anomala | 10 |
| 35 | エンテロバクター アエロゲネス | Enterobacter aerogenes | 1 | 75 | ピッチア メンブラナファシエンス | Pichia membranaefaciens | 10 |
| 36 | エンテロバクター クローケ | Enterobacter clocae | 10 | 76 | プロテウス ベルガリ | Proteus vulgaris | 15 |
| 37 | エンテロコッカス フィカリス | Enterrococcus faecalis | 80 | 77 | ピシウム ヴァンテルプーリー | Pythium vanterpoolii | 1 |
| 38 | エンテロコッカス フェシウム | Enterrococcus faecium | 80 | 78 | リジシウム シネレウム | Phyrasium cinereum | 1 |
| 39 | エンテロコッカス ニストリティカ | Enterrococcus nistolytica | 80 | 79 | プロピオバクテリウム アセン | Propionibacterium aces | 1 |
| 40 | エルウィニア カロトボラ | Erwinia carotovora | 1 | 80 | プロピオニバクテリウム セルマニー | Propionibacterium shermanii | 1 |

細菌類

24

(Results: Bacteria (2))

[Table 6]

(単位 ppm)

| No. | | | MIC |
|---|---|---|---|
| 81 | ロドスフェラ ルコトリカ | Podosphaera leucotricha | 1 |
| 82 | パラコラバクトラム アエロジェナイデス | Paracolabactrum aerogenoides | 1 |
| 83 | リゾクトニア バイオラシー | Rhizoctonia violacea | 1 |
| 84 | リゾクトニア ソラニ | Rhizoctonia solani | 1 |
| 85 | リケッチア リケッチー | Rickettsia rickettsii | 1 |
| 86 | ラミノコッカス | Ruminococcus | 1 |
| 87 | スクレロヴィーナ スクレチオラム | Scleotina scleotiorum | 1 |
| 88 | スポロボロマイセス ロシュー | Sporobolomyces roseus | 10 |
| 89 | ストレプトコッカス ラクティス | Streptococcus lactis | 10 |
| 90 | ストレプトコッカス ファエカリス | Streptococcus faecalis | 120 |
| 91 | ストレプトコッカス サーモフィラス | Streptococcus thermophilis | 120 |
| 92 | ストレプトコッカス アガラクティー | Streptococcus agalactiae | 8 |
| 93 | ストレプトマイセス グリシー | Streptomyces griseus | 120 |
| 94 | ストレプトマイセス オーレオファシエンス | Streptomyces aureofaciens | 120 |
| 95 | ストレプトマイセス カスガエンシス | Streptomyces kasugaensis | 120 |
| 96 | ストレプトマイセス ルブリレティキュリ | Streptomyces rubrireticuli | 60 |
| 97 | スキザサッカロマイセス ポンブ | Schizosaccharomyces pombe | 10 |
| 98 | サッカロマイコデス ラドウィギー | Saccharomycodes ludwigii | 10 |
| 99 | セラティア マルセセン | Serratia marcesens | 10 |
| 100 | セラティア リグファシエンス | Serratia liguefaciens | 1 |
| 101 | セラティア サリナリア | Serratia salinaria | 1 |
| 102 | スタフィロコッカス アウレス | Staphylococcus aureus | 10 |
| 103 | スタフィロコッカス フェカリス | Staphylococcus faecalis | 5 |
| 104 | スタフィロコッカス エピデルミディス | Staphylococcus epidermidis | 1 |
| 105 | スタフィロコッカス オミニス | Staphylococcus hominis | 1 |
| 106 | スタフィロコッカス アガラクティア | Staphylococcus agalactiae | 1 |
| 107 | スタフィロコッカス ニューモニエ | Staphylococcus pneumoniae | 1 |
| 108 | スタフィロコッカス ポジエン | Staphylococcus pyogenes | 1 |
| 109 | ストレプトベルティシラム レティカルム | Streptoverticillum reticulum | 5 |
| 110 | サルモネラ タフィマリウム | Salmonella typhimurium | 1 |
| 111 | サルモネラ エンテリティディス | Salmonella enteritidis | 3 |
| 112 | サルモネラ エンテリカ | Salmonella enterrica | 3 |
| 113 | サルモネラ アリゾナエ | Salmonella arizonae | 3 |
| 114 | サルモネラ パラティフィ | Salmonella paratyphi | 3 |
| 115 | サルモネラ コレランシス | Salmonella choleraesuis | 3 |
| 116 | サルモネラ タイフォサ | Salmonella typhosa | 1 |
| 117 | サッカロマイセス セレビジエ | Saccharomyces cerevisiae | 10 |
| 118 | サッカロマイセス ルキシー | Saccharomyces rouxii | 60 |
| 119 | サッカロマイセス パストリアヌス | Saccharomyces pasteurianus | 60 |
| 120 | サジェラン モザイク | Sugaran mosaic | 1 |

(単位 ppm)

| No. | | | MIC |
|---|---|---|---|
| 121 | サルチナ フレイバ | Sarcina flava | 1 |
| 122 | サルチナ ルテア | Sarcina lutea | 1 |
| 123 | スポロサイトファガ ミクソコッコイデス | Sporocytohaga myxococcoides | 1 |
| 124 | トルラ ニグラ | Torula nigra | 1 |
| 125 | サーモアクチノマイセス ブルガリス | Thermoactinomyces vulgaris | 1 |
| 126 | チオバチルス アシドフィルス | Thiobacillus acidophilus | 1 |
| 127 | チオバチルス デリカタス | Thiobacillus delicatus | 1 |
| 128 | チオバチルス デニトリフィカンス | Thiobacillus denitrificans | 1 |
| 129 | チオバチルス フェルオキシダンス | Thiobacillus ferrooxidans | 1 |
| 130 | チオバチルス インテレメディアス | Thiobacillus intermedius | 1 |
| 131 | チオバチルス カバリス | Thiobacillus kabobis | 1 |
| 132 | チオバチルス ナポリタンス | Thiobacillus neapolitans | 1 |
| 133 | チオバチルス ノベラス | Thiobacillus novellus | 1 |
| 134 | チオバチルス ペロメタボリス | Thiobacillus perometabolis | 1 |
| 135 | チオバチルス ルベラス | Thiobacillus rubellus | 1 |
| 136 | チオバチルス チオキシダンス | Thiobacillus thiooxidans | 1 |
| 137 | チオバチルス チオパラス | Thiobacillus thioparus | 1 |
| 138 | チオバチルス タソフィリカ イシュネスキ | Thiobacillus thermophilica imschnetskii | 1 |
| 139 | チオバチルス バスタス | Thiobacillus versutus | 1 |
| 140 | ベンチュリア イナクアリス | Venturia inaequalis | 1 |
| 141 | ビブレオ アルニフィカス | Vibrio ulnificus | 1 |
| 142 | ビブレオ パラハーモリティカス | Vibrio parahaemolyticus | 80 |
| 143 | ビブレオ フルビアリス | Vibrio fluvialis | 80 |
| 144 | ビブレオ ミミカス | Vibrio mimicus | 80 |
| 145 | ビブレオ オマ | Vibrio omma | 80 |
| 146 | エルシア エンテロコリティカ | Yersinia enterocolitica | 1 |
| 147 | ジゴサッカロマイセス ブラリー | Zygosaccharomyces bailii | 120 |
| 148 | ジゴサッカロマイセス ルーキー | Zygosaccharomyces rouxii | 120 |

細菌類

(Results: Algae)

[Table 7]

| No. | 実施例 | MIC (単位 ppm) |
|---|---|---|
| 1 | Anacystis nidulans アナキスティス ニデュランス | 10 |
| 2 | Anacystis montana アナキスティス モンタナ | 10 |
| 3 | Anacystis thermale アナキスティス サーマリ | 10 |
| 4 | Anabaena sp. アナベナ スピーシーズ | 10 |
| 5 | Ankistrodesmus angustus アンキストロデスムス アングスタス | 10 |
| 6 | Batrachospermum sp. バトラコスペルマム スピーシーズ | 10 |
| 7 | Chlorella vulgaris クロレラ バルガリス | 10 |
| 8 | Cladophora glomerata クラドフォラ グロメラタ | 10 |
| 9 | Chlamydomonas reinhardii クラミドモナス レインハルディ | 10 |
| 10 | Chlorococcum sp. クロロコッカム スピーシーズ | 10 |
| 11 | Calothrix parietina カロスリックス パリエティナ | 10 |
| 12 | Cylindrocapsa sp. シリンドロカプサ スピーシーズ | 10 |
| 13 | Chlorella emersonii クロレラ エマーソニ | 10 |
| 14 | Hormidium sp. ホルミディウム スピーシーズ | 10 |
| 15 | Hildenbrandia sp. ヒルデンブランディア スピーシーズ | 10 |
| 16 | Mesotaenium sp. メソテニウム スピーシーズ | 10 |
| 17 | Nostocales sp. ノストカレス スピーシーズ | 10 |
| 18 | Navicula sp. ナビキュラ スピーシーズ | 10 |
| 19 | Oscillatoria lutea オッシラトリア ルーテア | 10 |
| 20 | Pleurococcus sp. プレウロコッカス スピーシーズ | 10 |
| 21 | Scytonema hofmanii シトネマ ホフマニ | 10 |
| 22 | Schizothrix sp. シゾスリックス スピーシーズ | 10 |
| 23 | Tribonema sp. トリボネマ スピーシーズ | 10 |
| 24 | Trentepohlia odorata トレンテポーリア オドラタ | 10 |
| 25 | Trentepohlia aurea トレンテポーリア アウレア | 10 |
| 26 | Ulotrichaceae sp. ウロトリカセエ スピーシーズ | 10 |
| 27 | Zygogonium sp. ジゴゴニウム スピーシーズ | 10 |

Results in Tables 2 to 7 indicate that the antibacterial composition of Example 1 had MIC values within the range of 10 to 120 ppm on all the test microorganisms (fungi, bacteria, and algae) and could prevent propagation of each test microorganism at extremely low concentrations. Thus, it was confirmed that the antibacterial composition of Example 1 had a broad antibacterial spectrum and could effectively cope with a wide variety of microorganisms.

(Test Example 2)

Textile test:

The antibacterial compositions obtained in Example 1 and Comparison 1 were subjected to textile tests according to the test method described hereinbelow, and antibacterial performance relative to a general textile product was compared and evaluated.

Results in are shown in Table 8.

(Test method)

The test was performed as described in the following sections (i) to (iii) according to JIS L1902 (1998). As the test strain, *Staphylococcus aureus* was used.

(i) Preparation of textile sample

The antibacterial composition of Example 1 or Comparison 1 was added and dispersed in a one-pack polyurethane resin (Dainichiseika Color & Chemicals Mfg. Co., Ltd.) in an amount of 0.5 mass% on a dry weight basis.

Then, the obtained polyurethane resin was applied on a release paper by using a bar coater or a knife coater and dried at 80°C to prepare a 10-$\mu$m-thick polyurethane film containing 0.5 mass% of the antibacterial composition. The polyurethane film was affixed to a polyester texture with a two-pack reaction-curing-type polyurethane adhesive to obtain a textile sample having a size of 100 mm x 100 mm.

Note that a textile sample containing no antibacterial composition was also prepared as a blank.

(ii) Then, the textile sample containing the antibacterial composition of Example 1 or Comparison 1 (hereinafter, also referred to as "textile sample of Example 1 (or Comparison 1" for descriptive purposes) prepared in the section (i) above and the blank textile sample were charged in a liquid medium containing *Staphylococcus aureus,* respectively, and then cultivation was carried out in an incubator for 18 hours. After the completion of the cultivation, the number of cells was counted.

(iii) Here, the antibacterial performance was evaluated by calculating an antibacterial activity a by the following equation (I). Note that when the antibacterial activity a is larger than 0, the antibacterial composition is considered to have an antibacterial effect. The calculated antibacterial activities of the textile samples of Example 1 and Comparison 1 are shown in Table 8.

(Numeral 1)

$$\text{Antibacterial activity a} = \log_{10}A - \log_{10}C \quad ... (I)$$

A: Number of cells before cultivation of blank textile sample;
C: Number of cells after cultivation of textile sample of Example 1 or Comparison 1.

(Antibacterial activity)

[Table 8]

| Textile sample | Antibacterial activity a |
|---|---|
| Example 1 | 3.20 |
| Comparison 1 | -0.65 |

Results in Table 8 indicate that the textile sample provided with the antibacterial composition of Example 1 containing zirconium phosphate that is an inorganic antibacterial agent (textile sample of Example 1) showed a clear antibacterial activity (3.20). It was confirmed that the antibacterial composition of Example 1 could exhibit antibacterial performance in a short period of time (within 18 hours) even in ordinary textile products.

On the other hand, the textile sample provided with the antibacterial composition of Comparison 1 containing no inorganic antibacterial agent (textile sample of Comparison 1) had an antibacterial activity of less than 0 (-0.65) and showed no antibacterial activity.

(Test Example 3)

Antibacterial performance test for molding (sheet):

As a molding containing the antibacterial composition of Example 1, a sheet of the following constitution was prepared. Then, the antibacterial performance of the sheet on test microorganisms shown in Table 10 was confirmed by the following test method and criteria of judgment. Results are shown in Table 11.

(Constitution of sheet)

In the multilayer sheet 1 shown in Fig. 1, a material containing 0.05 mass% of the antibacterial composition of Example 1 based on a polypropylene resin (F744NP: manufactured by Idemitsu Petrochemical Co., Ltd. (now, Idemitsu Kosan Co., Ltd.)) in the intermediate layer 2 was extruded through a T-die as the intermediate layer 2 and the above-mentioned polypropylene resin was extruded as it was through a T-die as the outer layers 3 on both sides of the intermediate layer 2 to prepare a three-layer sheet made of a polypropylene resin.

Note that the intermediate layer 2 had a thickness of 100 $\mu$m and the outer layers 3 on both sides of the intermediate layer 2 each had a thickness of 20 $\mu$m (this is named "sheet of Example 1-a").

Further, a sheet prepared in the same manner as that of the sheet of Example 1-a except that the rate of content of the antibacterial composition in the intermediate layer 2 in the sheet of Example 1-a was changed to 0.1 mass%, and the sheet was named a "sheet of Example 1-b". Similarly, a sheet prepared in the same manner as that of the sheet of Example 1-a except that the rate of content of the antibacterial composition in the intermediate layer 2 in the sheet of Example 1-a was changed to 0.5 mass%, and the sheet was named a "sheet of Example 1-c".

Then, a 100-$\mu$m-thick single-layer sheet was prepared by extrusion molding of a material containing 0.5 mass% of the antibacterial composition of Example 1 with respect to a polyethylene resin (Moretech 0138: manufactured by Idemitsu Petrochemical Co., Ltd.) (This was named a "sheet of Example 1-d").

Further, as a blank, a 100-$\mu$m-thick single-layer sheet made of the above-mentioned polypropylene resin and containing no antibacterial composition was also prepared (this was named a "sheet of Reference Example").

(Test method)

(i) Preparation of inorganic salt medium:

An inorganic salt medium having the constitution shown in Table 9 was prepared. After being sterilized in an autoclave at 121°C for 20 minutes, the medium was adjusted to pH 6.0 to 6.5 with an aqueous caustic soda solution (aqueous NaOH solution).

(Inorganic salt medium)

[Table 9]

| Component | Blend amount |
|---|---|
| $KH_2PO_4$ | 0.7g |
| $K_2HPO_4$ | 0.7g |
| $MgSO_4 \cdot 7H_2O$ | 0.7g |
| $NH_4NO_3$ | 1.0g |
| NaCl | 0.005g |
| $FeSO_4 \cdot 7H_2O$ | 0.002g |
| $ZnSO_4 \cdot 7H_2O$ | 0.002g |
| $MnSO_4 \cdot 7H_2O$ | 0.001 g |
| Agar | 15.0g |
| Pure water | 1,000ml |

(ii) Preparation of mixed spore solution:

Spores of fungi of strains shown in Table 10 below were suspended in sterilized water and filtered to prepare a mixed spore solution having a concentration of about $1 \times 10^6$ cell/ml. Note that to suspend the spores, dispersion of spores was performed with sodium laurylsulfate.

(Kinds of strain)

[Table 10]

| 0. Alternaria alternata アルタナリア アルタナータ | 25. Eurotium rybrum ユーロチウム ルブラム | 49. Penicillium expansum ペニシリウム イクスパンザム |
|---|---|---|
| 0. Aspergillus niger アスペルギルス ニガー | 26. Eurotium chevalieri ユーロチウム シバリエリ | 50. Penicillium cyclopium ペニシリウム シクロピア |
| 0. Aspergillus oryzae アスペルギルス オリゼ | 27. Eurotium amstelodami ユーロチウム アムステロダミ | 51. Penicillium citreo-viride ペニシリウム シトレオビリデ |
| 0. Aspergillus flavus アスペルギルス フラバス | 28. Fusarium semitectum フザリウム セミテクタム | 52. Penicillium funiculosum ペニシリウム ファニキュラザム |
| 0. Aspergillus versicolor アスペルギルス バーシカラ | 29. Fusarium oxysporum フザリウム オキシスポラム | 53. Penicillium nigricans ペニシリウム ニグリカンス |
| 0. Aspergillus humigatus アスペルギルス フミガータス | 30. Fusarium solani フザリウム ソラニ | 54. Penicillium lilacinum ペニシリウム リラシナム |
| 0. Aspergillus terreus アスペルギルス テレーズ | 31. Fusarium roseum フザリウム ロゼアム | 55. Pestalotia adusta ペスタロチア アグスタ |
| 0. Aspergillus restrictus アスペルギルス レストリクタス | 32. Fusarium moniliforme フザリウム モニリフォルメ | 56. Pestalotia neglecta ペスタロチア ネグレクタ |
| 0. Aspergillus ochraceus アスペルギルス オクラシアス | 33. Fusarium proliferatum フザリウム プロリフェラタム | 57. Phoma citricarpa フォーマ シトリカルパ |
| 0. Aspergillus candidus アスペルギルス カンジダス | 34. Geotrichum candidum ゲオトリカム カンディダム | 58. Phoma terrestrius フォーマ テレストリアス |
| 11. Alternaria tenuis アルタルナリア テヌイス | 35. Geotrichum lactus ゲオトリカム ラクタス | 59. Phoma glomerata フォーマ グロミラタ |
| 12. Alcaligenes faecalis アルカリゲネス フェーカリス | 36. Gliocladium virens グリオクラディウム ビレンズ | 60. Rhizopus nigricans リゾプス ニグリカンス |
| 13. Alternaria brassicicola アルタルナリア ブラシコラ | 37. Monilia fructigena モニリア フルクティガナ | 61. Rhizopus oryzae リゾプス オリザエ |
| 14. Aureobasidium pullulans オーレオバシディウム プルランス | 38. Monilia nigral モニリア ニグラ | 62. Rhizopus storonifer リゾプス ストロニファー |
| 15. Candida albicans カンジダ アルビカンス | 39. Mucor racemosus ムコール ラセマウセス | 63. Rhizopus sorani リゾプス ソラニ |
| 16. Chaetomium globosum ケトミウム グロボサム | 40. Myrothecium verrucaria ミロテシウム バルカリア | 64. Scedosporium apiospermum セドスポリウム アピオスペルマ |
| 17. Cladosporium cladosporioides クラドスポリウム クラドスポリオイデス | 41. Mucor spinescens ムコール スピネッセンス | 65. Trichophyton mentagrophytes トリコヒートン メンタグロフィテス |
| 18. Cladosporium sphaerospermum クラドスポリウム スファエロスペルマム | 42. Nigrospora oryzae ニグロスポラ オリゼ | 66. Trichoderma viride トリコデルマ ビリデ |
| 19. Cladosporium herbarum クラドスポリウム ハーバラム | 43. Nigrospora sphaerica ニグロスポラ スフェリカ | 67. Trichoderma koningii トリコデルマ コニンギー |
| 20. Cladosporium resinae クラドスポリウム レジナエ | 44. Neurospora sitophila ニューロスポラ シトフィラ | 68. Trichoderma T1 トリコデルマ T-1 |
| 21. Curvularia lunata カルバラリア ルナータ | 45. Penicillium frequentance ペニシリウム フリークェンタンス | 69. Trichoderma harzianum トリコデルマ ハルジアナム |
| 22. Drechslera australiensis ドレッシラ オストラリエン | 46. Penicillium islandicum ペニシリウム イスランディカム | 70. Ulocladium atrum ウロクラディウム アトラム |
| 23. Epicoccum purpurascens エピコッカム パーパラセン | 47. Penicillium citrinum ペニシリウム シトリナム | 71. Wallemia sebi ワレミア セビ |
| 24. Eurotium tonophilum ユーロチウム タネフィラム | 48. Pullularia pullulans プルラリア プルランス | |

(iii) After the mixed spore solution prepared in the section (ii) was inoculated on the inorganic salt medium prepared in the section (i), a test piece obtained by cutting any one of the sheets of Examples 1-a, 1-b, 1-c, and 1-d and the sheet of Reference Example to a size of 50 mm x 50 mm was put on the medium from above. The medium was left to stand at a temperature of 28°C and a humidity of 85% RH or more for 28 days to cultivate the fungi. Then, the state of growth of the fungi was visually confirmed on every 7 days (confirmed on day 7, 14, 21, or 28 from the beginning of the cultivation), and evaluated based on the following criteria of judgment. Results are shown in Table 11.

(Criteria of judgment)

Contents of judgment

1 No growth of fungi on a surface of a test piece;
2 The fungi grew on less than 10% of the total surface of the test piece;
3 The fungi grew on 10% or more and less than 30% of the total surface of the test piece;
4 The fungi grew on 30% or more and less than 60% of the total surface of the test piece; and
5 The fungi grew on more than 60% of the total surface of the test piece.

(Results)

[Table 11]

| | Cultivation period | | | |
|---|---|---|---|---|
| | Day 7 | Day 14 | Day 21 | Day 28 |
| Example 1-a | 1 | 1 | 1 | 3~4 |
| Example 1-b | 1 | 1 | 1 | 1~2 |
| Example 1-c | 1 | 1 | 1 | 1 |
| Example 1-d | 1 | 1 | 1 | 1 |
| Reference Example | 1~2 | 2~3 | 4~5 | 5 |

As will be apparent from results shown in Table 11, among the sheets provided with the antibacterial composition of Example 1, in addition to the sheet of Example 1-d as a single-layer sheet in which the antibacterial composition was exposed as an outer layer (surface), the sheets of Examples 1-a, 1-b, and 1-c that are multilayer sheets having the antibacterial composition in an intermediate layer of the sheet were confirmed to exhibit antibacterial performance. These results suggest that even when the layer containing the antibacterial composition is provided as an intermediate layer of the molding, growth of fungi and the like on the surface of the sheet can be inhibited.

(Test Example 4)

Metal corrosion test of antibacterial composition:

Iron test pieces each having a size of 50 mm x 50 mm x 3 mm thickness were directly contacted with 50 g of the antibacterial composition of Example 1 and 50 g of the antibacterial composition of Example 2, respectively, and were left to stand at a temperature of 190°C for 90 hours, and then a change in surface condition of the iron test pieces was observed.

As a result, the iron test piece contacted with the antibacterial composition of Example 1 generated no fixation on the surface thereof and showed no change in the surface condition thereof. On the other hand, the iron test piece contacted with the antibacterial composition of Example 2 generated fixation on the surface thereof. The fixation could not be removed by wiping with a general solvent such as water or heptane and contaminated a metal such as iron. Therefore, the antibacterial composition containing halogen such as the one prepared in Example 2 is anticipated to cause deterioration of metallic parts such as metallic molds when kneading it in a resin material to obtain a molding therefrom.

(Test Example 5)

Evaluation of leather sheet containing antibacterial composition:

As a molding containing the antibacterial composition of Example 1, a leather sheet made of vinyl chloride which had a constitution shown in Table 12 and contained the antibacterial composition of Example 1 was prepared by using the production method described hereinbelow and was confirmed for its antibacterial performance. Results are shown in Table 13.

(Production method for leather sheet)

(i) The antibacterial composition of Example 1, a foaming agent (azodicarboxamide), and a vinyl chloride resin (vinyl chloride resin having a degree of polymerization of 1,300 to which an equal amount of diisodecyl phthalate as a plasticizer was added) were mixed so that 0.2 mass% of the antibacterial composition was contained in a foaming layer with respect to the sum of the foaming agent and the vinyl chloride resin that constituted the foaming layer and molded by a calender molding process to mold a 250-$\mu$m-thick sheet (foaming layer) (the foaming layer had a thickness of 500 µm by the foaming treatment described hereinbelow). Note that the foaming agent was blended in a ratio of 3.5 mass% with respect to the vinyl chloride resin.

The foaming layer was affixed to a polyester-rayon texture (thickness 600 µm) on which an adhesive was applied in advance to form an adhesive layer having a thickness of 10 µm.

(ii) A vinyl chloride resin (vinyl chloride resin having a degree of polymerization of 1,300 to which an equal amount of diisodecyl phthalate as a plasticizer was added) was molded into a sheet having a thickness of 200 µm by a calender molding process. The sheet was affixed to the foaming layer/adhesive layer/polyester-rayon texture obtained in the section (i) so that the sheet is laminated on an upper surface of the foaming layer to form a surface layer.

(iii) On the surface layer of the sheet obtained in the section (ii), a surface treating agent (a solvent type surface treating agent composed of vinyl chloride and an acrylic resin) was applied to a thickness of 5 µm after drying, and then dried at 110°C. After that, coated sheet was subjected to foaming treatment in a foaming oven at an atmospheric temperature of 230°C so that the foaming layer was 500 µm thick to obtain a leather sheet made of the vinyl chloride resin (this was referred to as a "leather sheet of Example 1").

Note that also a leather sheet made of the vinyl chloride resin containing no antibacterial composition was prepared as a blank (this was referred to as a "leather sheet of Reference Example").

(Constitution of leather sheet made of vinyl chloride)

[Table 12]

|  | Thickness (µm) |
| --- | --- |
| Surface treated layer | 5 |
| Surface layer | 200 |
| Foaming layer (note) | 500 |
| Adhesive layer | 10 |
| Polyester-rayon texture | 600 |

The antibacterial performance of the leather sheet of Example 1 made of vinyl chloride and leather sheet of Comparison thus obtained on test microorganisms shown in Table 10 was confirmed according to the test method and criteria of judgment shown in Test Example 3 (note that the test was continued till elapse of 21 days). Results are shown in Table 13.

(Results)

[Table 13]

|  | Cultivation period | | |
| --- | --- | --- | --- |
|  | Day 7 | Day 14 | Day 21 |
| Example 1 | 1 | 1 | 1 |
| Reference Example | 1 | 3 | 5 |

Results shown in Table 13 confirmed that the leather sheet of Comparison that contained no antibacterial composition suffered propagation of bacteria and growth of fungi as the cultivation period for bacteria elapsed while the leather sheet provided with the antibacterial composition of Example 1 prevented the propagation of bacteria and fungi were unable to grow at all, thus exhibiting excellent antibacterial performance.

Further, since the antibacterial composition was added to the foaming layer only, so the efficiency of contact with the microorganisms was low, but the leather was confirmed to have a repelling effect.

(Example 3 and Comparisons 2 and 3)

Based on the above-mentioned first embodiment, respective components of the formulation described in Table 14 were mixed to prepare antibacterial compositions of Example 3 and Comparisons 2 and 3.

Example 3 uses two kinds of organic antibacterial agent selected from those of the benzimidazole group, i.e., thiabendazole (2-(4-thiazolyl)-1H-benzimi dazole) and carbendazim (methyl methyl -2-benzimidazole carbamate). A blend of equal amounts (1:1) of these components was used as the organic antibacterial agent. Further, silver-supporting zirconium phosphate and zinc oxide were used in combination as the inorganic antibacterial agent in which 6 mass% of the silver-supporting zirconium phosphate and 28 mass% of zinc oxide were used.

Note that the constitution of Comparison 2 was such that no inorganic antibacterial agent was blended in the antibacterial composition of Example 3 and respective components were mixed in equal amounts (1/2). Further, the constitution of Comparison 3 was such that no organic antibacterial agent was blended in the antibacterial composition of Example 3 and the same components as the respective components of the inorganic antibacterial agent in Example 3 were blended as appropriate (33 mass% of the silver-supporting zirconium phosphate and 67 mass% of zinc oxide).

(Formulation of antibacterial composition)

[Table 14]

|  |  | Example3 | Comparison 2 | Comparison 3 |
|---|---|---|---|---|
| Benzimidazole-based organic antibacterial agent | Methyl methyl-2-benzimidazlecarbamate | 33 | 50 | - |
|  | 2-(4-thiazolyl)- H-benzimidazole | 33 | 50 | - |
| Inorganic antibacterial agent | Silver-supporting zirconium phosphate | 11 | - | 33 |
|  | Zinc oxide | 23 | - | 67 |

Then, as the test method, the same antibacterial performance test as that in Examples 1 and 2 and Comparison 1 was performed. Results are shown in Tables 15 to 20.

(Results: Fungi (1))

[Table 15]

| | | | MIC値 (ppm) | | |
|---|---|---|---|---|---|
| | | | 実施例 | 比較例2 | 比較例3 |
| 1 | アルタルナリア アルタナータ | Alternaria alternata | 1 | 1 | 250 |
| 2 | アスペルギルス アワモリ | Aspergillus awamori | 1 | 1 | |
| 3 | アスペルギルス ニガー | Aspergillus niger | 6 | 8 | 120 |
| 4 | アスペルギルス オリザエ | Aspergillus oryzae | 6 | 11 | 120 |
| 5 | アスペルギルス フラバス | Aspergillus flavus | 3 | | |
| 6 | アスペルギルス バーシカラ | Aspergillus versicolor | 10 | 8 | |
| 7 | アスペルギルス フミガータス | Aspergillus fumigatus | 3 | 3 | 250 |
| 8 | アスペルギルス ニデュランス | Aspergillus nidulans | 3 | | |
| 9 | アスペルギルス グラーカス | Aspergillus glaucus | 3 | | |
| 10 | アスペルギルス テレーズ | Aspergillus terreus | 8 | | |
| 11 | アスペルギルス ホエニカム | Aspergillus phoenicus | 8 | | |
| 12 | アスペルギルス タマリー | Aspergillus tamari | 3 | 3 | 120 |
| 13 | アスペルギルス ウェンティ | Aspergillus wentii | 3 | | |
| 14 | アスペルギルス レストリクタス | Aspergillus restrictus | 3 | | |
| 15 | アスペルギルス オクロシズ | Aspergillus ochraceus | 8 | | |
| 16 | アスペルギルス クラバタス | Aspergillus clavatus | 8 | | |
| 17 | アスペルギルス ウスタス | Aspergillus ustus | 1 | | |
| 18 | アスペルギルス カンジダス | Aspergillus candidus | 1 | 1 | 250 |
| 19 | アスペルギルス パラシティカス | Aspergillus parasiticus | 1 | | |
| 20 | アブシディア コリムビフェラ | Absidia corymbifera | 1 | | |
| 21 | アスペルギルス ラテェンシス | Aspergillus luchensis | 5 | | |
| 22 | アブシディア グラウカ | Absidia glauca | 5 | | |
| 23 | アルタルナリア テヌイス | Alternaria tenuis | 1 | | |
| 24 | アルタルナリア ピシ | Alternaria pisi | 6 | | |
| 25 | アルタルナリア カンジダス | Alternaria candidus | 3 | | |
| 26 | アルタルナリア ブランコラ | Alternaria brassicicola | 4 | 2 | 250 |
| 27 | オーレオバシディウム プルランス | Aureobasidium pullulans | 2 | 2 | 500 |
| 28 | アスコスフェラ アピス | Ascosphaera apis | 10 | | |
| 29 | アファノミセス コクリオデス | Aphanomyces cochlioides | 1 | | |
| 30 | アファニセス ラファニー | Aphanomyces raphani | 1 | | |
| 31 | ボトリティス シネレ | Botrytis cinerea | 1 | 3 | 500 |
| 32 | ビソクラミス ニベア | Byssochlamys nivea | 10 | | |
| 33 | カンジダ アルビカンス | Candida albicans | 3 | 3 | 250 |
| 34 | セレスポーラ ベティーラ | Cercospora beticola | 1 | | |
| 35 | セレスポーラ ムサー | Cercospora musae | 1 | | |
| 36 | クラビセプ パープルアー | Claviceps purpurea | 1 | | |
| 37 | コレトリカム トリフォーリ | Colletotrichum trifolii | 1 | | |
| 38 | セラトシスティス コラ | Ceratocystis cora | 1 | | |
| 39 | ケトジウム グラボサム | Chaetomium globosum | 3 | 2 | 500 |
| 40 | クラドスポリウム クラドスポリオイダス | Cladosporium cladosporoides | 6 | 6 | 250 |
| 41 | カルバリア ペニキュラータ | Curvularia peniculata | 6 | | |
| 42 | クリソスポリウム サーモフィラム | Chrysosporium thermophilum | 4 | | |
| 43 | カンジダ ギリモンディ | Candida guilliermondii | 1 | 1 | 125 |
| 44 | カンジダ リポリチカ | Candida lipolytica | 1 | 1 | 125 |
| 45 | カンジダ ペリキュローゼ | Candida pelliculosa | 1 | 1 | 125 |
| 46 | カンジダ トロピカリ | Candida tropicalis | 1 | 1 | 125 |
| 47 | カンジダ グラブラータ | Candida glabrata | 1 | 1 | 125 |
| 48 | カンジダ アキュータス | Candida acutus | 10 | 10 | 125 |
| 49 | カンジダ アチリス | Candida utilis | 10 | 10 | 125 |
| 50 | クラドスポリウム ナファエロスペルマム | Cladosporium sphaerospermum | 1 | 3 | 250 |
| 51 | クラドスポリウム ハーボラム | Cladosporium herbarum | 3 | 3 | 250 |
| 52 | コーチシウム ロルフシー | Corticium rolfsii | 1 | | |
| 53 | コレトリカム ホエイデス | Colletotrichum phomoides | 1 | 1 | 130 |
| 54 | コレトリカム フラガリエ | Colletotrichum fragariae | 1 | 1 | 130 |
| 55 | コレトリカム アトラメンタウム | Colletotrichum atramentarium | 1 | 1 | 130 |
| 56 | コレトリカム リンデマチーム | Colletotrichum lindemuthianum | 6 | | |
| 57 | セラトシスティス ウルミ | Ceratocystis ulmi | 1 | | |
| 58 | クロストリジウム アセトブチリカム | Clostridium acetobutylicum | 8 | | |
| 59 | クロストリジウム スポロゲネス | Clostridium sporogenes | 10 | | |
| 60 | クラドスポリウム カルボフィラム | Cladosporium carpophilum | 6 | | |
| 61 | カルバウリア ルナータ | Curvularia lunata | 1 | | |
| 62 | クリソスポリウム ケラチノフィラム | Chrysosporium keratinophilum | 4 | | |
| 63 | クリプトコッカス ラテイアス | Cryptococcus luteolus | 20 | | |
| 64 | クリプトコッカス ネオフォーマン | Cryptococcus neoformans | 10 | | |
| 65 | クラドスポリウム レジナエ | Cladosporium resinae | 6 | | |
| 66 | クリプトコッカス アルビダ | Cryptococcus albidus | 1 | | |
| 67 | ケトミウム クリバセウム | Chaetomium clivaceum | 1 | | |
| 68 | ダクティリウム デルドロイデス | Dactylium dendroides | 1 | | |
| 69 | ディプロディア ナタレンシス | Diplodia natalensis | 1 | | |
| 70 | ドレッシラ オストラライン | Drechslera australiensis | 3 | | |

(Results: Fungi (2))

[Table 16]

| | | | (MIC値[単位 μg/ml]) | | |
|---|---|---|---|---|---|
| | | | 実施例13 | 比較例2 | 比較例3 |
| 真菌類 | 71 | ニューロチウム タポフィラム | Eurotium tonophilum | 1 | | |
| | 72 | エピコッカム パープラセン | Epicoccum purpurascens | 1 | | |
| | 73 | ニューロチウム レペンス | Eurotium repens | 2 | | |
| | 74 | ニューロチウム ルブラム | Eurotium rubrum | 2 | | |
| | 75 | ユーロチウム シバリエリ | Eurotium chevalieri | 1 | | |
| | 76 | ニューロチウム アムステロダミ | Eurotium amstelodami | 2 | | |
| | 77 | エメリセラ ニドランス | Emericella nidulans | 3 | | |
| | 78 | エクソフィアラ ジーンセルメイ | Exophiale jeanselmei | 3 | | |
| | 79 | フザリウム セミテクタム | Fusarium semitectum | 1 | | |
| | 80 | フザリウム オキシスポラム | Fusarium oxysporum | 10 | | 200 |
| | 81 | フザリウム ボセウム | Fusarium roseum | 1 | | |
| | 82 | フザリウム モニリフォルメ | Fusarium moniliforme | 1 | | |
| | 83 | フザリウム ソラニ | Fusarium solani | 8 | | |
| | 84 | フザリウム ロゼウム | Fusarium roseum | 1 | | |
| | 85 | フザリウム ニバレ | Fusarium nivale | 1 | | |
| | 86 | フザリウム アベナシューム | Fusarium avenaceum | | | |
| | 87 | フザリウム アクミナタン | Fusarium acuminatum | | | |
| | 88 | フザリウム プロリフェラタム | Fusarium proliferatum | 1 | | |
| | 89 | フザリウム グラミネアラム | Fusarium graminearum | 1 | | |
| | 90 | フィマトリカム オムニバラン | Phymatotrichum omnivorum | 8 | | |
| | 91 | ゲオトリガム カンディダム | Geotrichum candidum | 3 | | |
| | 92 | ゲオトリカム ラクタス | Geotrichum lactus | 6 | | |
| | 93 | グリオクラデューム ビレン | Gliocladium virens | 8 | | |
| | 94 | グロメラータ シンブラータ | Glomerella cingulata | 6 | | |
| | 95 | ヘルミンソスポリウム スピーシーズ | Helminthosporium sp. | 6 | | |
| | 96 | ホモデラドラム ペドロシ | Haemodendrum pedrosoi | 3 | | |
| | 97 | ヘルミンソスポリウム グラミ | Helminthosporium graminearum | 20 | | |
| | 98 | レンズィティス トラバ | Lenzites trabea | 8 | | |
| | 99 | レンズィティス トラバエ | Lenzites trabea | 8 | | |
| | 100 | レンティナス レピデウス | Lentinus lepideus | 8 | | |
| | 101 | メデュレラ マイセトミ | Madurella mycetomii | 4 | | |
| | 102 | ミクロスポラム カニス | Microsporum canis | 3 | | |
| | 103 | ミクロスポラム ジプセューム | Microsporum gyseum | 1 | | |
| | 104 | ミクロスポラム オドウニ | Microsporum audouini | 10 | | |
| | 105 | ムコール ラセマサス | Mucor racemosus | 8 | | |
| | 106 | ミロアシウム バルカリア | Myrothecium verrucaria | 4 | | |
| | 107 | ムコール ムセド | Mucor mucedo | 4 | | |
| | 108 | ムコール バシラス | Mucor pusillus | 4 | | |
| | 109 | ムコール スピネッセンス | Mucor spinescens | 1 | | |
| | 110 | ムコール ルキシー | Mucor rouxii | 2 | | |
| | 111 | モナスカス ルバー | Monascus ruber | 6 | | |
| | 112 | モニリア カンディダ | Monilia candida | 1 | | |
| | 113 | モニリア フルクティガネ | Monilia fructigena | 10 | | |
| | 114 | モニリア ニグラ | Monilia nigra | 1 | | |
| | 115 | モニリア ラクサ | Monilia laxa | 1 | | |
| | 116 | メゴニエラ エキニータ | Megoniella echinata | 6 | | |
| | 117 | ニューロスポラ クラッサ | Neurospora crassa | 10 | | |
| | 118 | ニグロスポラ オリザエ | Nigrospora oryzae | 8 | | |
| | 119 | ニューロスポラ シトフィラ | Neurospora sitophila | 3 | | |
| | 120 | ニグロスポラ スフェリカ | Nigrospora sphaerica | 6 | | |
| | 121 | オキューレマニアム チャーティコラ | Ocuremonium chartiolo | 20 | | |
| | 122 | ペニシリウム フリークェンタス | Penicillium frequentans | 3 | 1 | 500 |
| | 123 | ペニシリウム シトリナム | Penicillium citrinum | 3 | 3 | 500 |
| | 124 | ペニシリウム バリアブル | Penicillium variabile | 1 | 1 | 500 |
| | 125 | ペニシリウム パルプロゲラム | Penicillium purpurogenum | 1 | 1 | 1000 |
| | 126 | ペニシリウム グローカム | Penicillium glaucum | 1 | 1 | 500 |
| | 127 | プルラリア プルランス | Pullularia pullulans | 1 | | |
| | 128 | ペニシリウム ロッソフォラティ | Penicillium roqueforti | 3 | 3 | 500 |
| | 129 | ペニシリウム ルテアム | Penicillium luteum | 3 | 3 | 500 |
| | 130 | ペニシリウム イクバンザム | Penicillium expansum | 3 | 3 | 500 |
| | 131 | ペニシリウム ビスカリウム | Penicillium piscarium | 3 | 3 | 1000 |
| | 132 | ペニシリウム ルグローサム | Penicillium rugulosum | 3 | 3 | 500 |
| | 133 | ペニシリウム シクロピアム | Penicillium cyclopium | 3 | 3 | 500 |
| | 134 | ペニシリウム クリソゲナム | Penicillium chrysogenum | 3 | 3 | 500 |
| | 135 | ペニシリウム シトレオ-ビリデ | Penicillium citreo-viride | 10 | | |
| | 136 | ペニシリウム ナタタム | Penicillium ristatum | 3 | 3 | 1000 |
| | 137 | ペニシリウム ルブラム | Penicillium rubrum | 3 | 3 | 1000 |
| | 138 | ペニシリウム オキザリカム | Penicillium oxalicum | 8 | 8 | 500 |
| | 139 | ペニシリウム ファニキュロサム | Penicillium funiculosum | 10 | 10 | 500 |
| | 140 | ペニシリウム ディジタータム | Penicillium digitatum | 10 | 10 | 500 |

(Results: Fungi (3))

[Table 17]

| No. | | | (MIC値(単位:ppm)) | | |
|---|---|---|---|---|---|
| | | | 基準例3 | 比較例2 | 比較例3 |
| 141 | ペニシリウム イスランディカム | Penicillium islandicum | 20 | 20 | 500 |
| 142 | ペニシリウム ニグリカンス | Penicillium nigricans | 3 | 3 | 500 |
| 143 | ペニシリウム リラシナム | Penicillium lilacinum | 20 | 20 | 500 |
| 144 | ペニシリウム スピナロザム | Penicillium spinulosum | 3 | 3 | 500 |
| 145 | ペスタロティア アダスタ | Pestalotia adusta | 20 | | |
| 146 | ペスタロティア ネグレクタ | Pestalotia neglecta | 10 | | |
| 147 | ホモプシス シトリ | Phomopsis citri | 3 | | |
| 148 | ペニシリウム ステッキイ | Penicillium stecke | 3 | | |
| 149 | フォーマ シトリカルパ | Phoma citricarpa | 3 | | |
| 150 | フォーマ テレストリス | Phoma terrestius | 3 | | |
| 151 | フォーマ グロメラタ | Phoma glomerota | 3 | | |
| 152 | フォーマ ピグメンティバロ | Phoma pigmentivora | 3 | | |
| 153 | ピッチア メムブラナファシエンス | Pichia membranaefaciens | 20 | | |
| 154 | ペプトコッカス スピーシーズ | Peptococcus sp. | 10 | | |
| 155 | プロテウス ミラビリス | Proteus mirabilis | 3 | 3 | 100 |
| 156 | ファシディオフィクテス ファンフランセ | Phacidiopycnis fumfuracea | 6 | | |
| 157 | ホモプシス フクシー | Phomopsis fukushii | 3 | | |
| 158 | ピシウム ドバリアナム | Pythium deboryanum | 3 | | |
| 159 | ピシウム イレギュラレ | Pythium debaryanum | 3 | | |
| 160 | ピシウム アファニデルマタム | Pythium aphanidermatum | 3 | | |
| 161 | ホモプシス ベーサン | Phomopsis vexan | 3 | | |
| 162 | フィトフィトラ メガスペルマ | Phytophthora megasperma | 3 | 3 | 100 |
| 163 | フィトフィトラ ニコチアネ | Phytophthora nicotianae | 3 | 3 | 100 |
| 164 | フィトフィトラ インフェスタンス | Phytophthora infestans | 3 | 3 | 100 |
| 165 | フィトフィトラ カプシシ | Phytophthora capsici | 1 | 3 | 100 |
| 166 | プラスモディオフォラ ブラシカエ | Plasmodiophora brasicae | 1 | | |
| 167 | ピレノケーター リカパーシシ | Pyrenochaeta licopersici | 1 | | |
| 168 | ロドトルラ ミヌタ | Rhodotorula minuta | 8 | | |
| 169 | ロドトルラ ムシラジノーサ | Rhodotorula muchilaginosa | 8 | | |
| 170 | ロドトルラ テキセンシス | Rhodotorula texensis | 8 | | |
| 171 | ロドトルラ グルチニス | Rhodotorula glutinis | 8 | | |
| 172 | ロドトルラ ガリニス | Rhodotorula galinis | 20 | | |
| 173 | ロドトルラ ラクトーサ | Rhodotorula lactosa | 20 | | |
| 174 | リゾプス ニグリカンス | Rhizopus nigricans | 3 | 3 | 500 |
| 175 | リゾプス オリザエ | Rhizopus oryzae | 3 | 3 | 500 |
| 176 | リゾプス ストロニファー | Rhizopus stoconifer | 3 | 3 | 500 |
| 177 | リゾプス デレマー | Rhizopus delemar | 8 | 8 | 500 |
| 178 | リゾプス ソラニ | Rhizopus solani | 3 | | |
| 179 | リゾプス ジャバニカス | Rhizopus javanicus | 8 | | |
| 180 | スポロトリカム シェンキー | Sporotrichum shenki | 10 | | |
| 181 | スティココッカス バシラビス | Stichococcus bacillavis | 10 | | |
| 182 | スクレロチニア フルクティガラ | Sclerotinia fructiogla | 10 | | |
| 183 | テッカロマイコダス パスタリアナス | Saccharomycodes pasteurianus | 3 | | |
| 184 | スタキボトリス スピーシーズ | Stachybotrys sp. | 3 | | |
| 185 | スパイカリア ボラシー | Saicaria Violacea | 3 | | |
| 186 | スコレコバシディウム カンストリクタム | Scolecobasidium constrictum | 8 | | |
| 187 | セドスポリウム アビオスペルマ | Scedosporium apiospermum | 10 | 10 | 100 |
| 188 | シンセファラストラム ラセモザム | Syncephalastrum racemosum | 3 | | |
| 189 | スタキボトリス チャートラム | Stachybotrys chartrum | 3 | | |
| 190 | スポロスリックス シェンキー | Sporothrix schenckii | 1 | | |
| 191 | スクレロチウム セピボウム | Sclerotium cepivorum | 1 | | |
| 192 | スファエロセカ フムリ | Sphaerotheca humuli | 1 | | |
| 193 | スクレロチニア スクレロチオラム | Sclerotinia sclerotiorum | 1 | | |
| 194 | スコプラリオプシス ブレビカウリス | Scopulariopsis brevicaulis | 10 | | |
| 195 | トリコヒューートン メンタグロフィテス | Trichophyton mentagrophytes | 3 | 3 | 1000 |
| 196 | トリコヒューートン ジプセーム | Trichophyton gypseum | 10 | 10 | 1000 |
| 197 | トリコヒューートン ルブラム | Trichophyton rubrum | 1 | 1 | 1000 |
| 198 | トリコテシウム ロゼアム | Trichothecium roseum | 3 | 3 | 1000 |
| 199 | トリコデルマ ビリデ | Trichoderma viride | 6 | | |
| 200 | トリコフィートン アジュロイ | Trichophyton ajelloi | 1 | 1 | 1000 |
| 201 | トリコデルマ コニンギー | Trichoderma koningii | 3 | | |
| 202 | トリコデルマ T-1 | Trichoderma T-1 | 1 | | |
| 203 | トリコデルマ ハルジアナム | Trichoderma harzianum | 6 | | |
| 204 | クラドプス カンジダ | Tolulopsis candida | 6 | | |
| 205 | トリコスポルム ククネオム | Trichosporum cutaneum | 1 | | |
| 206 | トリコデルマ リグノラム | Trichoderma lignorum | 1 | | |
| 207 | ウロクラディウム アトラム | Ulocladium atrum | 4 | | |
| 208 | ウスティラーゴ ゼア | Ustilago zeae | 10 | | |
| 209 | ヴェンティシリウム アルボアトラム | Verticillium albo-atrum | 10 | | |
| 210 | バーティシリウム ダーリエ | Verticillium dahliae | 3 | | |
| 211 | ワレミア セビ | Wallemia sebs | 3 | . | |
| 212 | オオウメラタケ | | 3 | | |
| 213 | なにわタケ | | 3 | | |
| 214 | ナミダタケ | | 3 | | |

(Results: Bacteria (1))

[Table 18]

| No. | 菌名 | Name | 実施例1 | 比較例2 | 比較例3 |
|---|---|---|---|---|---|
| | | | (MIC値 単位:ppm) | | |
| 1 | アルカリゲネス フェカリス | Alcaligenes faecalis | 1 | 8 | 80 |
| 2 | アルカリゲネス ビスコラクティス | Alcaligenes viscolactis | 1 | 8 | 80 |
| 3 | アスコフィータ ピシ | Ascophyta pisi | 10 | | |
| 4 | オートトロフィック バクテリア | Autotrophic bacteria | 30 | | |
| 5 | アスタ エロウス | Aster yellows | 1 | | |
| 6 | アキネトバクター カルコアセティカス | Acinetobacter calcoaceticus | 4 | | |
| 7 | アカクロモバクター ガルバタス | Achromobacter gulvatus | 1 | | |
| 8 | アエロバクター アエロゲネス | Aerobacter aerogenes | 1 | | |
| 9 | アエロバクター クローカエ | Aerobacter cloacae | 1 | 8 | 80 |
| 10 | ブラストマイセス イタリーカム | Blastomyces italicum | 1 | | |
| 11 | バチルス セレウス | Bacillus cereus | 1 | 8 | 80 |
| 12 | バチルス ミカイデス | Bacillus mycoides | 1 | 8 | 80 |
| 13 | バチルス ズブチルス | Bacillus subtilis | 10 | 10 | 80 |
| 14 | バチルス メガテリウム | Bacillus megaterium | 10 | 10 | 80 |
| 15 | バチルス アンスラシス | Bacillus anthracis | 10 | 10 | 80 |
| 16 | バチルス パンクタタン | Bacillus punctatum | 10 | 10 | 80 |
| 17 | バクテリウム バルガ | Bacterium vulgare | 1 | | |
| 18 | バクテリウム ロシアナム | Bacterium prorsygnum | 1 | | |
| 19 | ブラストマイセス ダマティデス | Blastomyces dermatidis | 1 | | |
| 20 | バクテロイド フラギリス | Bacteroid fragilis | 3 | | |
| 21 | カンピロバクター フィタス | Campylobacter fetus | 3 | | |
| 22 | クロストリジウム パンリゲンス | Clostridium perfringens | 3 | | |
| 23 | クロストリジウム ディフィシル | Clostridium difficile | 3 | | |
| 24 | カルティシアム フシフォルメ | Corticium fuciforme | 3 | | |
| 25 | クロストリジウム ボトリナム | Clostridium botulinum | 3 | | |
| 26 | クロエッケラ アピキュレータ | Cloeckera apiculata | 10 | | |
| 27 | セルロモナス イウジス | Cellulomonas fugis | 3 | | |
| 28 | カンピロバクター ジェジュニ／コリ | Campylobacter jejuni/coli | 10 | | |
| 29 | ドクティリウム デンドロイデス | Dactylium dendroides | 3 | | |
| 30 | ダイプロディア ビイティコラ | Diplodia viticol | 3 | | |
| 31 | デバリアマイセス ハンシニー | Debaryomyces hansenii | 15 | | |
| 32 | デサルホビブリオ デサルフリカンス | Desulfovibrio desulfuricans | 1 | | |
| 33 | エンダチア パラシティカ | Endothia parasitica | 1 | | |
| 34 | エスケリチア コリ | Escherichia coli | 15 | 15 | 400 |
| 35 | エンテロバクター アエロゲネス | Enterobacter aerogenes | 1 | | |
| 36 | エンテロバクター クローケ | Enterobacter cloacae | 10 | | |
| 37 | エルウィニア カロトボラ | Erwinia carotovora | 1 | | |
| 38 | フソバクテリウム ナクレタム | Fusobacterium nucleatum | 1 | | |
| 39 | フラボバクテリウム アミノゼン | Flavobacterium aminogenes | 10 | | |
| 40 | フラボバクテリウム メニンガセプティカム | Flavobacterium meningosepticum | 1 | | |
| 41 | グラコノバクター サバキダン | Gluconobacter suboxydans | 10 | | |
| 42 | ハンセヌラ アノマーラ | Hansenula anomala | 10 | | |
| 43 | クレブシエラ オキトカ | Klebsiella oxytoca | 10 | | |
| 44 | クレブシエラ ニューモニエ | Klebsiella pneumoniae | 3 | | |
| 45 | ラクトバシルス アシドフィラス | Lactobacillus acidophilus | 8 | | |
| 46 | ラクトバシルス プランタラム | Lactobacillus plantarum | 10 | | |
| 47 | リステリア モノシトザネス | Listeria monocytogenes | 10 | | |
| 48 | レジオネラ ニューモフィア | Legionella pneumophila | 1 | | |
| 49 | レプトスピラ インテロガン | Leptospira interrogans | 10 | | |
| 50 | レピタ クリスタダ | Lepiota crassta | 1 | | |
| 51 | レピタ カスタニー | Lupinta custanae | 1 | | |
| 52 | ラクトバシルス ブルガリクス | Lactobacillus bulgaricus | 1 | | |
| 53 | ミクロコッカス グラタミカス | Micrococcus glutamicus | 15 | 6 | 120 |
| 54 | ミクロバクテリウム ツベルクランス | Microbacterium tuberculosis | 15 | | |
| 55 | ミクロコッカス アルバス | Micrococcus albus | 1 | 80 | 120 |
| 56 | ミクロコッカス アグイリス | Micrococcus aquilis | 1 | 80 | 120 |
| 57 | ミクロコッカス コングロメラテス | Micrococcus conglomeratus | 1 | 8 | 120 |
| 58 | ミクロコッカス バリアンス | Micrococcus varians | 1 | 8 | 120 |
| 59 | パエシロミセス リカシナス | Paecilomyces lilacinus | 10 | 8 | 80 |
| 60 | ペディオコッカス ソアエ | Pediococcus soyae | 10 | | |
| 61 | ペディオコッカス アチディアチ | Pediococcus acidilactici | 10 | | |
| 62 | シュードモナス エレギノーサ | Pseudomonas aeruginosa | 20 | 8 | 125 |
| 63 | シュードモナス フルオレウス | Pseudomonas fluorescens | 3 | 8 | 125 |
| 64 | パエシロミセス バリオテ | Paecilomyces varioti | 2 | | |
| 65 | ファフィア ロドシーマ | Phaffia rhodozyma | 10 | | |
| 66 | ピッチア アノモラ | Pichia anomola | 10 | | |
| 67 | ピッチア メンブラナファシエンス | Pichia membranaefaciens | 10 | | |
| 68 | プロテウス ベルガリ | Proteus vulgaris | 15 | | |
| 69 | ピシウム ヴァンテルプーリー | Pythium vanterpoolii | 3 | 1 | 20 |
| 70 | ピシウム シネレアム | Phytesium canereum | 1 | | |
| 71 | プロピオニバクテリウム アセン | Propionibacterium acnes | 1 | | |
| 72 | プロピオニバクテリウム シェルマニ | Propionibacterium shermanii | 1 | | |
| 73 | ロドスフェラ ルコトリカ | Rodosphaera leucotricha | 1 | 8 | 20 |
| 74 | シュードモナス シリンガ | Pseudomonas syringae | 3 | 8 | 125 |
| 75 | シュードモナス ソラナセラム | Pseudomonas solanacearum | 3 | 8 | 125 |

(Results: Bacteria (2))

[Table 19]

| No. | | | (MIC値（単位 ppm)) | | |
|---|---|---|---|---|---|
| | | | 実施例43 | 比較例2 | 比較例4 |
| 76 | パラコラバクトラム アエロジーネイデス | Paracolobactrum aerogenoides | 1 | 3 | 120 |
| 77 | リゾクトニア バイオレンシ | Rhizoctonia violacea | 1 | 3 | 20 |
| 78 | リゾクトニア ソラニ | Rhizoctonia solani | 1 | 8 | 20 |
| 79 | リケッチア リケッテー | Rickettsia rickettsii | 1 | | |
| 80 | ラミノコッカス | Ruminococcus | 1 | | |
| 81 | スクレラティーナ スクレオラム | Sclerotina sclerotiorum | 1 | | |
| 82 | スパロボロマイセス ロジュー | Sporobolomyces roseus | 10 | | |
| 83 | ストレプトコッカス ラクティス | Streptococcus lactis | 10 | | |
| 84 | スキザサッカロマイセス ポンブ | Schizosaccharomyces pombe | 10 | | |
| 85 | サッカロマイコダス ラドウィギー | Saccharomycodes ludwigii | 10 | | |
| 86 | セラティア マルセセン | Serratia marcescens | 10 | | |
| 87 | スタフィロコッカス アウレス | Staphylococcus aureus | 10 | 8 | 125 |
| 88 | サルモネラ タフィーリテム | Salmonella typhimurium | 1 | 8 | |
| 89 | ストレプトベニシリウム レティクルム | Strepsoverticillum reticulum | 5 | | |
| 90 | スタフィロコッカス フェカリス | Staphylococcus faecalis | 5 | 8 | 60 |
| 91 | サルモネラ エンテリティディス | Salmonella enteritidis | 3 | 8 | 60 |
| 92 | サルモネラ エンテリカ | Salmonella enterica | 3 | 8 | 60 |
| 93 | サルモネラ アリゾネ | Salmonella arizonae | 3 | 8 | 60 |
| 94 | サルモネラ パラティフィ | Salmonella paratyphi | 3 | 8 | 60 |
| 95 | サルモネラ コトラシス | Salmonella cholerasuis | 3 | 8 | 60 |
| 96 | ストレプトコッカス アガラクティー | Streptococcus agalactiae | 3 | | |
| 97 | セラティア マルセシュー | Serratia marcescens | 1 | | |
| 98 | セラティア リグファシエンス | Serratia liquefaciens | 1 | | |
| 99 | サッカロマイセス セレビシエ | Saccharomyces cerevisiae | 1 | 10 | 120 |
| 100 | サジェラン モザイク | Sugarin mosaic | 1 | | |
| 101 | スタフィロコッカス エピデルミディス | Staphylococcus epidermidis | 1 | 8 | 125 |
| 102 | スタフィロコッカス オミニス | Staphylococcus hominis | 1 | 8 | 125 |
| 103 | スタフィロコッカス アガラクティア | Staphylococcus agalactiae | 1 | 8 | 125 |
| 104 | スタフィロコッカス ニューモニエ | Staphylococcus pneumoniae | 1 | 8 | 125 |
| 105 | スタフィロコッカス ピジエン | Staphylococcus pyogenes | 1 | 8 | 125 |
| 106 | セラアリア サリナリア | Serratia salinaria | 1 | | |
| 107 | サルモネラ タイフォテ | Salmonella typhosa | 1 | 3 | 120 |
| 108 | サルチナ フレイバ | Sarcina flava | 1 | | |
| 109 | サルチナ ルテア | Sarcina lutea | 1 | | |
| 110 | スポロシトファガ ミクソコイデス | Sporocytophaga myxococcoides | 1 | | |
| 111 | トルラ ニグラ | Torula nigra | 1 | 16 | 100 |
| 112 | サーモアクチノマイセス ブルガリス | Thermoactinomyces vulgaris | 1 | | |
| 113 | チオバチルス アシドフィラス | Thiobacillus acidophilus | 1 | 4 | 20 |
| 114 | チオバチルス デリカタス | Thiobacillus delicatus | 1 | 4 | 20 |
| 115 | チオバチルス デニトリフィカンス | Thiobacillus denitrificans | 1 | 4 | 20 |
| 116 | チオバチルス フェルキシダンス | Thiobacillus ferrooxidans | 1 | 4 | 20 |
| 117 | チオバチルス インテレメディアス | Thiobacillus intermedius | 1 | 4 | 20 |
| 118 | チオバチルス カベリス | Thiobacillus kabobis | 1 | 4 | 20 |
| 119 | チオバチルス ナポリタンス | Thiobacillus neapolitanus | 1 | 4 | 20 |
| 120 | チオバチルス ノベラス | Thiobacillus novellus | 1 | 4 | 20 |
| 121 | チオバチルス ペロメタボリス | Thiobacillus perometabolis | 1 | 4 | 20 |
| 122 | チオバチルス ルベラス | Thiobacillus rubellus | 1 | 4 | 20 |
| 123 | チオバチルス チオキシダンス | Thiobacillus thiooxidans | 1 | 4 | 20 |
| 124 | チオバチルス チオパラス | Thiobacillus thioparus | 1 | 4 | 20 |
| 125 | チオバチルス タンフィリカ イシェネスキ | Thiobacillus thermophilica imschenetskii | 1 | 4 | 20 |
| 126 | チオバチルス バスタス | Thiobacillus versutus | 1 | 4 | 20 |
| 127 | ビブレオ アルニフィカス | Vibrio alnificus | 1 | 8 | 20 |
| 128 | ベンチュリア イナクアリス | Venturia inaequalis | 1 | | |
| 129 | エルシア エンテロコリティカ | Yersinia enterocolitica | 1 | | |
| 130 | コリネバクテリウム ジフテリア | corynebacterium diphtheriae | 0.3 | 1 | 20 |
| 131 | コリネバクテリウム グルタミカム | corynebacterium glutamicum | 0.2 | 1 | 20 |

(Results: Algae)

[Table 20]

| 藻類 | | | | (MIC単位：ppm) | | |
|---|---|---|---|---|---|---|
| | | | | 実施例3 | 比較例2 | 比較例3 |
| | 1 | アナサイスティス ニデュランス | Anacystis nidulans | 10 | | |
| | 2 | アナサイスティス モンタナ | Anacystis montana | 10 | | |
| | 3 | アナサイスティス テマーリ | Anacystis thermale | 10 | | |
| | 4 | アナベナ スピーシーズ | Anabaena sp. | 10 | | |
| | 5 | アンキストロデスマ アンガステス | Ankistrodesmus angustus | 10 | | |
| | 6 | バトラコスペルマ スピーシーズ | Batrachospermum sp. | 10 | | |
| | 7 | クロレラ ブルガリ | Chlorella vulgaris | 10 | | |
| | 8 | クラドフォラ グロメラタ | Cladophora glomerata | 10 | | |
| | 9 | クラミドモナス レインハルディ | Chlamydomonas reinhardii | 10 | | |
| | 10 | クロロネッカム スピーシーズ | Chloincoccum sp. | 10 | | |
| | 11 | カロスリックス パリエティナ | Calothrix parietina | 10 | | |
| | 12 | サイリンドロカプテ スピーシーズ | Cylindrocapsa sp. | 10 | | |
| | 13 | クロレラ エマソーニ | Chlorella emersonii | 10 | | |
| | 14 | オルミディアム スピーシーズ | Hormidium sp. | 10 | | |
| | 15 | ハイルデンブランディア スピーシーズ | Hildenbrandia sp. | 10 | | |
| | 16 | メソタニウム スピーシーズ | Mesotaenium sp. | 10 | | |
| | 17 | ナスタカレ スピーシーズ | Naslcuralex sp. | 10 | | |
| | 18 | ナビキューラ スピーシーズ | Navicula sp. | 10 | | |
| | 19 | オシセラトリア ルーティーユ | Oscillatoria lutea | 10 | | |
| | 20 | プロロコッカス スピーシーズ | Pleurococcus sp. | 10 | | |
| | 21 | サイトニマ ホフマニ | Scytonema hofmanti | 10 | | |
| | 22 | シサスリックス スピーシーズ | Schizothrix sp. | 10 | | |
| | 23 | トライボニーマ スピーシーズ | Tribonema sp. | 10 | | |
| | 24 | トレンテポーリア オドラータ | Trentepohlia odorata | 10 | | |
| | 25 | トレンテポーリア アウレア | Trentepohlia aurea | 10 | | |
| | 26 | ウロスリコセー スピーシーズ | Ulotrichaceae sp. | 10 | | |
| | 27 | ジゴゴニウム スピーシーズ | Zygogonium sp. | 10 | | |

Usually, the concentration at which the antibacterial composition of the present invention is added to the solids is equal to or 100 times larger than an MIC value and hence MIC values equal to or less than 50 ppm were defined as being on a practical level in the present invention taking into consideration economical efficiency and safety.

That is, although 800 ppm or less is on an acceptance level as an antibacterial agent according to the definition (standard value) by Japan Textile Evaluation Technology Council, corporate juridical person, 100 times 800 ppm means addition of 8 mass% of the antibacterial composition, which might cause adverse influences on economical efficiency and physical properties of antibacterial moldings or antibacterial solutions.

As mentioned above, the results shown in Tables 15 to 20 indicate that the antibacterial composition of Example 3 showed MIC values of 50 ppm or less on any of test microorganisms (fungi, bacteria, and algae) and could prevent the propagation of various test microorganisms at extremely low concentrations. Thus, it was confirmed that the antibacterial composition of Example 3 had a broad antibacterial spectrum and could efficiently cope with a wide variety of microorganisms.

(Example 4 and Comparisons 4 to 7)

(Sample)

Based on the first embodiment as mentioned above, a tatami facing mat was fabricated as the antibacterial molding of the present invention and antibacterial properties thereof were compared and evaluated.

As Example 4, a polyolefin film was fabricated by mixing 0.2 mass% of the antibacterial composition of Example 3 with a polyolefin resin, kneading the mixture, and subjecting it to inflation molding. The film was molded into the form of fibers and the fibers were interwoven into a tatami facing mat.

As Comparison 4, a tatami facing mat made of polyolefin was fabricated by using thiabendazole, a commercially available antibacterial agent, in a blend ratio of 0.2 mass% in a manner similar to that in Example 4. In a manner similar to that in Example 4, silver-supporting zeolite (Shinanen Seomic (trade name)) was used in a blend ratio of 0.2 mass% to fabricate a tatami facing mat made of polyolefin as Comparison 5, while silver-supporting zeolite (Shinanen Seomic (trade name)) was used in a blend ratio of 1.0 mass% to fabricate a tatami facing mat made of polyolefin as Comparison 6. As Comparison 7, a tatami facing mat made of polyolefin was fabricated in the same manner as that in Example 4 except that no antibacterial agent was blended.

(Evaluation method)

(1) Preparation of inorganic salt medium

After the mixed spore solution shown in Table 10 was inoculated in the inorganic salt medium shown in Table 9 in Test Example 3, test pieces obtained by cutting the sheets of Example 4 and Comparisons 4 to 7 to a size of 50 mm x 50 mm were placed thereon and fungi were cultivated under conditions of a temperature of 28°C and a humidity of 85% RH or more for 28 days. Then, the state of growth of the fungi was visually confirmed and evaluated based on the criteria of judgment as used in Test Example 3. Results are shown in Table 21.

Further, Examples 4 and Comparisons 4 to 7 were also compared and evaluated for sterilizing activity (general applications) of *Staphylococcus aureus* as a strain stipulated by Japan Textile Evaluation Technology Council, corporate juridical person. Results are shown together in Table 21.

(Evaluation results)

[Table 21]

| | Sample | Evaluation of antibacterial performance after elapse of 28 days | Sterilizing activity (*Staphylococcus aureus*) |
|---|---|---|---|
| Example 4 | Product blended with antibacterial agent | 1 | 1.9 or more 1.9 or more |
| Comparison 4 | Product blended with thiabendazole (0.2 wt% blended) | 3 | -1.9 |
| Comparison 5 | Silver-supporting zeolite (0.2 wt% blended) | 4 | -1.2 |
| Comparison 6 | Silver-supporting zeolite (1.0 wt% blended) | 4 | 1.9 or more |
| Comparison | No antibacterial agent | 5 | -2 or less |

As shown in Table 21, the surface of the antibacterial composition-containing tatami mat of the present invention was confirmed to exhibit a markedly stronger fungi-preventing property than the tatami facing mat blended with thiabendazole, a conventional fungi-preventing agent. Further, Example 4 satisfied log(A/C)≥0 (A: number of microorganisms in a standard cloth immediately after inoculation, C: number of viable microorganisms in a processed cloth after cultivation of 18 hours) regarding the sterilizing activity (general applications) stipulated by Japan Textile Evaluation Technology Council, corporate juridical person, and was awarded good evaluation of the antibacterial property (sterilizing activity).

(Example 5)

(Sample)

Based on the first embodiment as mentioned above, floor waxes as a floor surface treating agent, which was a detergent, were prepared as the antibacterial composition-containing solution of the present invention, and the antibacterial properties thereof were compared and evaluated.

As the sample, an antibacterial composition-containing solution was prepared by charging ethyl alcohol, the surfactants described below, and the antibacterial composition of Example 3 in a propeller type agitator and agitating sufficiently. The blend ratios were 68 mass% of ethyl alcohol, 30 mass% of the antibacterial composition of Example 1, and 2 mass% of the above-mentioned surfactant.

Note that the surfactant was a mixture of 1 mass% of an aliphatic higher alcohol-ethylene oxide adduct and 1 mass% of a linear alkylbenzenesulfonic acid.

(Test method)

(1) The antibacterial composition-containing solution prepared by the above-mentioned method and a commercially available floor wax (trade name: LINDA super hard coat, manufactured by Yokohama Oils & Fats Industry Co., Ltd.) were appropriately agitated and mixed using a propeller type agitator to prepare cleaner waxes. The cleaner waxes were prepared such that the blend amounts of the antibacterial composition were 0 mass%, 0.05 mass%, or 0.2 mass%, respectively, as rates of content in the cleaner waxes after drying.

(2) The cleaner waxes prepared in the section (1) above were each applied on a polyethylene sheet uniformly in a state of 70 g/m$^2$ and naturally dried to obtain test pieces. Note that the coating weight after drying was about 18 g/m$^2$. Then, sterilizing activities (general applications) for *Staphylococcus aureus, Klebsiella pneumoniae,* and methicillin-resistant *Staphylococcus aureus* (MRSA) as strains stipulated by Japan Textile Evaluation Technology Council, corporate juridical person was compared and evaluated. Results are shown in Table 22.

(Evaluation method)

Evaluation was performed in the same manner as the evaluation method in the experiments in (Example 4 and Comparisons 4 to 7) mentioned above. That is, after the mixed spore solution shown in Table 10 was inoculated in the inorganic salt medium shown in Table 9 in Test Example 3, the prepared test pieces were placed thereon and fungi were cultivated under conditions of a temperature of 28°C and a humidity of 85% RH or more for 28 days. Then, the state of growth of the fungi was visually confirmed and evaluated based on the criteria of judgment as used in Test Example 3. Results are shown in Table 23.

(Sterilizing activity)

[Table 22]

| Bacteria for antibacterial test | Sterilizing activity | | |
|---|---|---|---|
| | 0 wt% coated sheet | 0.05wt% | 0.2wt% |
| *Staphylococcus aureus* | -2 or less | 0.5 | 1.9 or more |
| *Klebsiella pneumoniae* | -2 or less | 0.1 | 1.9 or more |
| MRSA | -2 or less | -0.3 | 1.9 or more |

(Evaluation Results)

[Table 23]

| Sample | Antibacterial evaluation after elapse of 28 days |
|---|---|
| 0 wt% coated sheet | 5 |
| 0.05wt% coated sheet | 3 |
| 0.2wt% coated sheet | 1 |

Tables 22 and 23 indicate that sterilizing effects were observed at a low concentration of 0.05 mass% of the antibacterial composition. The coated sheet blended with 0.2 mass% of the antibacterial composition was confirmed to have extremely excellent antibacterial and antifungal properties.

Then, specific experimental results on the second embodiment of the present invention, that is, those in which two kinds of the same group alone were used as the organic antibacterial agent are explained.

(Experiment 1)

(Examples 6 and Comparisons 8 and 9)

Based on the above-mentioned second embodiment, respective components of the formulation described in Table 24 were mixed to prepare antibacterial compositions of Example 6 and Comparisons 8 and 9.
Note that the constitution of Comparison 8 was such that no inorganic antibacterial agent was blended in the antibacterial composition of Example 6 and the respective components were mixed in equal amounts (1/2). The constitution of Comparison 9 was such that no organic antibacterial agent was blended in the antibacterial composition of Example 6 and the same components as the respective components of the inorganic antibacterial agent in Example 6 were blended as appropriate (33 mass% of silver-supporting zirconium phosphate and 67 mass% of zinc oxide).

(Formulation of antibacterial composition)

[Table 24]

| | | | | (Unit: mass%) |
| --- | --- | --- | --- | --- |
| | | Example 6 | Comparison 8 | Comparison 9 |
| Imidazole-ba sed organic antibacterial agent | Methyl 2-benzimidazole carbamate | 33 | 50 | - |
| | 2-(4-thiazolyl)-1H-benzimidazole | 33 | 50 | |
| Inorganic antibacterial agent | Silver-supporting zirconium phosphate | 11 | - | 33 |
| | Zinc oxide | 23 | - | 67 |

(Test method)

Antibacterial performance test on antibacterial compositions was performed.

As the antibacterial performance test, the antibacterial compositions obtained in Example 6 and Comparisons 8 and 9 were measured for minimum inhibitory concentration (MIC value: ppm) by the following test method and their antibacterial performance was evaluated.
(1) The antibacterial composition was diluted with dimethyl sulfoxide to predetermined concentrations (1,000 ppm, 100 ppm, 50 ppm, and the like) to prepare antibacterial suspensions.
(2) In a 9-cm Petri dish, 9 ml of an agar medium sterilized in an autoclave at 121°C for 20 minutes was cast and 1 ml of the antibacterial suspension prepared in the section (I) above was added thereto and agitated. Then, the Petri dish was left to stand at room temperature to solidify the agar med ium.
(3) On the other hand, a test strain was separately diluted to 1 x $10^6$ CFU/ml, and the resultant test strain dilution and 5 ml of a sterilized 0.9% agar medium which had been incubated at 40°C were mixed to prepare a test strain-containing agar solution.
(4) The test strain-containing agar solution prepared in the section (3) was overlaid on the agar medium in the section (2) above and solidified. In an incubator, fungi were cultivated at 27°C for 72 hours and bacteria were cultivated at 30°C for 24 hours, and then their growth was confirmed. Among the media in which the test microorganism did not grow, the one having the lowest concentration of the antibacterial composition was defined as a medium containing the antibacterial composition at minimum inhibitory concentration (MIC value: ppm). Tables 25 to 30 show the results.

(Results: Fungi (1))

[Table 25]

| | | | (MIC値(単位:ppm)) | | |
|---|---|---|---|---|---|
| | | | 実施例18 | 比較例19 | 比較例19 |
| 真菌 | 1 | アルタナリア アルタナータ | Alternaria alternata | 3 | 1 | 250 |
| | 2 | アスペルギルス アワモリ | Aspergillus awamori | 1 | 1 | |
| | 3 | アスペルギルス ニガー | Aspergillus niger | 6 | 6 | 120 |
| | 4 | アスペルギルス オリゼエ | Aspergillus oryzae | 6 | 1 | 120 |
| | 5 | アスペルギルス フラバス | Aspergillus flavus | 3 | | |
| | 6 | アスペルギルス バーシカラ | Aspergillus versicolor | 10 | 5 | |
| | 7 | アスペルギルス フミガータス | Aspergillus fumigatus | 3 | 3 | 250 |
| | 8 | アスペルギルス ニダランス | Aspergillus nidulans | 3 | | |
| | 9 | アスペルギルス グラーカス | Aspergillus glaucus | 3 | | |
| | 10 | アスペルギルス テレース | Aspergillus terreus | 5 | | |
| | 11 | アスペルギルス ホエニカス | Aspergillus phoenicus | 8 | | |
| | 12 | アスペルギルス タマリー | Aspergillus tamarii | 3 | 3 | 120 |
| | 13 | アスペルギルス ウェンティ | Aspergillus wentii | 5 | | |
| | 14 | アスペルギルス レストリクタス | Aspergillus restrictus | 3 | | |
| | 15 | アスペルギルス オクロシアス | Aspergillus ochraceus | 8 | | |
| | 16 | アスペルギルス クラバタス | Aspergillus clavatus | 5 | | |
| | 17 | アスペルギルス ウスタス | Aspergillus ustus | 1 | | |
| | 18 | アスペルギルス カンジダス | Aspergillus candidus | 1 | 1 | 250 |
| | 19 | アスペルギルス パラシティカス | Aspergillus parasiticus | 1 | | |
| | 20 | アブシディア コリムビフェラ | Absidia corymbifera | 1 | | |
| | 21 | アスペルギルス ラチュエンシス | Aspergillus luchuensis | 5 | | |
| | 22 | アブシディア グラウカ | Absidia glauca | 5 | | |
| | 23 | アルタルナリア テヌイス | Alternaria tenuis | 1 | | |
| | 24 | アルタルナリア ピシ | Alternaria pisi | 6 | | |
| | 25 | アルタルナリア カンジダス | Alternaria candidus | 3 | | |
| | 26 | アルタルナリア ブラシコウ | Alternaria brassicicola | 3 | 2 | 250 |
| | 27 | オーレオバシディウム プルランス | Aureobasidium pullulans | 3 | 2 | 500 |
| | 28 | アスコスフェラ アピス | Ascosphaera apis | 10 | | |
| | 29 | アファノミセス コクリオデス | Aphanomyces cochlioides | 1 | | |
| | 30 | アファノミセス ラプサニ | Aphanomyces raphani | 1 | | |
| | 31 | ボトリアチス サイネラ | Botrytis cinerea | 1 | 1 | 500 |
| | 32 | ビソクラミス ニベア | Byssochlamys nivea | 10 | | |
| | 33 | カンジダ アルビカンス | Candida albicans | 3 | 3 | 250 |
| | 34 | セレスポーラ ベティコツ | Cercospora beticola | 1 | | |
| | 35 | セレスポーラ ムソー | Cercospora musae | 1 | | |
| | 36 | クラビセプス パービュレアー | Claviceps purpurea | 1 | | |
| | 37 | コレトリカム トリフォーリ | Colletotrichum trifolii | 1 | | |
| | 38 | セラトシスタイス コラ | Ceratocystis coffeae | 1 | | |
| | 39 | ケトミウム グラボサム | Chaetomium globosum | 3 | 2 | 500 |
| | 40 | クラドスポリウム クラドスポリオイデス | Cladosporium cladosporioides | 6 | 5 | 250 |
| | 41 | カルバラリア ゼニキュラータ | Curvularia geniculata | 6 | | |
| | 42 | クリソスポリウム サーモフィラム | Chrysosporium thermophilum | 4 | 1 | |
| | 43 | カンジダ ギリモンディ | Candida guilliermondii | 1 | 1 | 125 |
| | 44 | カンジダ リポリチカ | Candida lipolytica | 1 | 1 | 125 |
| | 45 | カンジダ ペリキュローゼ | Candida pelliculosa | 1 | 1 | 125 |
| | 46 | カンジダ トロピカリ | Candida tropicalis | 1 | 1 | 125 |
| | 47 | カンジダ グラブラータ | Candida glabrata | 1 | 1 | 125 |
| | 48 | カンジダ アキュータス | Candida acutus | 10 | 10 | 125 |
| | 49 | カンジダ アチリス | Candida utilis | 10 | 10 | 125 |
| | 50 | ケラドスポリウム スファエロスペルマム | Cladosporium sphaerospermum | 3 | 3 | 250 |
| | 51 | クラドスポリウム ハーボラム | Cladosporium herbarum | 3 | 3 | 250 |
| | 52 | コーテンシウム ロルツシー | Corticium rolfsii | 1 | | |
| | 53 | コレトリカム ホモイデス | Colletotrichum phomoides | 1 | 1 | 120 |
| | 54 | コレトリカム フラガリエ | Colletotrichum fragariae | 1 | 1 | 120 |
| | 55 | コレトリカム アトラメンタリウム | Colletotrichum atramentarium | 1 | 1 | 120 |
| | 56 | コレトリカム リンデマチアヌム | Colletotrichum lindemuthianum | 8 | | |
| | 57 | セラトシスタイス アルミ | Ceratocystis ulmi | 1 | | |
| | 58 | クロストリディウム アセトバティリカム | Clostridium acetobutylicum | 8 | | |
| | 59 | クロストリディウム スポロゼネス | Clostridium sporogenes | 10 | | |
| | 60 | クラドスポリウム カルバフィーラム | Cladosporium carpophilum | 6 | | |
| | 61 | カルバラリア ルナータ | Curvularia lunata | 1 | | |
| | 62 | クリソスポリウム ケラチノフィラム | Chrysosporium keratinophilum | 4 | | |
| | 63 | クリプトコッカス ラフィアス | Cryptococcus luteolus | 20 | | |
| | 64 | クリプトコッカス ネオフォーマン | Cryptococcus neoformans | 10 | | |
| | 65 | クラドスポリウム レジナエ | Cladosporium resinae | 6 | | |
| | 66 | クリプトコッカス アルビダ | Cryptococcus albidus | 1 | | |
| | 67 | ケトミウム クリバセウム | Chaetomium olivaceum | 1 | | |
| | 68 | ダクティリウム デルドロイデス | Dactylium dendroides | 1 | | |
| | 69 | ディプロティア ナタレンシス | Diplodia natalensis | 1 | | |
| | 70 | ドレッシラ オストラライン | Drechslera australiensis | 3 | | |

(Results: Fungi (2))

[Table 26]

|  |  |  | MIC値(単位:ppm) | | |
|---|---|---|---|---|---|
|  |  |  | 実施例6 | 比較例8 | 比較例9 |
| 真菌類 | 71 | ニューロチウム タキシアナム | Eurotium tonophilum | 1 |  |  |
|  | 72 | エピコッカム パープラセン | Epicoccum purpurascens | 1 |  |  |
|  | 73 | ユーロチウム レペンス | Eurotium repens | 2 |  |  |
|  | 74 | ニューロチウム ルブラム | Eurotium rubrum | 2 |  |  |
|  | 75 | ユーコチウム シバリエリ | Eurotium chevalieri | 1 |  |  |
|  | 76 | ニューロチウム アムステロダミ | Eurotium amstelodami | 2 |  |  |
|  | 77 | エメリセラ ニドランス | Emericella nidulans | 3 |  |  |
|  | 78 | エクソフィアラ ジーンセルメイ | Exophiala jeanselmei | 3 |  |  |
|  | 79 | フザリウム セミテクツム | Fusarium semitectum | 1 |  |  |
|  | 80 | フザリウム オキシスポラム | Fusarium oxysporum | 10 |  | 200 |
|  | 81 | フザリウム ロセウム | Fusarium roseum | 1 |  |  |
|  | 82 | フザリウム モニリフォルメ | Fusarium moniliforme | 1 |  |  |
|  | 83 | フザリウム ソラニ | Fusarium solani | 5 |  |  |
|  | 84 | フザリウム ロゼアム | Fusarium roseum | 1 |  |  |
|  | 85 | フザリウム ニバレ | Fusarium nivale | 1 |  |  |
|  | 86 | フザリウム アベナシューム | Fusarium avenaceum | 1 |  |  |
|  | 87 | フザリウム アカミナタン | Fusarium acuminatum | 1 |  |  |
|  | 88 | フザリウム プロリフェラタム | Fusarium proliferatum | 1 |  |  |
|  | 89 | フザリウム グラミネアラム | Fusarium graminearum | 1 |  |  |
|  | 90 | フィマトリカム オムニボラン | Phymatotrichum omnivorum | 4 |  |  |
|  | 91 | ゲオトリカム カンディウム | Geotrichum candidum | 1 |  |  |
|  | 92 | ゲオトリカム ラクタス | Geotrichum lactis | 6 |  |  |
|  | 93 | グリオクラジューム ビレン | Gliocladium virens | 8 |  |  |
|  | 94 | グロメラータ シングラータ | Glomerella cingulata | 6 |  |  |
|  | 95 | エルミンツスポリウム スピーシーズ | Helminthosporium sp. | 8 |  |  |
|  | 96 | オモデアドラム ペドロシ | Hormodendrum pedrosoi | 3 |  |  |
|  | 97 | エルミンツスポリウム グラミネ | Helminthosporium gramineum | 20 |  |  |
|  | 98 | レンズィティス トラベ | Lenzites trabea | 8 |  |  |
|  | 99 | レンズィティス トラバエ | Lenzites trabae | 8 |  |  |
|  | 100 | レンティヌス レピデウス | Lentinus lepideus | 8 |  |  |
|  | 101 | マデュレラ マイセトミ | Madurella mycetomi | 4 |  |  |
|  | 102 | ミクロスポラム カニス | Microsporum canis | 3 |  |  |
|  | 103 | ミクロスポラム ジプセーム | Microsporum gypseum | 1 |  |  |
|  | 104 | ミクロスポラム オドウニ | Microsporum audouini | 10 |  |  |
|  | 105 | ムコール ラセマウセス | Mucor racemosus | 8 |  |  |
|  | 106 | ミロテシウム バルカリア | Myrothecium verrucaria | 1 |  |  |
|  | 107 | ムコール ムセド | Mucor mucedo | 4 |  |  |
|  | 108 | ムコール パシラス | Mucor pusillus | 4 |  |  |
|  | 109 | ムコール スピヌッセンス | Mucor spinescens | 1 |  |  |
|  | 110 | ムコール ルキシー | Mucor rouxii | 8 |  |  |
|  | 111 | モナスカス ルバー | Monascus ruber | 6 |  |  |
|  | 112 | モニリア カンディダ | Monilia candida | 1 |  |  |
|  | 113 | モニリア フルクティガナ | Monilia fructigena | 10 |  |  |
|  | 114 | モニリア ニグラ | Monilia nigra | 1 |  |  |
|  | 115 | モニリア ラクサ | Monilia laxa | 1 |  |  |
|  | 116 | メノニエラ エキニータ | Menoniella echinata | 6 |  |  |
|  | 117 | ニューロスポラ クラッサ | Neurospora crassa | 10 |  |  |
|  | 118 | ニグロスポラ オリザエ | Nigrospora oryzae | 1 |  |  |
|  | 119 | ニューロスポラ シトフィラ | Neurospora sitophila | 3 |  |  |
|  | 120 | ニグロスポラ スフェリカ | Nigrospora sphaerica | 6 |  |  |
|  | 121 | オキュレーマニアム チャルティカラ | Ocuremonium chartcola | 20 |  |  |
|  | 122 | ペニシリウム フリークエンタンス | Penicillium frequentans | 1 | 1 | 500 |
|  | 123 | ペニシリウム シトリナム | Penicillium citrinum | 1 | 3 | 500 |
|  | 124 | ペニシリウム バリアブル | Penicillium variabile | 1 | 1 | 500 |
|  | 125 | ペニシリウム パルロゲナム | Penicillium purpurogenum | 1 | 1 | 1000 |
|  | 126 | ペニシリウム グローカム | Penicillium glaucum | 1 | 1 | 500 |
|  | 127 | プルラリア プルランス | Pullularia pullulans | 1 |  |  |
|  | 128 | ペニシリウム ロックフォラティ | Penicillium roqueforti | 1 | 3 | 500 |
|  | 129 | ペニシリウム ルテアム | Penicillium luteum | 3 | 3 | 500 |
|  | 130 | ペニシリウム イタパンザム | Penicillium expansum | 3 | 3 | 500 |
|  | 131 | ペニシリウム ピスカリム | Penicillium piscarium | 3 | 3 | 1000 |
|  | 132 | ペニシリウム ルグロッサム | Penicillium rugulosum | 3 | 3 | 500 |
|  | 133 | ペニシリウム シクロピアム | Penicillium cyclopium | 3 | 3 | 500 |
|  | 134 | ペニシリウム クリソゲナム | Penicillium chrysogenum | 3 | 3 | 500 |
|  | 135 | ペニシリウム シトレオビリデ | Penicillium citreo-viride | 10 |  |  |
|  | 136 | ペニシリウム ノタタム | Penicillium notatum | 3 | 3 | 1000 |
|  | 137 | ペニシリウム ルブラム | Penicillium rubrum | 3 | 3 | 1000 |
|  | 138 | ペニシリウム オクザリカム | Penicillium oxalicum | 8 | 8 | 500 |
|  | 139 | ペニシリウム フェニキュロサム | Penicillium funiculosum | 10 | 10 | 500 |
|  | 140 | ペニシリウム ディジタータム | Penicillium digitatum | 10 | 10 | 500 |

(Results: Fungi (3)).

[Table 27]

| No. | 和名 | 学名 | 実施例 | 比較例 | 計数値 |
|---|---|---|---|---|---|
| 141 | ペニシリウム イスランディカム | Penicillium islandicum | 20 | 20 | 500 |
| 142 | ペニシリウム ニグリカンス | Penicillium nigricans | 3 | 3 | 500 |
| 143 | ペニシリウム リラシナム | Penicillium lilacinum | 20 | 20 | 500 |
| 144 | ペニシリウム スピノロサム | Penicillium spinulosum | 3 | 3 | 500 |
| 145 | ペスタロティア アダスタ | Pestalotia adusta | 20 | | |
| 146 | ペスタロティア ネグレクタ | Pestalotia neglecta | 10 | | |
| 147 | ホモプシス シトリ | Phomopsis citri | 3 | | |
| 148 | ペニシリウム スケッキイ | Penicillium steckii | 3 | | |
| 149 | フォーマ シトリカルパ | Phoma citricarpa | 3 | | |
| 150 | フォーマ テレスチアス | Phoma terrestris | 3 | | |
| 151 | フォーマ グロメラタ | Phoma glomerata | 3 | | |
| 152 | フォーマ ピグメンティバラ | Phoma pigmentivara | 3 | | |
| 153 | ピッチア ミムブラナファシエンス | Pichia membranaefaciens | 20 | | |
| 154 | ペプトコッカス スピーシーズ | Peptococcus sp. | 10 | | |
| 155 | プロテウス ミラビリス | Proteus mirabilis | 1 | 1 | 100 |
| 156 | ファシディオミケス ファンフランゼ | Phacidiomyces funfuracea | 6 | | |
| 157 | ホモプシス フクシ | Phomopsis fukushi | 1 | | |
| 158 | ピシウム ドバリアナム | Pythium debaryanum | 1 | | |
| 159 | ピシウム デバリュアレ | Pythium debaryanum | 1 | | |
| 160 | ピシウム アファニデルマタム | Pythium aphanidermatum | 1 | | |
| 161 | ホモプシス ベーザン | Phomopsis vexan | 1 | | |
| 162 | フィトフィトラ メガスペルマ | Phytophthora megasperma | 1 | 1 | 100 |
| 163 | フィトフィトラ ニコチアネ | Phytophthora nicotianae | 1 | 1 | 100 |
| 164 | フィトフィトラ インフェスタンス | Phytophthora infestans | 1 | 1 | 100 |
| 165 | フィトフィトラ カブシン | Phytophthora capsici | 1 | 1 | 100 |
| 166 | プラスモディオフェラ ブランカエ | Plasmodiophora brassicae | 1 | | |
| 167 | ピンノケーター リコパーシシ | Pyrenochaeta lycopersici | 1 | | |
| 168 | ロドトルラ ミムタ | Rhodotorula minuta | 8 | | |
| 169 | ロドトルラ ムシラギノーサ | Rhodotorula mucilaginosa | 8 | | |
| 170 | ロドトルラ テキサンシス | Rhodotorula texensis | 8 | | |
| 171 | ロドトルラ グルチニス | Rhodotorula glutinis | 8 | | |
| 172 | ロドトルラ ガリニス | Rhodotorula gallinis | 20 | | |
| 173 | ロドトルラ ラクトーサ | Rhodotorula lactosa | 20 | | |
| 174 | リゾプス ニグリカンス | Rhizopus nigricans | 3 | 3 | 500 |
| 175 | リゾプス オリザ | Rhizopus oryzae | 3 | 1 | 500 |
| 176 | リゾプス ストロニファー | Rhizopus stolonifer | 3 | 2 | 500 |
| 177 | リゾプス デルマ | Rhizopus delemar | 8 | 8 | 500 |
| 178 | リゾプス ソラニ | Rhizopus solani | 3 | | |
| 179 | リゾプス ジャバニカス | Rhizopus javanicus | 8 | | |
| 180 | スポロトリカム シェンキー | Sporotrichum shenki | 10 | | |
| 181 | スティココッカス バシラビス | Stichococcus bacillaris | 10 | | |
| 182 | スクレロヴェニア フルクティネオラ | Sclerotinia fructicola | 10 | | |
| 183 | サッカロマイカダス パストリアナス | Saccharomycodes pastourianus | 3 | | |
| 184 | スタキボトリス スピーシーズ | Stachybotrys sp. | 3 | | |
| 185 | スパイカリア バイラシー | Spicaria Violacea | 3 | | |
| 186 | スコレコバシディウム カンストリクタム | Scolecobasidium constrictum | 8 | | |
| 187 | セドスポリウム アピオスペルマ | Scedosporium apiospermum | 10 | 10 | 100 |
| 188 | シンセファラストラム ケモサム | Syncephalastrum racemosum | 1 | | |
| 189 | スタキボトリス チャルトラム | Stachybotrys chartrum | 1 | | |
| 190 | スポロスリックス シェンキー | Sporothrix schenckii | 1 | | |
| 191 | スクレロチウム セピボラム | Sclerotium cepivorum | 1 | | |
| 192 | スファエロセカ フムリ | Sphaerotheca humuli | 1 | | |
| 193 | スクレロチニア スクレロチオラム | Sclerotinia sclerotiorum | 1 | | |
| 194 | スコプラリオプシス ブレビカウリス | Scopulariopsis brevicaulis | 10 | | |
| 195 | トリコヒィートン メンタグロフィテス | Trichophyton mentagrophytes | 3 | 3 | 1000 |
| 196 | トリコヒィートン ジプシューム | Trichophyton gypseum | 10 | 10 | 1000 |
| 197 | トリコヒィートン ルブラム | Trichophyton rubrum | 1 | 1 | 1000 |
| 198 | トリコテシウム ロゼアム | Trichothecium roseum | 3 | 3 | 1000 |
| 199 | トリコデルマ ビリデ | Trichoderma viride | 6 | | |
| 200 | トリコフィートン アジェロイ | Trichophyton ajelloi | 1 | 1 | 1000 |
| 201 | トリコデルマ コニンギー | Trichoderma koningii | 3 | | |
| 202 | トリコデルマ T-1 | Trichoderma T-1 | 1 | | |
| 203 | トリコデルマ ハルジアナム | Trichoderma harzianum | 6 | | |
| 204 | タラロプス カンジダ | Talulopsis candida | 6 | | |
| 205 | トリコスポラム クタネウム | Trichosporum cutaneum | 1 | | |
| 206 | トリコデルマ リグノラム | Trichoderma lignorum | 1 | | |
| 207 | ウロクラディウム アトラム | Ulocladium atrum | 4 | | |
| 208 | ウスティラーゴ ゼア | Ustilago zeae | 10 | | |
| 209 | ヴェンティシリウム アルボアトラム | Verticillium albo-atrum | 10 | | |
| 210 | バーティシリウム ダーリエ | Verticillium dahliae | 1 | | |
| 211 | ワレミア セビ | Wallemia sebi | 1 | | |
| 212 | オオウズワタケ | | | 1 | |
| 213 | カワラタケ | | | 1 | |
| 214 | ナミダタケ | | | 1 | |

(Results: Bacteria (1))

[Table 28]

(MIC値(単位:ppm))

| | | | | 実施例6 | 比較例8 | 比較例9 |
|---|---|---|---|---|---|---|
| 細菌 | 1 | アルカリゲネス フェカリス | Alcaligenes faecalis | 1 | 8 | 80 |
| | 2 | アルカリゲネス ビスコラクティス | Alcaligenes viscolactis | 1 | 8 | 80 |
| | 3 | アスコフィーダ ピシ | Ascophyta pisi | 10 | | |
| | 4 | オートトロフィック バクテリア | Autotrophic bacteria | 20 | | |
| | 5 | アスタ エロウス | Aster yellows | 1 | | |
| | 6 | アキネトバクター カリカセティカス | Acinetobacter calcoaceticus | 4 | | |
| | 7 | アカクロモバクター ガルヤタス | Achromobacter gulyatus | 1 | | |
| | 8 | アエロバクター アエロゼネス | Aerobacter aerogenes | 1 | | |
| | 9 | アエロバクター クローカエ | Aerobacter cloacae | 1 | 8 | 80 |
| | 10 | ブラストマイセス イタリーカム | Blastomyces italicum | 1 | | |
| | 11 | バチルス セレウス | Bacillus cereus | 1 | 8 | 80 |
| | 12 | バチルス ミカイデス | Bacillus mycoides | 1 | 8 | 80 |
| | 13 | バチルス ズブチルス | Bacillus subtillis | 10 | 10 | 80 |
| | 14 | バチルス メガティリウム | Bacillus megaterrium | 10 | 10 | 80 |
| | 15 | バチラス アンスラシス | Bacillus anthracis | 10 | 10 | 80 |
| | 16 | バチルス パンクタタン | Bacillus punctatum | 10 | 10 | 80 |
| | 17 | バクテリウム バルガ | Bacterium vulgaro | 1 | | |
| | 18 | バクテリウム ロシアナム | Bacterium pyocyaneum | 1 | | |
| | 19 | ブラストマイセス ダマティヂス | Blastomyces deematidis | 1 | | |
| | 20 | バクテロイド フラギリス | Bacterroid fragilis | 3 | | |
| | 21 | カンピロバクター フィダス | Campylobacter fetus | 3 | | |
| | 22 | クロストリジウム パフリゲンス | Clostridium perfringens | 3 | | |
| | 23 | クロストリジウム ディフィシル | Clostridium difficile | 3 | | |
| | 24 | カルティシアム ファシフォルメ | Corticium fuciforme | 3 | | |
| | 25 | クロストリジウム ボトリナム | Clostridium botulinum | 3 | | |
| | 26 | クロエッケラ アピキュレータ | Cloechera apiculata | 10 | | |
| | 27 | セルロモナス イウジス | Cellulomonas iugis | 1 | | |
| | 28 | カンピロバクター ジュジュニ/コリ | Campylobacter jejuni/coli | 10 | | |
| | 29 | ドクティリウム デンドロイダス | Dactylium dendroides | 3 | | |
| | 30 | ダイプロディア バイティコラ | Diplodia viticol | 3 | | |
| | 31 | デバリアマイセス ハンシニー | Debaryamyces hansenii | 15 | | |
| | 32 | デザルホビブリオ デザルホリカンス | Desulfovibrio desullfuricans | 1 | | |
| | 33 | エンダチア パラシティカ | Endothia paracitica | 1 | | |
| | 34 | エスケリチア コリ | Escherichia coli | 15 | 15 | 400 |
| | 35 | エンテロバクター アエロゲネス | Enterobacter aerogenes | 1 | | |
| | 36 | エンテロバクター クローケ | Enterobacter clocae | 10 | | |
| | 37 | エルウィニア カロタボラ | Erwinia carotovora | 1 | | |
| | 38 | フソバクテリウム ナクレタム | Fusobacterium nucleatum | 1 | | |
| | 39 | フラボバクテリウム アミノゼン | Flavobacterium aminogenes | 10 | | |
| | 40 | フラボバクテリウム メニンガセプティカム | Flavobacterium meningosepticum | 1 | | |
| | 41 | グラコノバクター サバキタン | Gluconobacter suboxydans | 10 | | |
| | 42 | ハンセヌラ アノマーラ | Hansenula anomala | 10 | | |
| | 43 | クレブシエラ オキトカ | Klebsiella oxytoca | 10 | | |
| | 44 | クレブシエラ ニューモニエ | Klebsiella pneumoniae | 3 | | |
| | 45 | ラクトバシルス アシドフィラス | Lactbacillus acidophilus | 8 | | |
| | 46 | ラクトバシルス プランタラム | Lactbacillus planntarum | 10 | | |
| | 47 | リステリア モノシトゲネス | Listeria monocytogenes | 10 | | |
| | 48 | レジオネラ ニューモフィラ | Legionella pneamophila | 1 | | |
| | 49 | レプトスピラ インテロガン | Leptospira interrogans | 10 | | |
| | 50 | レピタ クリストスタ | Lepiota criststa | 1 | | |
| | 51 | レピタ カスタニー | Lepiota castanae | 1 | | |
| | 52 | ラクトバシルス ブルガリクス | Lactbacillus bulgericus | 1 | | |
| | 53 | ミクロコッカス グラタミカス | Micrococcus glatamicus | 15 | 6 | 120 |
| | 54 | ミクロバクテリウム タベルクラシス | Microbacterrium tuberculosis | 15 | | |
| | 55 | ミクロコッカス アルバス | Micrococcus albus | 1 | 80 | 120 |
| | 56 | ミクロコッカス アクイリス | Micrococcus aquilis | 1 | 80 | 120 |
| | 57 | ミクロコッカス コングロメラテス | Micrococcus conglomerates | 1 | 8 | 120 |
| | 58 | ミクロコッカス バリアンス | Micrococcus varians | 1 | 8 | 120 |
| | 59 | パエシロミセス リカシナス | Paecilomyces lilacinus | 10 | 8 | 80 |
| | 60 | パディオコッカス ソアエ | Podiococcus soyae | 10 | | |
| | 61 | パディオコッカス アキディラティ | Podiococcus acidilactici | 10 | | |
| | 62 | シュードモナス エレギノーサ | Pseudomonas aeruginosa | 20 | 8 | 125 |
| | 63 | シュードモナス フルレセウス | Pseudomonas fluresceus | 3 | 8 | 125 |
| | 64 | パエシロミセス バリオティ | Paecilomyces variotti | 2 | | |
| | 65 | ファフィア ロドシーマ | Phaffia rhodozyma | 10 | | |
| | 66 | ピッチア アノモラ | Pichia anomala | 10 | | |
| | 67 | ピッチア メンブラナファシエンス | Pichia membranaefaciens | 10 | | |
| | 68 | プロテウス ベルガリ | Proteus vulgaris | 15 | | |
| | 69 | ピシウム ヴァンテルプーリー | Pythium vanterpoolii | 1 | 1 | 20 |
| | 70 | リラシウム シネレアム | Phyrasium cinereum | 1 | | |
| | 71 | プロピオニバクテリウム アセン | Propionibacterium aces | 1 | | |
| | 72 | プロピオニバクテリウム セルマニ | Propionibacterium shermanii | 1 | | |
| | 73 | ロドスフェラ ルコトリカ | Podosphaera leucotricha | 1 | 8 | 20 |
| | 74 | シュードモナス シリンガ | Pseudomonas syringae | 3 | 8 | 125 |
| | 75 | シュードモナス ソラナセラム | Pseudomonas solanacearum | 3 | 8 | 125 |

(Results: Bacteria (2))

[Table 29]

| | No. | | | MIC値(単位：μg/ml) | | |
|---|---|---|---|---|---|---|
| | | | | 実施例6 | 比較例B | 比較例C |
| 細菌 | 76 | パラコロバクトリウム　アエロジェナイデス | Paracolobacterium aerogenoides | 1 | 3 | 120 |
| | 77 | リゾクトニア　バイオラシー | Rhizoctonia violacea | 1 | 3 | 20 |
| | 78 | リゾクトニア　ソラニ | Rhizoctonia solani | 1 | 8 | 20 |
| | 79 | リッケチア　リケッチー | Rickettsia rickettsii | 1 | | |
| | 80 | ルミノコッカス | Ruminococcus | 1 | | |
| | 81 | スクレロティニア　スクレロチオラム | Sclerotinia sclerotiorum | 1 | | |
| | 82 | スピロバロマイセス　ロシュー | Spirobolomyces roseus | 10 | | |
| | 83 | ストレプトコッカス　ラクティス | Streptococcus lactis | 10 | | |
| | 84 | スキザサッカロマイセス　ポンベ | Schizosaccharomyces pombe | 10 | | |
| | 85 | サッカロマイコデス　ラドウィギー | Saccharomycodes ludwigii | 10 | | |
| | 86 | セラチア　マルセセン | Serratia marcescens | 10 | | |
| | 87 | スタフィロコッカス　アウレス | Staphylococcus aureus | 10 | 8 | 125 |
| | 88 | サルモネラ　タイフィリウム | Salmonella typhimurium | | 8 | |
| | 89 | ストレプトベルティシラム　レティカルム | Streptoverticillium reticulum | 5 | | |
| | 90 | スタフィロコッカス　フェカリス | Staphylococcus faecalis | 5 | 8 | 60 |
| | 91 | サルモネラ　エンテリティディス | Salmonella enteritidis | 3 | 8 | 60 |
| | 92 | サルモネラ　エンテリカ | Salmonella enterica | 3 | 8 | 60 |
| | 93 | サルモネラ　アリゾナエ | Salmonella arizonae | 3 | 8 | 60 |
| | 94 | サルモネラ　パラティフィ | Salmonella paratyphi | 3 | 8 | 60 |
| | 95 | サルモネラ　コレラエスイス | Salmonella choleraesuis | 3 | 8 | 60 |
| | 96 | ストレプトコッカス　アガラクティー | Streptococcus agalactiae | 8 | | |
| | 97 | セラチア　マルセシュー | Serratia marcescens | 1 | | |
| | 98 | セラチア　リクファシエンス | Serratia liquefaciens | 1 | | |
| | 99 | サッカロマイセス　セレビシエ | Saccharomyces cerevisiae | 3 | 10 | 120 |
| | 100 | シュガーン　モザイク | Sugaren mosaic | 1 | | |
| | 101 | スタフィロコッカス　エピデルミディス | Staphylococcus epidermidis | 1 | 8 | 125 |
| | 102 | スタフィロコッカス　オミニス | Staphylococcus hominis | 1 | 8 | 125 |
| | 103 | スタフィロコッカス　アガラクティア | Staphylococcus agalactiae | 1 | 8 | 125 |
| | 104 | スタフィロコッカス　ニューモニエ | Staphylococcus pneumoniae | 1 | 8 | 125 |
| | 105 | スタフィロコッカス　ピオゲン | Staphylococcus pyogenes | 1 | 8 | 125 |
| | 106 | セラティア　サリナリア | Serratia salinaria | 1 | 8 | |
| | 107 | サルモネラ　タイフォサ | Salmonella typhosa | 1 | 8 | 125 |
| | 108 | サルシナ　フラバ | Sarcina flava | 1 | | |
| | 109 | サルシナ　ルテア | Sarcina lutea | 1 | | |
| | 110 | スポロシトファガ　ミクソコイデス | Sporocytophaga myxococcoides | 1 | | |
| | 111 | トルラ　ニグラ | Torula nigra | 1 | 16 | 160 |
| | 112 | サーモアクチノマイセス　ブルガリス | Thermoactinomyces vulgaris | 1 | | |
| | 113 | チオバチルス　アシドフィラス | Thiobacillus acidophilus | 1 | 4 | 20 |
| | 114 | チオバチルス　デリカタス | Thiobacillus delicatus | 1 | 4 | 20 |
| | 115 | チオバチルス　ヂニトリフィカンス | Thiobacillus denitrificans | 1 | 4 | 20 |
| | 116 | チオバチルス　フェルキシダンス | Thiobacillus ferrooxidans | 1 | 4 | 20 |
| | 117 | チオバチルス　インテレメディアス | Thiobacillus intermedius | 1 | 4 | 20 |
| | 118 | チオバチルス　カバリス | Thiobacillus kabobis | 1 | 4 | 20 |
| | 119 | チオバチルス　ナポリタンス | Thiobacillus neapolitanus | 1 | 4 | 20 |
| | 120 | チオバチルス　ノベラス | Thiobacillus novellus | 1 | 4 | 20 |
| | 121 | チオバチルス　ペロメタボリス | Thiobacillus perometabolis | 1 | 4 | 20 |
| | 122 | チオバチルス　ルベラス | Thiobacillus rubellus | 1 | 4 | 20 |
| | 123 | チオバチルス　チオキシダンス | Thiobacillus thiooxidans | 1 | 4 | 20 |
| | 124 | チオバチルス　チオパラス | Thiobacillus thioparus | 1 | 4 | 20 |
| | 125 | チオバチルス　テンフィリカ　イシェネスキ | Thiobacillus thermophilica imschenetskii | 1 | 4 | 20 |
| | 126 | チオバチルス　バスタス | Thiobacillus versutus | 1 | 4 | 20 |
| | 127 | ビブリオ　アルニフィカス | Vibrio ulnificus | 1 | 8 | 20 |
| | 128 | ベンチュリア　イナクアリス | Venturia inaequalis | 1 | | |
| | 129 | エルシニア　エンテロコリティカ | Yersinia enterocolitica | 1 | | |
| | 130 | コリネバクテリア　ジフテリア | corynebacterium diphtheriae | 0.3 | 1 | 20 |
| | 131 | コリネバクテリウム　グルタミカム | corynebacterium glutamicum | 0.3 | 1 | 20 |

(Results: Algae)

[Table 30]

| | | | MIC 銀 (単位 ppm) | | |
|---|---|---|---|---|---|
| | | | 実施例6 | 比較例 | 比較例 |
| 1 | アナシスティス ニデュランス | Anacystis nidulans | 10 | | |
| 2 | アナシスティス モンタナ | Anacystis montana | 10 | | |
| 3 | アナシスティス サーマリ | Anacystis thermalis | 10 | | |
| 4 | アナベナ スピーシーズ | Anabaena sp. | 10 | | |
| 5 | アンキストロデスマ アンガスタス | Ankistrodesmus angustus | 10 | | |
| 6 | バトラコスペルマ スピーシーズ | Batrachospermum sp. | 10 | | |
| 7 | クロレラ バルガリ | Chlorella vulgaris | 10 | | |
| 8 | クラドフォラ グロメラタ | Cladophora glomerata | 10 | | |
| 9 | クラミドモナス レイゼルディ | Chlamydomonas reinhardii | 10 | | |
| 10 | クロロコッカム スピーシーズ | Chlorococcum sp. | 10 | | |
| 11 | カロスリックス パリエティナ | Calothrix parietina | 10 | | |
| 12 | シリンドラカプサ スピーシーズ | Cylindrocapsa sp. | 10 | | |
| 13 | クロレラ エマソーニ | Chlorella emersonii | 10 | | |
| 14 | ホルミディアム スピーシーズ | Hormidium sp. | 10 | | |
| 15 | ハイルデンブランディア スピーシーズ | Hildenbrandia sp. | 10 | | |
| 16 | メソテニアム スピーシーズ | Mesotaenium sp. | 10 | | |
| 17 | ノストカレ スピーシーズ | Nostocales sp. | 10 | | |
| 18 | ナビキューラ スピーシーズ | Navicula sp. | 10 | | |
| 19 | オッシラトリア ルーティーエ | Oscillatoria lutea | 10 | | |
| 20 | プロロコッカス スピーシーズ | Pleurococcus sp. | 10 | | |
| 21 | サイトニマ ホフマネ | Scytonema hofmanii | 10 | | |
| 22 | シザスリックス スピーシーズ | Schizothrix sp. | 10 | | |
| 23 | トリボネーマ スピーシーズ | Tribonema sp. | 10 | | |
| 24 | トレンテポーリア オドラータ | Trentepohlia odorata | 10 | | |
| 25 | トレンテポーリア アウレア | Trentepohlia aurea | 10 | | |
| 26 | ウロトリカヤー スピーシーズ | Ulothrichaceae sp. | 10 | | |
| 27 | ジゴゴニウム スピーシーズ | Zygogonium sp. | 10 | | |

Usually, the concentration at which the antibacterial composition of the present invention is added to the solids is equal to or 100 times larger than MIC value and hence MIC values equal to or less than 50 ppm were defined as being on a practical level in the present invention taking into consideration economical efficiency and safety.

That is, although 800 ppm or less is on an acceptance level as an antibacterial agent according to the definition (standard value) by Japan Textile Evaluation Technology Council, corporate juridical person, 100 times 800 ppm means addition of 8 mass% of the antibacterial composition, which might cause adverse influences on economical efficiency and physical properties of antibacterial moldings or antibacterial solutions.

As mentioned above, the results shown in Tables 25 to 30 indicate that the antibacterial composition of Example 6 showed MIC values of 50 ppm or less on any of test microorganisms (fungi, bacteria, and algae) and could prevent the propagation of various test microorganisms at extremely low concentrations. Thus, it was confirmed that the antibacterial composition of Example 6 had a broad antibacterial spectrum and could efficiently cope with a wide variety of microorganisms.

(Experiment 2)

(Sample)

A tatami facing mat was fabricated as the antibacterial molding of the present invention and antibacterial properties thereof were compared and evaluated.

As Example 7, a polyolefin film was fabricated by mixing 0.2 mass% of the antibacterial composition of Example 6 with a polyolefin resin, kneading the mixture, and subjecting it to inflation molding. The film was molded into the form of fibers and the fibers were interwoven into a tatami facing ma t.

As Comparison 10, a tatami facing mat made of polyolefin was fabricated by using thiabendazole, a commercially available antibacterial agent, in a blend ratio of 0.2 mass% and in a manner similar to that in Example 7. In a manner similar to that in Example 7, silver-supporting zeolite (Shinanen Seomic (trade name)) was used in a blend ratio of 0.2 mass% to fabricate a tatami facing mat made of polyolefin as Comparison 11, while silver-supporting zeolite (Shinanen Seomic (trade name)) was used in a blend ratio of 1.0 mass% to fabricate a tatami facing mat made of polyolefin as Comparison 12. As Comparison 13, a tatami facing mat made of polyolefin was fabricated in the same manner as that in Example 7 except that no antibacterial agent was blended.

EP 1 779 727 A1

(Evaluation method)

(1) Preparation of inorganic salt medium

An inorganic salt medium as shown in Table 31 was prepared. After being sterilized in an autoclave at 121°C for 20 minutes, the medium was adjusted to pH 6.0 to 6.5 with an aqueous caustic soda solution (aqueous NaOH solution).

(Inorganic salt medium)

[Table 31]

| $KH_2PO_4$ | 0.7g | $FeSO_4 \cdot 7H_2O$ | 0.002g |
|---|---|---|---|
| $K_2HPO_4$ | 0.7g | $ZnSO_4 \cdot 7H_2O$ | 0.002g |
| $MgSO_4 \cdot 7H_2O$ | 0.7g | $MnSO_4 \cdot 7H_2O$ | 0.001g |
| $NH_4NO_3$ | 1.0g | Agar | 15g |
| NaCl | 0.005g | Pure water | 1,000ml |

(2) Preparation of mixed spore solution

Spores of fungi of strains shown in Table 32 below were suspended in sterilized water and filtered to prepare a mixed spore solution having a concentration of about 1 x $10^6$ cell/ml. Note that to suspend the spores, dispersion of spores was performed with sodium laurylsulfate.

(Kind of Strain)

[Table 32]

試験菌（真菌 71菌）

◇6℃±4℃、30日以内保存ストックカルチャー純培養菌使用

| | | |
|---|---|---|
| 1. Alternaria alternata アルタルナリア アルタナータ | 25. Eurotium rybrum ユーロチウム ルブラム | 49. Penicillium expansum ペニシリウム イクパンザム |
| 2. Aspergillus niger アスペルギルス ニガー | 26. Eurotium chevalieri ユーロチウム シバリエリ | 50. Penicillium cyclopium ペニシリウム シクロピアム |
| 3. Aspergillus oryzae アスペルギルス オリゼ | 27. Eurotium amstelodami ユーロチウム アムステロダミ | 51. Penicillium curvo-viride ペニシリウム シトレオビリデ |
| 4. Aspergillus flavus アスペルギルス フラバス | 28. Fusarium semitectum フザリウム セミテクタム | 52. Penicillium funiculosum ペニシリウム ファニキュローサム |
| 5. Aspergillus versicolor アスペルギルス バーシカラ | 29. Fusarium oxysporum フザリウム オキシスポラム | 53. Penicillium nigricans ペニシリウム ニグリカンス |
| 6. Aspergillus fumigatus アスペルギルス フミガータス | 30. Fusarium solani フザリウム ソラニ | 54. Penicillium lilacinum ペニシリウム リラシナム |
| 7. Aspergillus terreus アスペルギルス テレーズ | 31. Fusarium roseum フザリウム ロゼアム | 55. Pestalotia adusta ペスタロティア アダスタ |
| 8. Aspergillus restrictus アスペルギルス レストリクタス | 32. Fusarium moniliforme フザリウム モニリフォルメ | 56. Pestalotia neglecta ペスタロティア ネグレクタ |
| 9. Aspergillus ochraceus アスペルギルス オクラセアス | 33. Fusarium proliferatum フザリウム プラリフェラタム | 57. Phoma citricarpa フォーマ シトリカルパ |
| 10. Aspergillus candidus アスペルギルス カンジダス | 34. Geotrichum candidum ゲオトリカム カンディダム | 58. Phoma terrestris フォーマ テレスチアス |
| 11. Alternaria tenuis アルタルナリア テヌイス | 35. Geotrichum lactus ゲオトリカム ラクタス | 59. Phoma glomerata フォーマ グロミラタ |
| 12. Alcaligenes faecalis アルカリゲネス フューカリス | 36. Gliocladium virens グリオクラディウム ビレンズ | 60. Rhizopus nigricans リゾプス ニグリカンス |
| 13. Alternaria brassicicola アルタルナリア ブラシコラ | 37. Monilia fructigena モニリア フルクティゲナ | 61. Rhizopus oryzae リゾプス オリゼ |
| 14. Aureobasidium pullulans オーレオバシディウム プルランス | 38. Monilia nigra モニリア ニグラ | 62. Rhizopus stoconifer リゾプス ストコニファー |
| 15. Candida albicans カンジダ アルビカンス | 39. Mucor racemosus ムコール ラセマウセス | 63. Rhizopus sorani リゾプス ソラニ |
| 16. Chaetomium globosum ケトミウム グロボサム | 40. Myrothecium verrucaria ミロテシウム バルカリア | 64. Scedosporium apiospermum セドスポリウム アビオスペルマ |
| 17. Cladosporium cladosporioides クラドスポリウム | 41. Mucor spinescens ムコール スピネッセンス | 65. Trichophyton mentagrophytes トリコヒィートン ミンタグルフィテス |
| 18. Cladosporium sphaerospermum クラドスポリウム サファエロスペルマム | 42. Nigrospora oryzae ニグロスポラ オリザエ | 66. Trichoderma viride トリコヒィートン ビリデ |
| 19. Cladosporium herbarum クラドスポリウム ハーバラム | 43. Nigrospora sphaerica ニグロスポラ スフェリカ | 67. Trichoderma koningii トリコデルマ コニンギー |
| 20. Cladosporium resinae クラドスポリウム レジナエ | 44. Neurospora sitophila ニューロスポラ シトフィラ | 68. Trichoderma T-1 トリコデルマ T-1 |
| 21. Curvularia lunata カルバラリア ルナータ | 45. Penicillium frequentans ペニシリウム フリークェンタス | 69. Trichoderma harzianum トリコデルマ ハルジアナム |
| 22. Drechslera australiensis ドレシラ オストラライン | 46. Penicillium islandicum ペニシリウム イスランディカム | 70. Ulocladium atrum ウロクラディウム アトラム |
| 23. Epicoccum purpurascens エピコッカム パープラセン | 47. Penicillium citrinum ペニシリウム シトリナム | 71. Wallemia sebi ワレミア セビ |
| 24. Eurotium tonophilum ユーロチウム タネフィラム | 48. Pullularia pullulans プルラリア プルランス | |

(3) After the mixed spore solution prepared in the section (2) above was inoculated in the inorganic salt medium prepared in the section (1) above, test pieces obtained by cutting the sheets of Example 7 and Comparisons 10 and 11 to a size of 50 mm × 50 mm were placed thereon, and fungi were cultivated under conditions of a temperature of 28°C and a humidity of 85% RH or more for 28 days. Then, the state of growth of the fungi was visually confirmed and evaluated based on the criteria of judgment shown in Table 33. Results are shown in Table 34.

Further, Example 7 and Comparisons 10 and 11 were also compared and evaluated for the sterilizing activity (general applications) for *Staphylococcus aureus* as a strain stipulated by Japan Textile Evaluation Technology Council, corporate juridical person. Results are shown together in Table 34.[0178] (Criteria of judgment)

[Table 33]

| Evaluation | State of growth of fungi (visual) |
|---|---|
| 1 | No growth of fungi on a surface of a test piece |
| 2 | The fungi grew on less than 10% of the total surface of the test piece |

(continued)

| Evaluation | State of growth of fungi (visual) |
|---|---|
| 3 | The fungi grew on 10% or more and less than 30% of the total surface of the test piece |
| 4 | The fungi grew on 30% or more and less than 60% of the total surface of the test piece |
| 5 | The fungi grew on more than 60% of the total surface of the test piece |

(Evaluation Results)

[Table 34]

| | Sample | Evaluation of antibacterial performance after elapse of 28 days | Sterilizing activity (*Staphylococcus aureus*) |
|---|---|---|---|
| Example 7 | Product blended with antibacterial agent | 1 | 1.9 or more |
| Comparison 10 | Product blended with thiabendazole (0.2 wt% blended) | 3 | -1.9 |
| Comparison 11 | Silver-supporting zeolite (0.2 wt% blended) | 4 | -1.2 |
| Comparison 12 | Silver-supporting zeolite (1.0 wt% blended) | 4 | 1.9 or more |
| Comparative Example 13 | No antibacterial agent | 5 | -2 or less |

As shown in Table 34, the surface of the antibacterial composition-containing tatami mat of the present invention was confirmed to exhibit a markedly stronger fungi-preventing property than the tatami facing mat blended with thiabendazole, a conventional fungi-preventing agent. Further, Example 7 satisfied log(A/C)≥0 (A: number of microorganisms in a standard cloth immediately after inoculation, C: number of viable microorganisms in a processed cloth after cultivation of 18 hours) regarding the sterilizing activity (general applications) stipulated by Japan Textile Evaluation Technology Council, corporate juridical person, and was awarded good evaluation of the antibacterial property (sterilizing activity).

(Experiment 3)

(Sample)

Floor wax as a floor surface treating agent, which was a detergent, was prepared as the antibacterial composition-containing solution of the present invention, and the antibacterial properties thereof were compared and evaluated. As the sample, an antibacterial composition-containing solution was prepared by charging ethyl alcohol, the surfactants described below, and the antibacterial composition of Example 6 in a propeller type agitator and agitating sufficiently. The blend ratios were 68 mass% of ethyl alcohol, 30 mass% of the antibacterial composition of Example 6, and 2 mass% of the above-mentioned surfactant.
Note that the surfactant was a mixture of 1 mass% of an aliphatic higher alcohol-ethylene oxide adduct and 1 mass% of a linear alkylbenzenesulfonic acid.

(Test method)

(1) The antibacterial composition-containing solution prepared by the above-mentioned method and a commercially available floor wax (trade name: LINDA super hard coat, manufactured by Yokohama Oils & Fats Industry Co., Ltd.) were appropriately agitated and mixed using a propeller type agitator to prepare cleaner waxes. The cleaner waxes were prepared such that the blend amounts of the antibacterial composition were 0 mass%, 0.05 mass%, or 0.2 mass%, respectively, as rates of content in the cleaner waxes after drying.

(2) The cleaner waxes prepared in the section (1) above were each applied on a polyethylene sheet uniformly in a state of 70 g/m$^2$ and naturally dried to obtain test pieces. Note that the coating weight after drying was about 18 g/m$^2$. Then, sterilizing activities (general applications) for *Staphylococcus aureus, Klebsiella pneumoniae,* and methicillin-resistant *Staphylococcus aureus* (MRSA) as strains stipulated by Japan Textile Evaluation Technology Council, corporate juridical person were compared and evaluated.

Results are shown in Table 35.

(Evaluation method)

Evaluation was performed in the same manner as the evaluation method in Experiment 2 mentioned above. That is, after the mixed spore solution shown in Table 32 was inoculated in the inorganic salt medium shown in Table 31, the prepared test pieces were placed thereon and fungi were cultivated under conditions of a temperature of 28°C and a humidity of 85% RH or more for 28 days. Then, the state of growth of the fungi was visually confirmed and evaluated based on the criteria of judgment as shown in Table 33. Results are shown in Table 36.

(Sterilizing activity)

[Table 35]

| Bacteria for antibacterial test | Sterilizing activity | | |
|---|---|---|---|
| | 0 wt% coated sheet | 0.05wt% | 0.2wt% |
| *Staphylococcus aureus* | -2 or less | 0.5 | 1.9 or more |
| *Klebsiella pneumoniae* | -2 or less | 0.1 | 1.9 or more |
| MRSA | -2 or less | -0.3 | 1.9 or more |

(Evaluation Results)

[Table 36]

| Sample | Antibacterial evaluation after elapse of 28 days |
|---|---|
| 0 wt% coated sheet | 5 |
| 0.05wt% coated sheet | 3 |
| 0.2wt% coated sheet | 1 |

Tables 35 and 36 indicate that sterilizing effects were observed at a lo w concentration of 0.05 mass% of the antibacterial composition. The coated sheet blended with 0.2 mass% of the antibacterial composition was confirmed to have extremely excellent antibacterial and antifungal properties.

INDUSTRIAL APPLICABILITY

The present invention can be utilized as an antibacterial composition containing an organic antibacterial agent and an inorganic antibacterial agent, an antibacterial molding provided with the antibacterial composition, and a solution, a detergent, a tatami facing mat, and a tatami mat each containing the antibacterial composition, and can be widely used for resin-made parts and coating materials for use in environment where microorganisms are apt to propagate.

**Claims**

1. An antibacterial composition, comprising an organic antibacterial agent and an inorganic antibacterial agent.

2. The antibacterial composition according to claim 1, wherein the inorganic antibacterial agent is zirconium having supported thereon a metal or a salt thereof or zeolite having supported thereon a metal.

3. The antibacterial composition according to claim 2, wherein the inorganic antibacterial agent is zirconium phosphate having supported thereon silver or copper or a salt thereof or zeolite having supported thereon silver or copper.

4. The antibacterial composition according to claim 1, wherein the inorganic antibacterial agent is at least one of a silver-based antibacterial agent and zinc oxide.

5. The antibacterial composition according to claim 4, wherein the silver-based antibacterial agent is zirconium having supported thereon silver or a salt thereof or zeolite having supported thereon silver.

6. The antibacterial composition according to claim 5, wherein the inorganic antibacterial agent contains: the zirconium having supported thereon silver or a salt thereof or zeolite having supported thereon silver; and the zinc oxide in a blend ratio of 1:1 to 1:10 by mass.

7. The antibacterial composition according to any one of claims 1 to 6, wherein the organic antibacterial agent is a pyridine-based antibacterial agent or a benzimidazole-based antibacterial agent.

8. The antibacterial composition according to claim 7, wherein:

   the pyridine-based antibacterial agent is 2-mercaptopyridine-N-oxide sodium; and
   the benzimidazole-based antibacterial agent is at least one of carbendazim (methyl 1H-2-benzimidazole carbamate) and thiabendazole (2-(4-thiazolyl)-1H-benzimidazole).

9. The antibacterial composition according to any one of claims 1 to 6, wherein the organic antibacterial agent comprises two kinds selected from the benzimidazole-based antibacterial agents.

10. The antibacterial composition according to any one of claims 1 to 6, wherein the antibacterial composition comprises:

    at least two kinds selected from imidazole-based organic antibacterial agents; and
    the inorganic antibacterial agent.

11. The antibacterial composition according to claim 9 or 10, wherein the two kinds selected from the benzimidazole-based organic antibacterial agents comprises a benzimidazole-based organic agent having a thiazolyl group on a benzimidazole ring thereof and a benzimidazole-based organic agent having a carbamate group on a benzimidazole ring thereof.

12. The antibacterial composition according to claim 11, wherein:

    the benzimidazole-based organic agent having a thiazolyl group on a benzimidazole ring thereof is 2-(4-thiazolyl)-1H-benzimidazole; and
    the benzimidazole-based organic agent having a carbamate group on a benzimidazole ring thereof is methyl 2-benzimidazole carbamate.

13. The antibacterial composition according to any one of claims 7 to 12, wherein the imidazole-based organic antibacterial agent and the inorganic antibacterial agent are contained in a blend ratio of 1:1 to 5:1 by mass.

14. The antibacterial composition according to any one of claims 1 to 13, wherein the organic antibacterial agent and the inorganic antibacterial agent contain substantially no halogen.

15. The antibacterial composition according to any one of claims 1 to 13, wherein the antibacterial composition contains no halogen compound and is substantially insoluble in water.

16. The antibacterial composition according to any one of claims 1 to 15, wherein the inorganic antibacterial agent has a rate of content of 0.1 mass% or more and 70 mass% or less with respect to a total composition.

17. An antibacterial molding, comprising the antibacterial composition according to any one of claims 1 to 16.

18. The antibacterial molding according to claim 17, wherein the antibacterial composition is contained in an amount of 0.01 mass% or more and 10.0 mass% or less with respect to the molding.

19. The antibacterial molding according to claim 17 or 18, wherein:

the antibacterial molding contains the antibacterial composition such that the inorganic antibacterial agent is contained in an amount of less than 0.5 mass% with respect to a total mass; and
the antibacterial molding has a sterilizing activity (general applications) stipulated by Japan Textile Evaluation Technology Council of conditions as mentioned below:

$$\log (A/C) \geq 0;$$

A: Number of microorganism on a standard cloth immediately after inoculation;
C: Number of viable microorganism on a processed cloth after cultivation of 18 hours;
Kind of microorganism: *Staphylococcus aureus* and *Klebsiella pneumoniae.*

20. The antibacterial molding according to claim 17 or 18, wherein the antibacterial molding is in the form of a film or a sheet or a laminate of these.

21. The antibacterial molding according to any one of claims 17 to 20, wherein:

the molding is a multilayer sheet; and
a layer containing the antibacterial composition is not exposed as an outer layer.

22. An antibacterial composition-containing solution, comprising a solution having dispersed therein the antibacterial composition according to any one of claims 1 to 16.

23. The antibacterial composition-containing solution according to claim 22, wherein the antibacterial composition is dispersed in a concentration of 0.1 mass% or more and 50 mass% or less.

24. The antibacterial composition-containing solution according to claim 22, wherein the antibacterial composition is dispersed such that the antibacterial composition-containing solution is capable of being diluted to have a concentration of the antibacterial composition upon use of 10 ppm or more and 1,000 ppm or less.

25. A detergent, comprising the antibacterial composition-containing solution according to any one of claims 22 to 24.

26. A tatami facing mat, comprising a film containing the antibacterial composition according to any one of claims 1 to 16.

27. A tatami mat, comprising a film containing the antibacterial composition according to any one of claims 1 to 16.

# FIG.1

<table>
<tr><td align="center">**INTERNATIONAL SEARCH REPORT**</td><td>International application No.<br>PCT/JP2005/012834</td></tr>
</table>

A. CLASSIFICATION OF SUBJECT MATTER
Int.Cl⁷ A01N59/16, 43/40, 43/78, 47/18, 59/20, 43/52, C08K3/00, 5/00, C08L101/00, C11D3/48, B32B27/18, E04F15/02

According to International Patent Classification (IPC) or to both national classification and IPC

B. FIELDS SEARCHED

Minimum documentation searched (classification system followed by classification symbols)
Int.Cl⁷ A01N59/16, 43/40, 43/78, 47/18, 59/20, 43/52, C08K3/00, 5/00, C08L101/00, C11D3/48, B32B27/18, E04F15/02

Documentation searched other than minimum documentation to the extent that such documents are included in the fields searched
Jitsuyo Shinan Koho       1922-1996   Jitsuyo Shinan Toroku Koho   1996-2005
Kokai Jitsuyo Shinan Koho  1971-2005   Toroku Jitsuyo Shinan Koho   1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 10-109912 A   (Akio SUGANUMA),<br>28 April, 1998 (28.04.98),<br>Claims; Par. Nos. [0008] to [0016]<br>& EP 834253 A2          & US 5985795 A<br>& CN 1181185 A          & KR 98032546 A | 1-5,9-27<br>6-8 |
| X<br>Y | JP 2003-522734 A   (ARCH CHEMICALS, INC.),<br>29 July, 2003 (29.07.03),<br>Claims<br>& EP 1189504 A1          & WO 200100021 A1<br>& CN 1364056 A          & KR 2002020746 A<br>& AU 200060548 A | 1-6,14-27<br>7-13 |

☒ Further documents are listed in the continuation of Box C.   ☐ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>06 September, 2005 (06.09.05) | Date of mailing of the international search report<br>04 October, 2005 (04.10.05) |
|---|---|
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

| International application No. |
| --- |
| PCT/JP2005/012834 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X<br>Y | JP 7-76654 A  (Shin-Etsu Chemical Co., Ltd.),<br>20 March, 1995 (20.03.95),<br>Claims; Par. Nos. [0008] to [0011]<br>(Family: none) | 1-5,7,9-27<br>6,8 |
| X<br>Y | JP 11-21400 A  (Otsuka Chemical Co., Ltd.),<br>26 January, 1999 (26.01.99),<br>Claims; Par. Nos. [0007] to [0011]<br>(Family: none) | 1-5,7,9-27<br>6,8 |
| Y | JP 8-239507 A  (Toppan Printing Co., Ltd.),<br>17 September, 1996 (17.09.96),<br>Claims; Par. Nos. [0011], [0019] to [0031]<br>(Family: none) | 6,8 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 8092012 A

- JP 2004339102 A